# EUROPEAN PATENT SPECIFICATION

(11) **EP 2 473 043 B1**
(45) Date of publication and mention of the grant of the patent: **25.03.2020**
(21) Application number: 10814537.6
(22) Date of filing: 02.09.2010
(51) Int. Cl.: A01N 43/04, A61Q 17/00, A61K 8/73, A61Q 11/00

(54) **ORAL CARE METHODS AND COMPOSITIONS UTILIZING CHITOSAN-DERIVATIVE COMPOUNDS**
MUNDPFLEGEVERFAHREN UND -ZUSAMMENSETZUNGEN MIT CHITOSANDERIVAT-KOMPONENTEN
PROCÉDÉS ET COMPOSITIONS DE SOINS ORAUX UTILISANT DES COMPOSÉS DÉRIVÉS DE CHITOSANE

(30) Priority: 02.09.2009 US 239181 P
(43) Date of publication of application: 11.07.2012
(73) Proprietor: Synedgen, Inc., Claremont, CA 91711 (US); Baker, Shena D., Upland, CA 91784 (US); Wiesmann, William P., Washington, DC 20007 (US); Townsend, Stacy, Claremont, CA 91711 (US)
(72) Inventor: BAKER, Shena, D., Upland, CA 91784 (US); WIESMANN, William, P., Washington, DC 20007 (US); TOWNSEND, Stacy, Claremont, CA 91711 (US)
(74) Representative: Williams, Gareth Owen
(86) International application number: PCT/US2010/047759
(87) International publication number: WO 2011/028968

(56) References cited:
- WO-A1-2006/005211
- JP-A- 2007 314 505
- US-A- 4 512 968
- US-A1- 2005 163 727
- US-A1- 2007 281 904
- US-A1- 2008 242 794
- DATABASE GNPD [Online] MINTEL; June 2000 (2000-06), Optima Healthcare: "Aloe Toothpaste Range", XP002742494, Database accession no. 26767
- JAE-YOUNG JE ET AL: "Chitosan Derivatives Killed Bacteria by Disrupting the Outer and Inner Membrane", JOURNAL OF AGRICULTURAL AND FOOD CHEMISTRY, vol. 54, no. 18, 1 September 2006 (2006-09-01), pages 6629-6633, XP055203350, US ISSN: 0021-8561, DOI: 10.1021/jf061310p
- TANG H ET AL: "Antibacterial action of a novel functionalized chitosan-arginine against Gram-negative bacteria", ACTA BIOMATERIALIA, ELSEVIER, AMSTERDAM, NL, vol. 6, no. 7, 1 July 2010 (2010-07-01), pages 2562-2571, XP027052687, ISSN: 1742-7061 [retrieved on 2010-05-15]

## Description

### Field of the Invention

The invention relates to soluble derivatized chitosans and their use to reduce bacteria in the mouth in a subject.

### Background

Microbial populations are present in body cavities including the mouth and throat. Unbalanced populations of bacteria can cause increases in particular microbial populations that are no longer controlled or in balance with the body. These conditions can occur through e.g., pathogenic infections, compromised immune system, and side effects from antibiotics. The antimicrobial activities of six kinds of water-soluble chitosan have been shown to disrupt the outer and inner membranes of bacteria leading to growth suppression (Jae-Young Je et al, Journal of Agricultural and Food Chemistry, 2006, pages 6629-6633). An oral hygiene composition comprising a chitosan derivative and its use for the prevention or treatment of caries is described in WO 2006/005211. The use of chitosan-derivative compounds in controlling microbial populations in humans is described in US 2007/281904.

### Summary of the Invention

The present invention provides a soluble derivatised chitosan for use in an oral rinse composition, in accordance with claim 1. Further features of the invention are defined in the dependent claims. Oral care compositions comprising soluble derivatized chitosans are described herein. Exemplary methods using the compositions described herein include methods of reducing bacteria in the mouth of a subject, methods of clumping bacteria and removing the resulting clumped bacteria from a subject (e.g., from the mouth of a subject), and disrupting a biofilm in the mouth in a subject. In some embodiments of the disclosure a composition described herein can be used to treat or prevent a disorder (e.g., a disorder in the mouth of a subject).

In one aspect, the disclosure features a method of reducing bacteria in the mouth of a subject, the method comprising: contacting (e.g., rinsing) the mouth with an effective amount of a composition comprising a soluble chitosan or derivatized chitosan, thereby reducing bacteria in the mouth of the subject.

In one embodiment, the amount of the bacteria in the mouth of the subject is reduced by at least 10, 20, 30, 40, 50, 60, 70, 80, 90, 95, 99, 99.9, 99.99 or 99.999%, compared to the amount of the bacteria that has not been contacted with the composition.

In one embodiment, the subject rinses the mouth with at least 2, 4, 6, 8, 10, 12, 14, 16, 18, 20 ml of the composition comprising the composition.

In one embodiment, the subject rinses the mouth with the composition for a period of at least 15 seconds, 30 seconds, 1 minute, 2 minutes, 3 minutes, 4 minutes or 5 minutes.

In one embodiment, the composition is not ingested by the subject (e.g., no substantial amount of the composition).

In one embodiment, the subject rinses the mouth with the composition at least 1, 2, 3, 4, 5 or 6 times per day.

In one embodiment, the subject rinses the mouth with the composition at least once per day for a period of at least 1, 5, 10, 30, 60, 90, 120, 150, or 180 days.

In one embodiment, the subject rinses the mouth with 10 ml of the composition twice per day.

In one embodiment, the concentration of the soluble derivatized chitosan is at least 5, 10, 50, 100, 200, 300, 400, 500, or 1000 ppm.

In one embodiment, the concentration of the soluble derivatized chitosan is at least 5, 10, 50, 100, 200, 300, 400, 500, or 1000 µg/ml.

In one embodiment, the subject has one or more oral diseases or conditions, e.g., pridontitis, dental plaque, gingivitis, dental caries, or halitosis.

In one embodiment, the subject has dental plaque, swollen gums; mouth sores; bright-red, or purple gums; shiny gums; swollen gums that emit pus; severe oral odor; gums that are painless; except when pressure is applied; gums that bleed easily, even with gentle brushing, and especially when flossing; or gums that itch with varying degrees of severity.

In one embodiment, the subject is infected with *Streptococcus mutans, Streptococcus sanguis, Treponema denticola, Porphyromonas gingivalis, Aggregatibacter actinomycetemcomitans, Fusospirochetes, Veillonella,* and some forms of pathogenic *Lactobacilli, Actinomyces viscosus,* or *Nocardia spp.*

In one embodiment, the method further comprises the step of administering an antibiotic or antiseptic (e.g., metronidazole, hydrogen peroxide, cetylpryridinium chloride, xylitol, or chlorhexidine) to a subject, e.g., in a dosage to achieve a synergistic effect.

In one embodiment, the method further comprises the step of physically removing superficial layers of bacteria and related debris from the mouth through use of mechanical or ultrasonic debridement.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 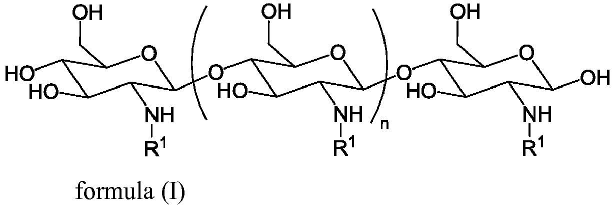 wherein:
n is an integer between 20 and 6000; and
each R¹ is independently selected for each occurrence from hydrogen, acetyl, and a group of formula (II): 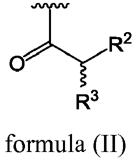 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, wherein R¹ is selected from one of the following:

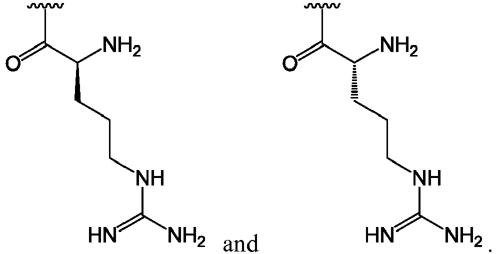

In one embodiment, R² is amino and R³ is a lysine side chain.

In one embodiment, R¹ is selected from one of the following:

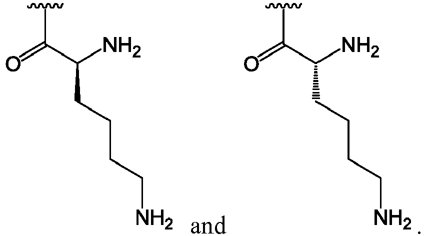

In one embodiment, R² is amino and R³ is a histidine side chain.

In one embodiment, R¹ is selected from one of the following:

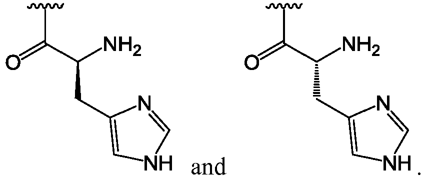

In one embodiment, at least 1% of R¹ substituents are selected from one of the following: 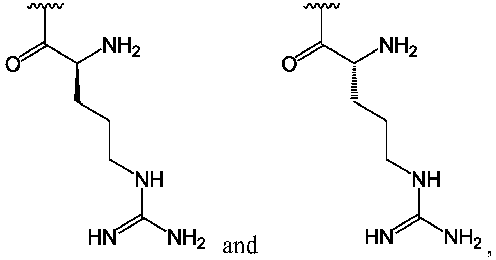 and at least 1% of R¹ substituents are selected from the following:

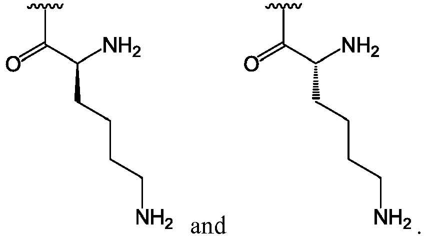

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

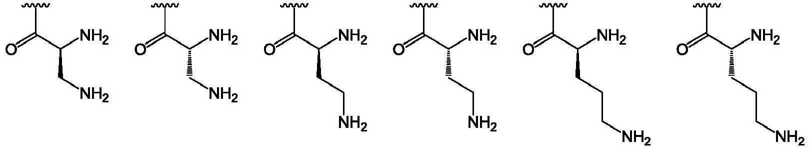

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

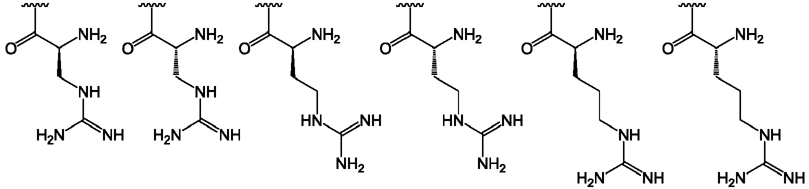

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

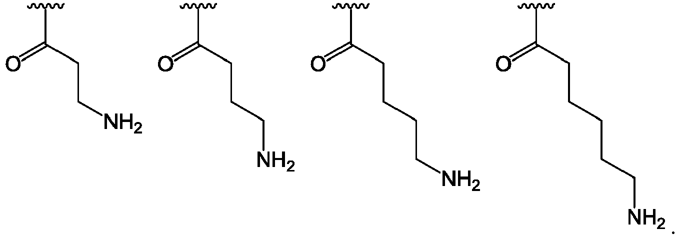

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

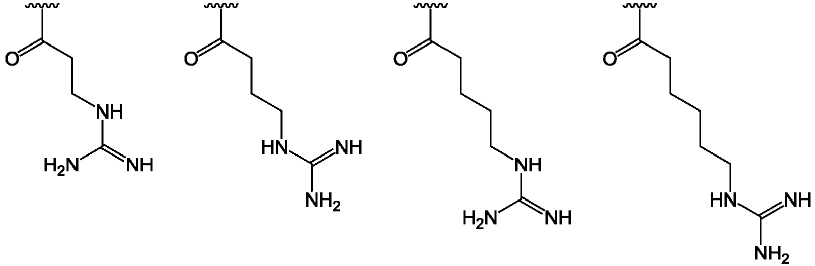

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In a preferred embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the composition further comprises an additional agent, e.g., a pharmaceutical agent such as metronidazole, hydrogen peroxide, cetylpyridinium chloride, xylitol, or chlorhexidine, or a non-pharmaceutical agent (e.g., a non-toxic surfactant). In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the composition has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the composition has less than about 20%, 15%, 10%, 5%, 2%, or 1%, or is substantially free, of a chitosan polymer having a molecular weight of less than 15,000 Da, 10,000 Da, or 5,000 Da.

In another aspect, the disclosure features a method of disrupting (e.g., reducing the viscosity of, or dissolving) a biofilm, or preventing the formation of a biofilm (e.g., reducing the ability of a biofilm to form) in the mouth of a subject, the method comprising: contacting (e.g., rinsing) the mouth with an effective amount of a composition comprising a soluble chitosan or derivatized chitosan, thereby disrupting (e.g., reducing the viscosity of, or dissolving) the biofilm in the mouth of the subject.

In one embodiment, the viscosity (e.g., apparent viscosity) of the biofilm is reduced by at least 10, 20, 30, 40, 50, 60, 70, 80, 90, 95, or 99%, compared to the biofilm that has not been contacted with the soluble chitosan or derivatized chitosan.

In one embodiment, the biofilm is partially dissolved, e.g., at least 10, 20, 30, 40, 50, 60, 70, 80, 90, 95, 99, 99.9, 99.99, or 99.999% of the biofilm is dissolved, compared to the biofilm that has not been contacted with the soluble chitosan or derivatized chitosan.

In one embodiment, the method further comprises administering an antibiotic or anti-inflammatory compound to a subject in conjunction with or subsequent to the administration of the composition comprising the soluble derivatized chitosan.

In one embodiment, the second compound is administered in a dosage to achieve a synergistic effect.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 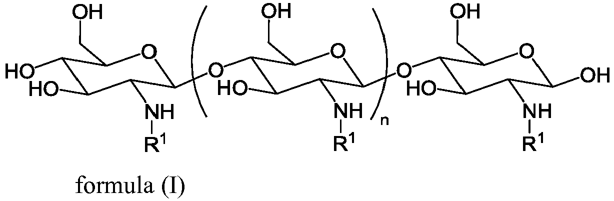 wherein:
n is an integer between 20 and 6000; and
each R¹ is independently selected for each occurrence from hydrogen, acetyl, and a group of formula (II): 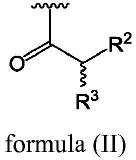 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, R¹ is selected from one of the following:

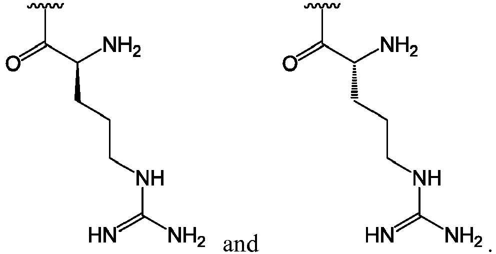

In one embodiment, R² is amino and R³ is a lysine side chain.

In one embodiment, R¹ is selected from one of the following:

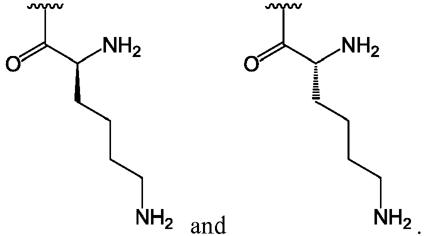

In one embodiment, R² is amino and R³ is a histidine side chain.

In one embodiment, R¹ is selected from one of the following:

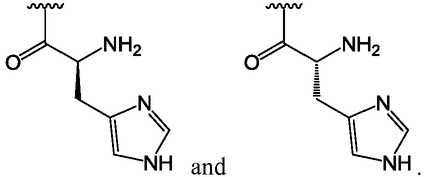

In one embodiment, at least 1% of R¹ substituents are selected from one of the following: 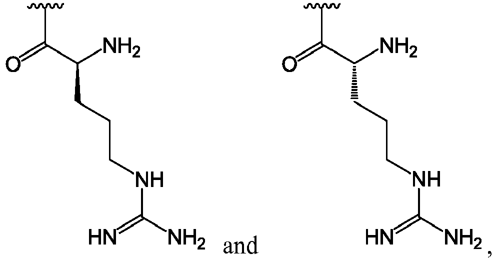 AND at least 1% of R¹ substituents are selected from the following:

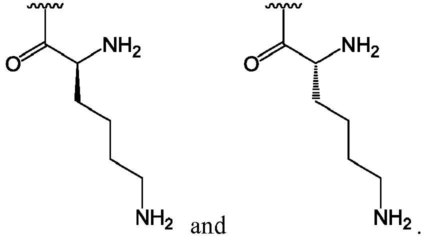

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

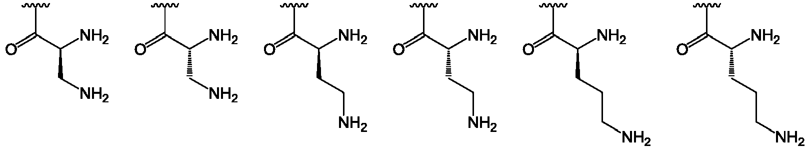

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

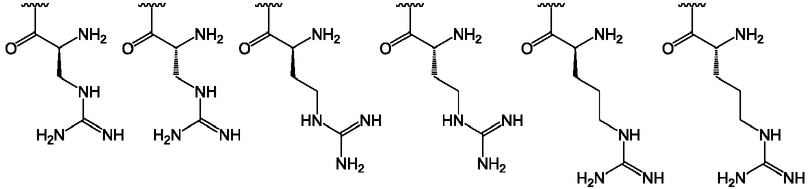

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

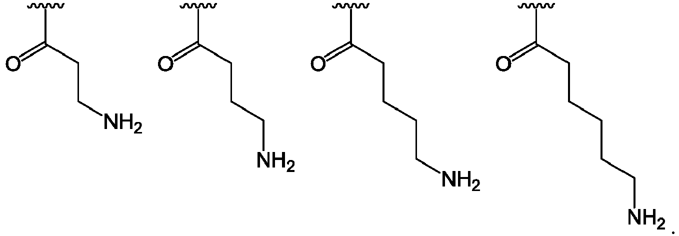

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

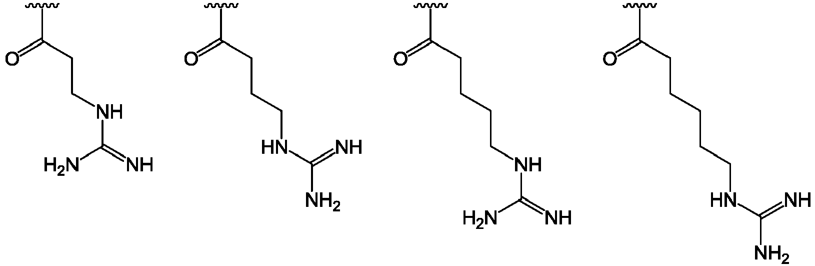

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In a preferred embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the composition further comprises an additional agent, e.g., a pharmaceutical agent such as metronidazole, hydrogen peroxide, cetylpyridinium chloride, xylitol, or chlorhexidine, or a non-pharmaceutical agent (e.g., a non-toxic surfactant). In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the composition has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the composition has less than about 20%, 15%, 10%, 5%, 2%, or 1%, or is substantially free, of a chitosan polymer having a molecular weight of less than 15,000 Da, 10,000 Da, or 5,000 Da.

In another aspect, the disclosure features a method of lowering the amount of the C-reactive proteins and/or lipoprotein-associated phospholipase A2 in a subject, the method comprising contacting (e.g., rinsing) the mouth with an effective amount of a composition comprising a soluble derivatized chitosan, thereby lowering the amount of the C-reactive proteins and/or lipoprotein-associated phospholipase A2.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 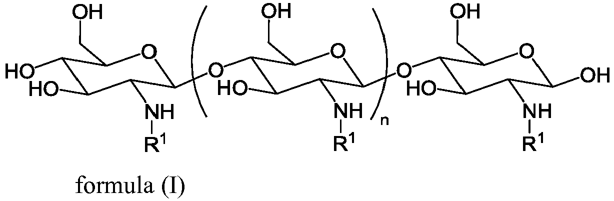 wherein:
n is an integer between 20 and 6000; and
each R¹ is independently selected for each occurrence from hydrogen, acetyl, and a group of formula (II): 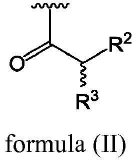 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, R¹ is selected from one of the following:

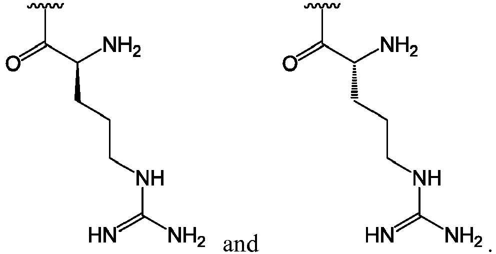

In one embodiment, R² is amino and R³ is a lysine side chain.

In one embodiment, R¹ is selected from one of the following:

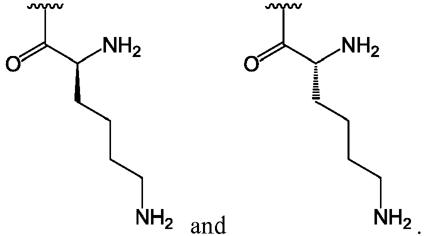

In one embodiment, R² is amino and R³ is a histidine side chain.

In one embodiment, R¹ is selected from one of the following:

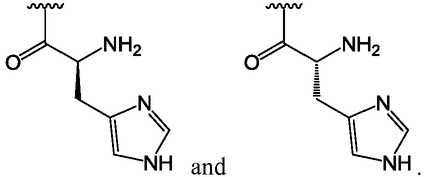

In one embodiment, at least 1% of R¹ substituents are selected from one of the following: 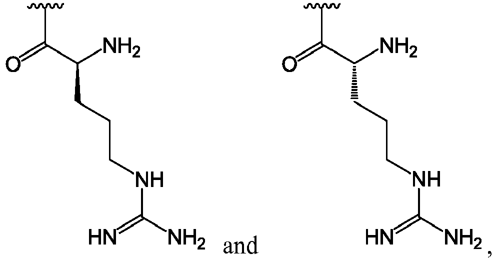 and at least 1% of R¹ substituents are selected from the following:

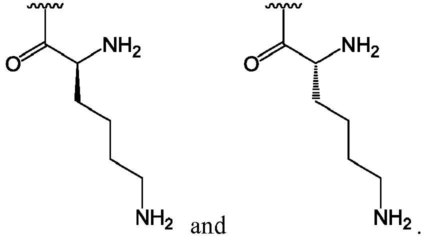

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

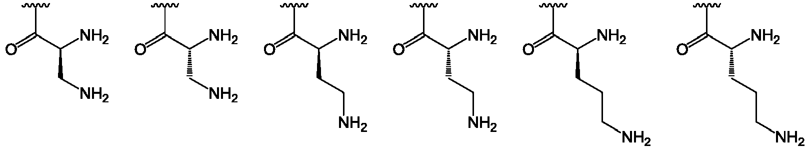

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

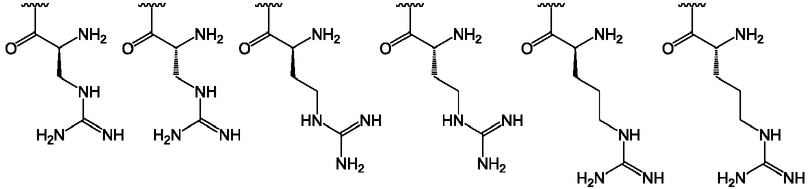

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

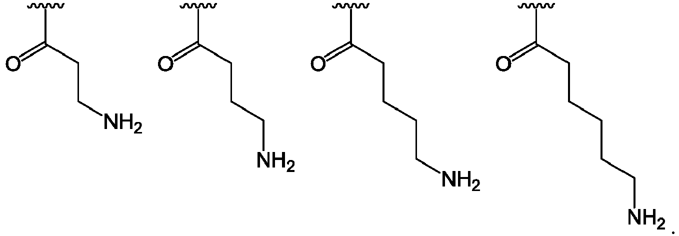

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

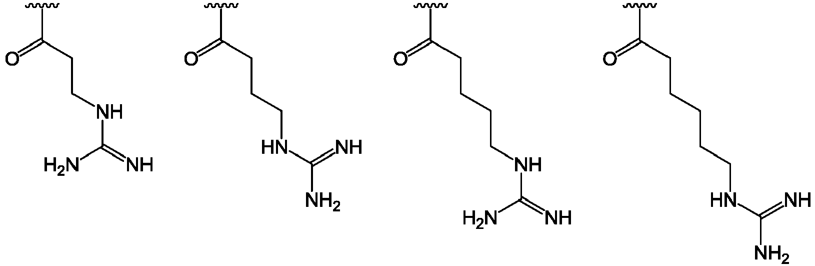

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In one embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the composition further comprises an additional agent, e.g., a pharmaceutical agent such as metronidazole, hydrogen peroxide, cetylpyridinium chloride, xylitol, or chlorhexidine, or a non-pharmaceutical agent (e.g., a non-toxic surfactant). In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the composition has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the composition has less than about 20%, 15%, 10%, 5%, 2%, or 1%, or is substantially free, of a chitosan polymer having a molecular weight of less than 15,000 Da, 10,000 Da, or 5,000 Da.

In another aspect, the disclosure features an oral rinse composition comprising a soluble derivatized chitosan described herein.

In one embodiment, the concentration of the soluble derivatized chitosan is at least 5, 10, 50, 100, 200, 300, 400, 500, or 1000 ppm.

In one embodiment, the concentration of the soluble derivatized chitosan is from about 10 to about 250 ppm.

In one embodiment, the concentration of the soluble derivatized chitosan is about 100 ppm.

In one embodiment, the concentration of the soluble derivatized chitosan is least 5, 10, 50, 100, 200, 300, 400, 500, or 1000 µg/ml.

In one embodiment, the composition further comprises an antiseptic agent, e.g., thymol.

In one embodiment, the composition further comprises an anesthetic agent, e.g., a local anesthetic agent (e.g., menthol).

In one embodiment, the composition further comprises a dissolving agent, e.g., ethanol.

In one embodiment, the composition further comprises a cleaning agent, e.g., methyl salicylate.

In one embodiment, the composition further comprises an anti-cavity agent, e.g., sodium fluoride.

In one embodiment, the composition further comprises a whitening agent, e.g., hydrogen peroxide.

In one embodiment, the composition further comprises an antibacterial or bubbling agent, e.g., hydrogen peroxide, e.g., to improve mechanical removal.

In one embodiment, the composition further comprises a desensitizing agent, e.g., potassium nitrate.

In one embodiment, the composition further comprises a coloring agent.

In one embodiment, the composition further comprises a flavoring agent.

In one embodiment, the composition has a pH at about 5.0, 5.5, 6.0, 6.5, 7.0, 7.5, 8.0, 8.5, or 9.0.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 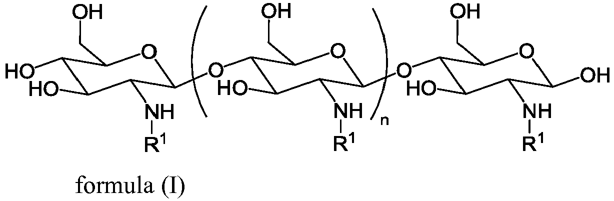 wherein:
n is an integer between 20 and 6000; and
each R¹ is independently selected for each occurrence from hydrogen, acetyl, and a group of formula (II): 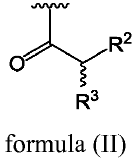 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, R¹ is selected from one of the following:

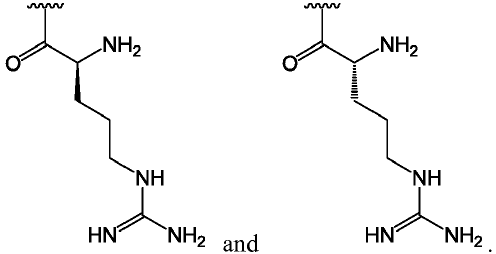

In one embodiment, R² is amino and R³ is a lysine side chain.

In one embodiment, R¹ is selected from one of the following:

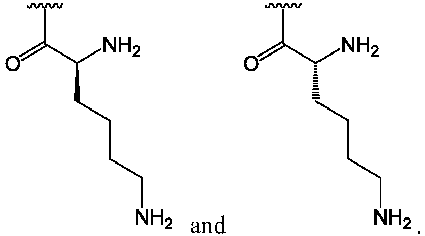

In one embodiment, R² is amino and R³ is a histidine side chain.

In one embodiment, R¹ is selected from one of the following:

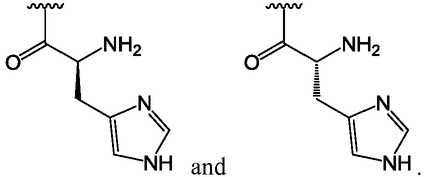

In one embodiment, at least 1% of R¹ substituents are selected from one of the following: 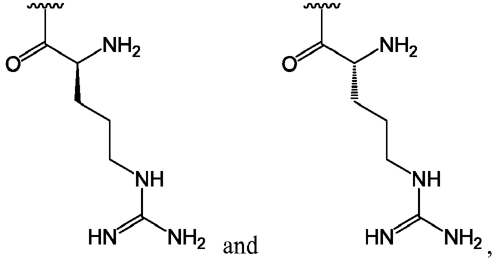 and at least 1% of R¹ substituents are selected from the following:

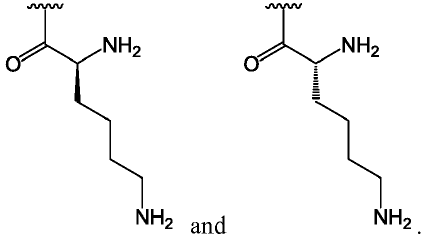

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

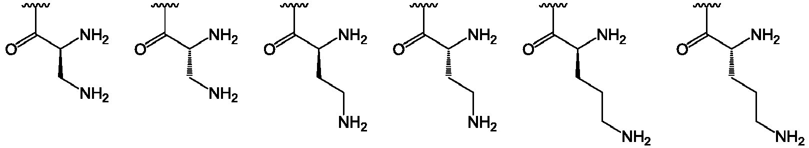

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

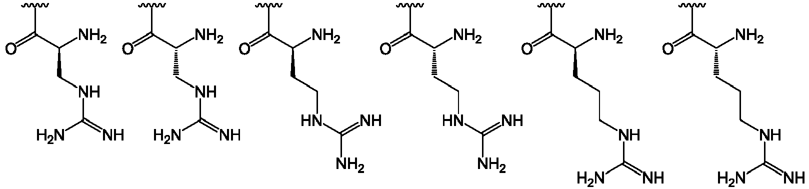

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

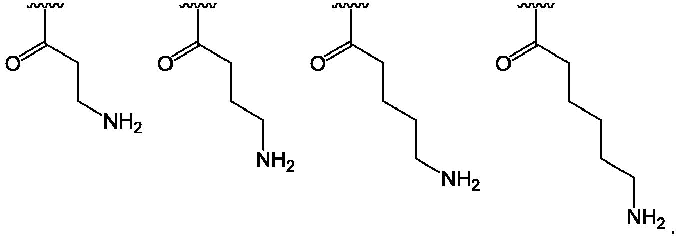

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

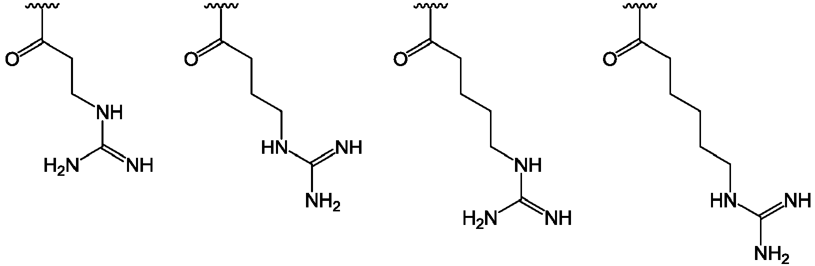

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In a preferred embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the composition further comprises an additional agent, e.g., a pharmaceutical agent such as metronidazole, hydrogen peroxide, cetylpyridinium chloride, xylitol, or chlorhexidine, or a non-pharmaceutical agent (e.g., a non-toxic surfactant). In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the composition has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the composition has less than about 20%, 15%, 10%, 5%, 2%, or 1%, or is substantially free, of a chitosan polymer having a molecular weight of less than 15,000 Da, 10,000 Da, or 5,000 Da.

In another aspect, the disclosure features a dentifrice composition comprising a soluble derivatized chitosan described herein.

In one embodiment, the concentration of the soluble derivatized chitosan is at least 5, 10, 50, 100, 200, 300, 400, 500, or 1000 ppm.

In one embodiment, the concentration of the soluble derivatized chitosan is from about 10 to about 250 ppm.

In one embodiment, the concentration of the soluble derivatized chitosan is about 100 ppm.

In one embodiment, the concentration of the soluble derivatized chitosan is least 5, 10, 50, 100, 200, 300, 400, 500, or 1000 µg/ml.

In one embodiment, the composition further comprises an antiseptic agent, e.g., thymol.

In one embodiment, the composition further comprises an anesthetic agent, e.g., a local anesthetic agent (e.g., menthol).

In one embodiment, the composition further comprises a dissolving agent, e.g., ethanol.

In one embodiment, the composition further comprises a cleaning agent, e.g., methyl salicylate.

In one embodiment, the composition further comprises an anti-cavity agent, e.g., sodium fluoride.

In one embodiment, the composition further comprises a whitening agent, e.g., hydrogen peroxide.

In one embodiment, the composition e further comprises an antibacterial or bubbling agent, e.g., hydrogen peroxide, e.g., to improve mechanical removal.

In one embodiment, the composition further comprises a desensitizing agent, e.g., potassium nitrate.

In one embodiment, the composition further comprises a coloring agent.

In one embodiment, the composition further comprises a flavoring agent.

In one embodiment, the composition has a pH at about 5.0, 5.5, 6.0, 6.5, 7.0, 7.5, 8.0, 8.5, or 9.0.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 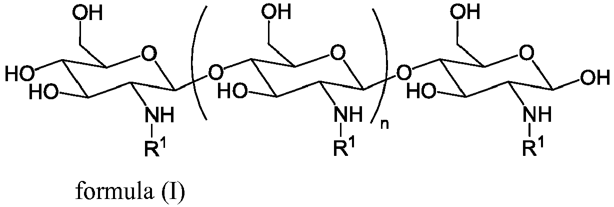 wherein:
n is an integer between 20 and 6000; and
each R¹ is independently selected for each occurrence from hydrogen, acetyl, and a group of formula (II): 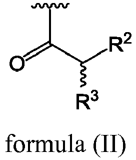 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, R¹ is selected from one of the following:

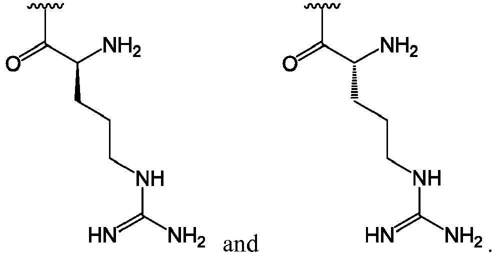

In one embodiment, R² is amino and R³ is a lysine side chain.

In one embodiment, R¹ is selected from one of the following:

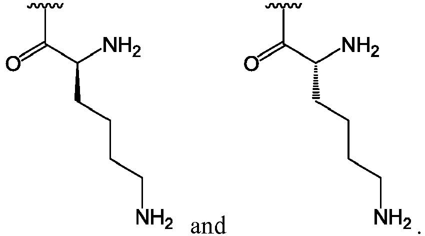

In one embodiment, R² is amino and R³ is a histidine side chain.

In one embodiment, R¹ is selected from one of the following:

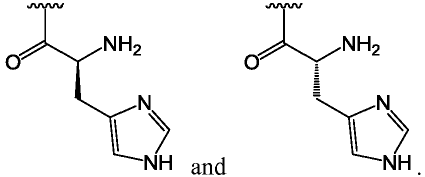

In one embodiment, at least 1% of R¹ substituents are selected from one of the following: 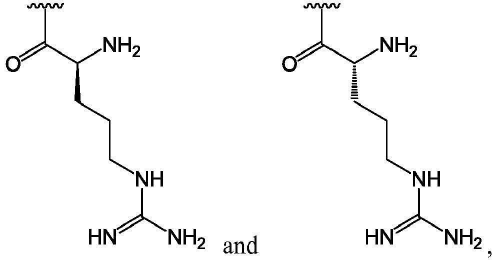 and at least 1% of R¹ substituents are selected from the following:

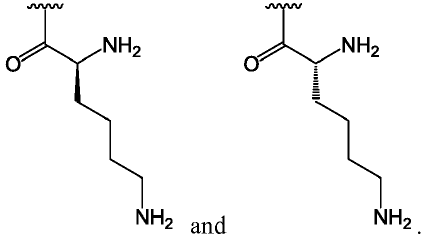

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

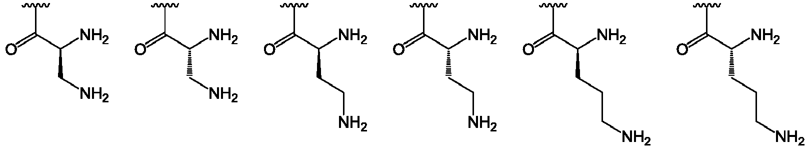

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

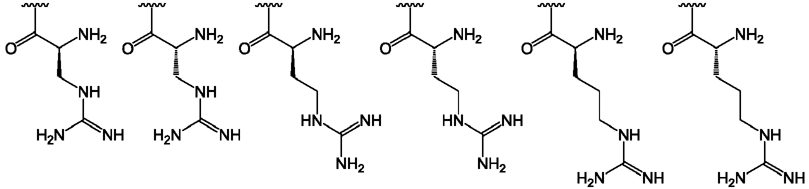

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

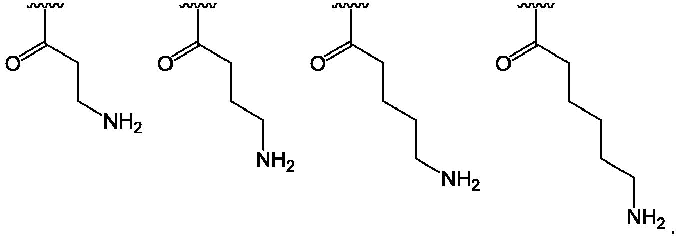

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

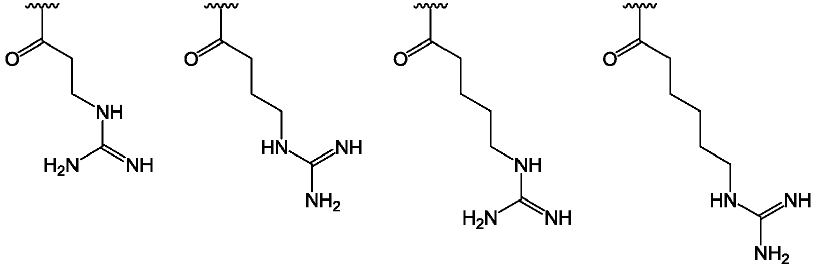

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In a preferred embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the composition further comprises an additional agent, e.g., a pharmaceutical agent such as metronidazole, hydrogen peroxide, cetylpyridinum chloride, xylitol, or chlorhexidine, or a non-pharmaceutical agent (e.g., a non-toxic surfactant). In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the composition has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the composition has less than about 20%, 15%, 10%, 5%, 2%, or 1%, or is substantially free, of a chitosan polymer having a molecular weight of less than 15,000 Da, 10,000 Da, or 5,000 Da.

In another aspect, the disclosure features a gel, liquid, or semisolid composition (e.g., a slow dissolving gel, liquid, or semisolid composition, e.g., can be used in tooth strip) comprising a soluble derivatized chitosan described herein.

In one embodiment, the concentration of the soluble derivatized chitosan is at least 5, 10, 50, 100, 200, 377, 400, 500, or 1000 ppm.

In one embodiment, the concentration of the soluble derivatized chitosan is from about 10 to about 100 ppm10 to about 250 ppm.

In one embodiment, the concentration of the soluble derivatized chitosan is about 100 ppm.

In one embodiment, the concentration of the soluble derivatized chitosan is least 5, 10, 50, 100, 200, 377, 400, 500, or 1000 µg/ml.

In one embodiment, the composition further comprises an antiseptic agent, e.g., thymol.

In one embodiment, the composition further comprises an anesthetic agent, e.g., a local anesthetic agent (e.g., menthol).

In one embodiment, the composition further comprises a dissolving agent, e.g., ethanol.

In one embodiment, the composition further comprises a cleaning agent, e.g., methyl salicylate.

In one embodiment, the composition further comprises an anti-cavity agent, e.g., sodium fluoride.

In one embodiment, the composition further comprises a whitening agent, e.g., hydrogen peroxide.

In one embodiment, the composition further comprises an antibacterial or bubbling agent, e.g., hydrogen peroxide, e.g., to improve mechanical removal.

In one embodiment, the composition further comprises a desensitizing agent, e.g., potassium nitrate.

In one embodiment, the composition further comprises a coloring agent.

In one embodiment, the composition further comprises a flavoring agent.

In one embodiment, the composition has a pH at about 5.0, 5.5, 6.0, 6.5, 7.0, 7.5, 8.0, 8.5, or 9.0.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 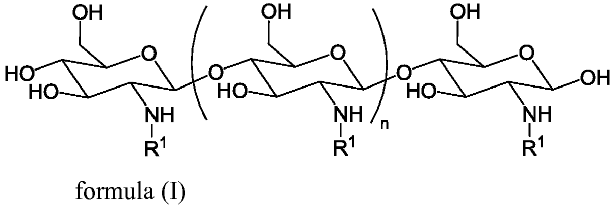 wherein:
n is an integer between 20 and 6000; and
each R¹ is independently selected for each occurrence from hydrogen, acetyl, and a group of formula (II): 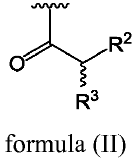 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, R¹ is selected from one of the following:

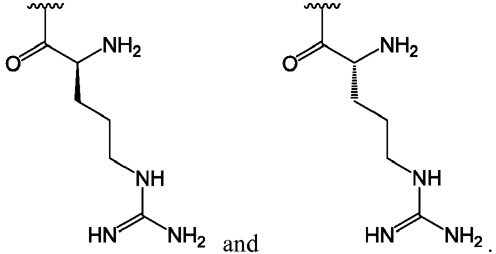

In one embodiment, R² is amino and R³ is a lysine side chain.

In one embodiment, R¹ is selected from one of the following:

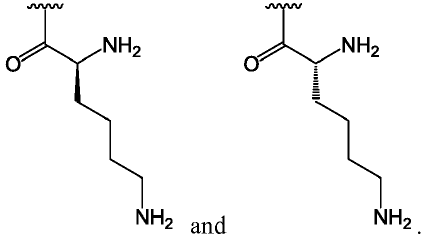

In one embodiment, R² is amino and R³ is a histidine side chain.

In one embodiment, R¹ is selected from one of the following:

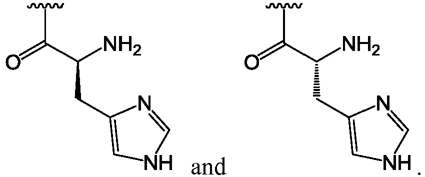

In one embodiment, at least 1% of R¹ substituents are selected from one of the following: 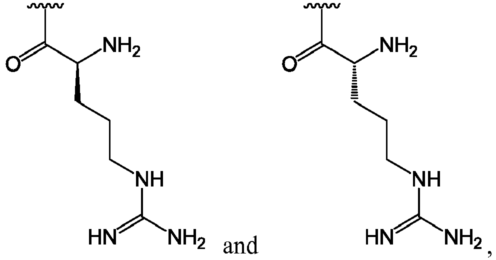 and at least 1% of R¹ substituents are selected from the following:

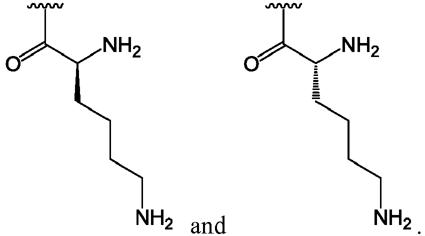

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

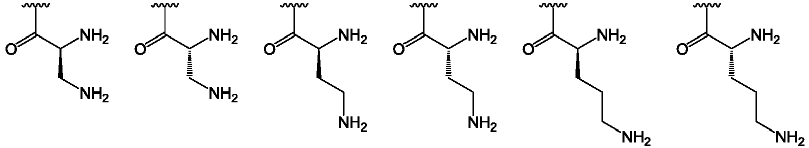

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

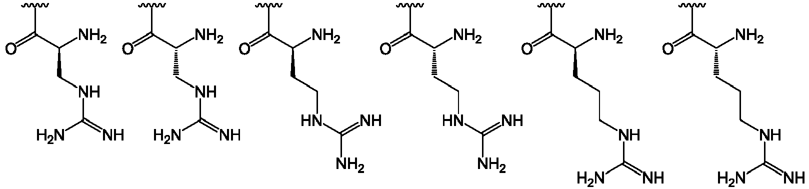

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

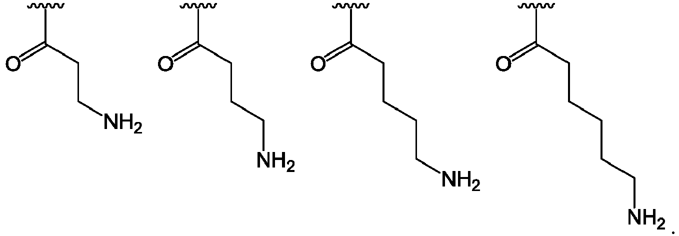

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

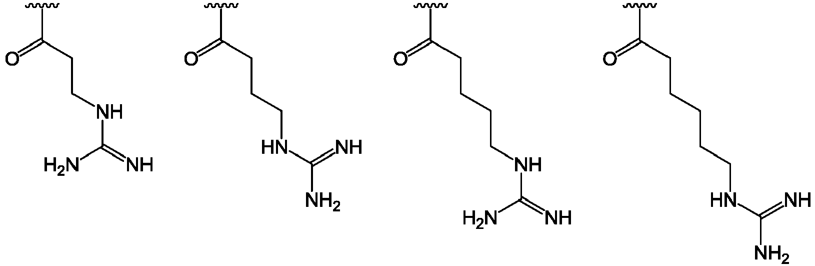

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In a preferred embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the composition further comprises an additional agent, e.g., a pharmaceutical agent such as metronidazole, hydrogen peroxide, cetylpyridinium chloride, xylitol, or chlorhexidine, or a non-pharmaceutical agent (e.g., a non-toxic surfactant). In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the composition has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the composition has less than about 20%, 15%, 10%, 5%, 2%, or 1%, or is substantially free, of a chitosan polymer having a molecular weight of less than 15,000 Da, 10,000 Da, or 5,000 Da.

In another aspect, the disclosure features an oral rinse kit comprising a soluble derivatized chitosan described herein and instructions to reduce bacteria or disrupt a biofilm in the mouth of a subject.

In one embodiment, the concentration of the soluble derivatized chitosan is at least 5, 10, 50, 100, 200, 300, 400, 500, or 1000 ppm.

In one embodiment, the concentration of the soluble derivatized chitosan is from about 10 to about 250 ppm.

In one embodiment, the concentration of the soluble derivatized chitosan is about 100 ppm.

In one embodiment, the concentration of the soluble derivatized chitosan is least 5, 10, 50, 100, 200, 300, 400, 500, or 1000 µg/ml.

In one embodiment, the kit further comprises an antiseptic agent, e.g., thymol.

In one embodiment, the kit further comprises an anesthetic agent, e.g., a local anesthetic agent (e.g., menthol).

In one embodiment, the kit further comprises a dissolving agent, e.g., ethanol.

In one embodiment, the kit further comprises a cleaning agent, e.g., methyl salicylate.

In one embodiment, the kit further comprises an anti-cavity agent, e.g., sodium fluoride.

In one embodiment, the kit further comprises a whitening agent, e.g., hydrogen peroxide.

In one embodiment, the kit further comprises an antibacterial or bubbling agent, e.g., hydrogen peroxide, e.g., to improve mechanical removal.

In one embodiment, the kit further comprises a desensitizing agent, e.g., potassium nitrate.

In one embodiment, the kit further comprises a coloring agent.

In one embodiment, the kit further comprises a flavoring agent.

In one embodiment, the derivatized chitosan has a pH at about 5.0, 5.5, 6.0, 6.5, 7.0, 7.5, 8.0, 8.5, or 9.0.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 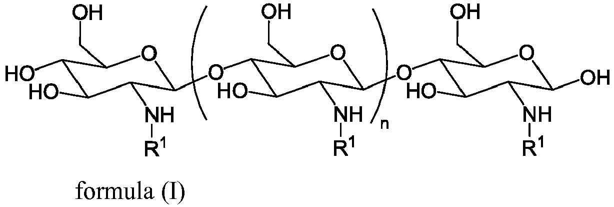 wherein:
n is an integer between 20 and 6000; and
each R¹ is independently selected for each occurrence from hydrogen, acetyl, and a group of formula (II): 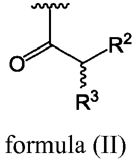 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, R¹ is selected from one of the following:

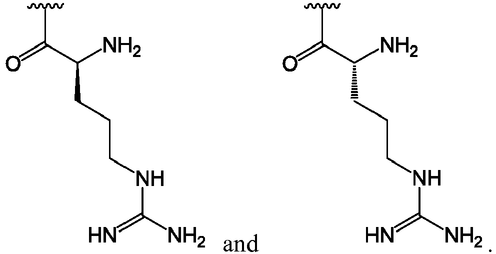

In one embodiment, R² is amino and R³ is a lysine side chain.

In one embodiment, R¹ is selected from one of the following:

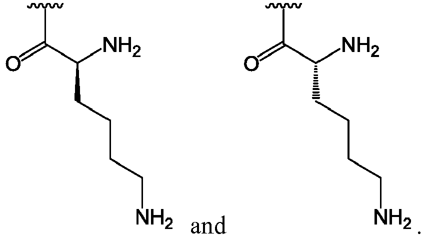

In one embodiment, R² is amino and R³ is a histidine side chain.

In one embodiment, R¹ is selected from one of the following:

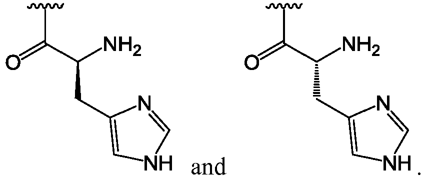

In one embodiment, at least 1% of R¹ substituents are selected from one of the following: 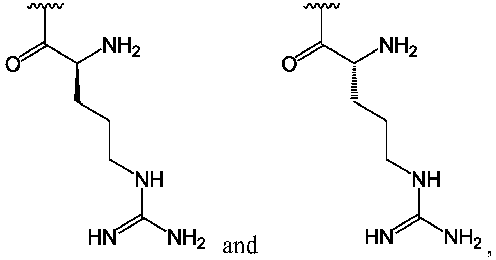 and at least 1% of R¹ substituents are selected from the following:

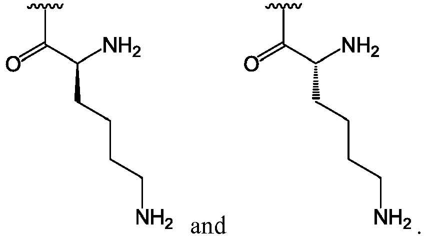

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

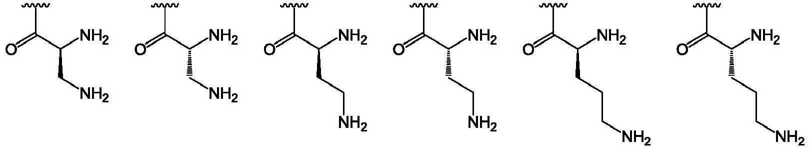

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

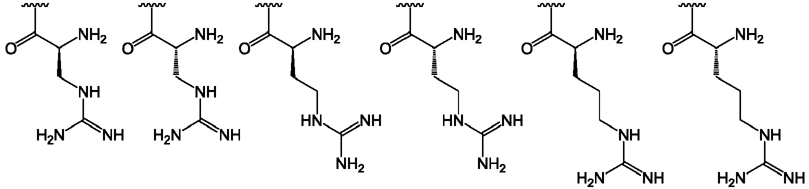

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

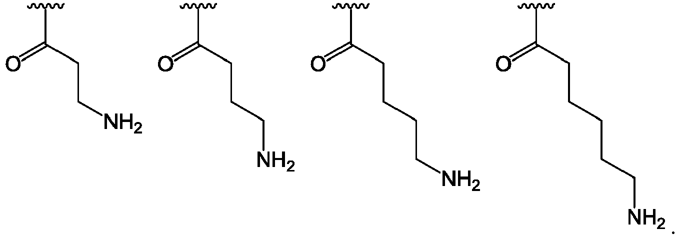

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

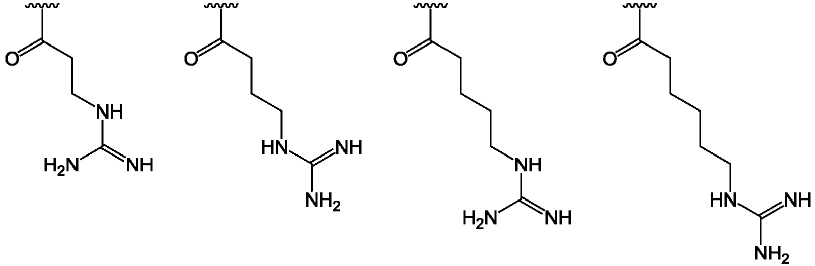

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In a preferred embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the kit further comprises an additional agent, e.g., a pharmaceutical agent such as metronidazole, hydrogen peroxide, cetylpyridinium chloride, xylitol, or chlorhexidine, or a non-pharmaceutical agent (e.g., a non-toxic surfactant). In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the kit has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the kit has less than about 20%, 15%, 10%, 5%, 2%, or 1%, or is substantially free, of a chitosan polymer having a molecular weight of less than 15,000 Da, 10,000 Da, or 5,000 Da.

In another aspect, the disclosure features a medical device constructed to fit into the mouth of a subject, e.g., to contact front and back surfaces of the teeth, the medical device comprising a soluble derivatized chitosan described herein. In one embodiment, the surface of the device is coated with a soluble derivatzed chitosan.

In one embodiment, the concentration of the soluble derivatized chitosan is at least 5, 10, 50, 100, 200, 300, 400, 500, or 1000 ppm.

In one embodiment, the concentration of the soluble derivatized chitosan is from about 10 to about 250 ppm.

In one embodiment, the concentration of the soluble derivatized chitosan is about 100 ppm.

In one embodiment, the concentration of the soluble derivatized chitosan is least 5, 10, 50, 100, 200, 300, 400, 500, or 1000 µg/ml.

In one embodiment, the medical device further comprises an antiseptic agent, e.g., thymol.

In one embodiment, the medical device further comprises an anesthetic agent, e.g., a local anesthetic agent (e.g., menthol).

In one embodiment, the medical device further comprises a dissolving agent, e.g., ethanol.

In one embodiment, the medical device further comprises a cleaning agent, e.g., methyl salicylate.

In one embodiment, the medical device further comprises an anti-cavity agent, e.g., sodium fluoride.

In one embodiment, the medical device further comprises a whitening agent, e.g., hydrogen peroxide.

In one embodiment, the medical device further comprises an antibacterial or bubbling agent, e.g., hydrogen peroxide, e.g., to improve mechanical removal.

In one embodiment, the medical device further comprises a desensitizing agent, e.g., potassium nitrate.

In one embodiment, the medical device further comprises a coloring agent.

In one embodiment, the medical device further comprises a flavoring agent.

In one embodiment, the composition has a pH at about 5.0, 5.5, 6.0, 6.5, 7.0, 7.5, 8.0, 8.5, or 9.0.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 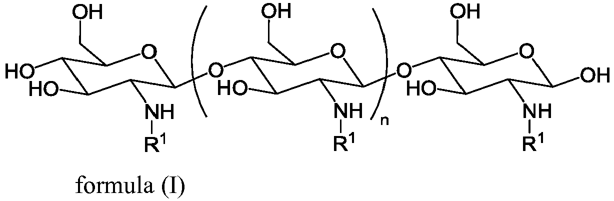 wherein:
n is an integer between 20 and 6000; and
each R¹ is independently selected for each occurrence from hydrogen, acetyl, and a group of formula (II): 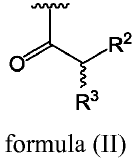 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, R¹ is selected from one of the following:

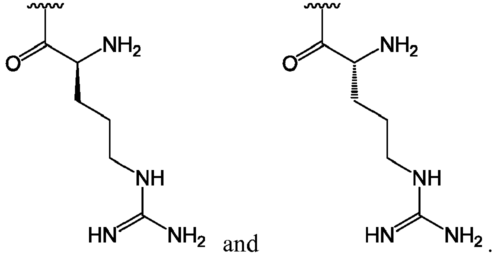

In one embodiment, R² is amino and R³ is a lysine side chain.

In one embodiment, R¹ is selected from one of the following:

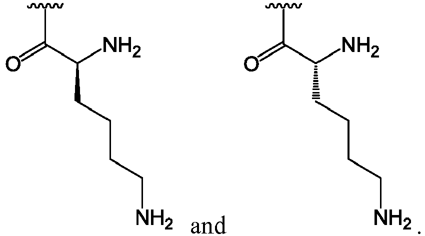

In one embodiment, R² is amino and R³ is a histidine side chain.

In one embodiment, R¹ is selected from one of the following:

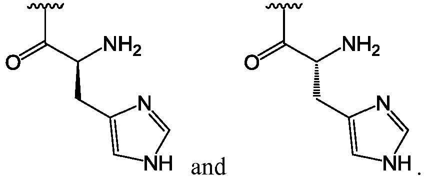

In one embodiment, at least 1% of R¹ substituents are selected from one of the following: 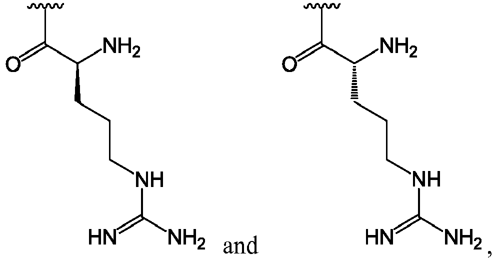 and at least 1% of R¹ substituents are selected from the following:

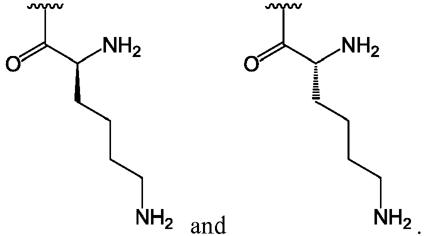

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

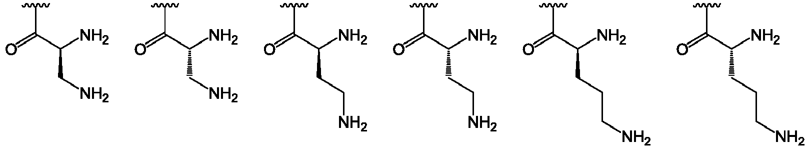

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

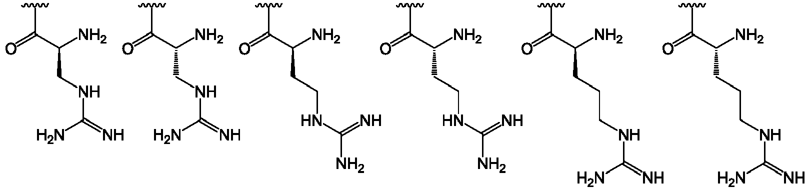

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

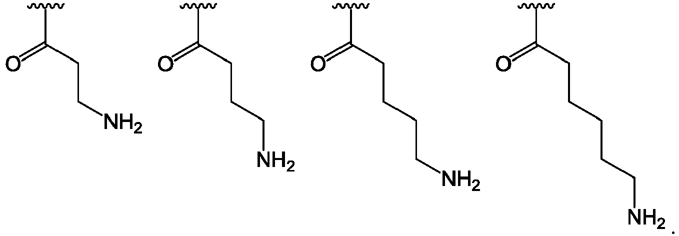

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

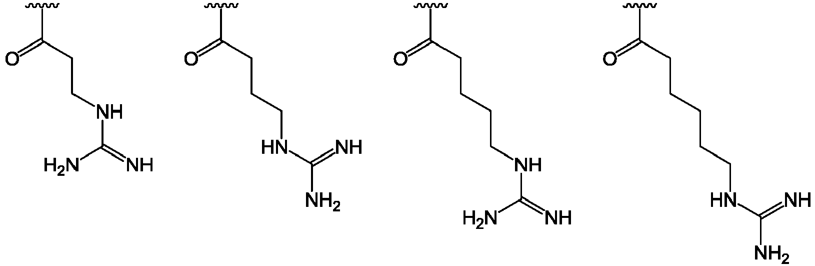

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In a preferred embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the medical device further comprises an additional agent, e.g., a pharmaceutical agent such as metronidazole, hydrogen peroxide, cetylpyridinium chloride, xylitol, or chlorhexidine, or a non-pharmaceutical agent (e.g., a non-toxic surfactant). In one embodiment, the surface of the device is coated with the second agent. In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the composition has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the composition has less than about 20%, 15%, 10%, 5%, 2%, or 1%, or is substantially free, of a chitosan polymer having a molecular weight of less than 15,000 Da, 10,000 Da, or 5,000 Da.

In another aspect, the disclosure features a toothbrush comprising a soluble derivatized chitosan described herein.

In one embodiment, the concentration of the soluble derivatized chitosan is at least 5, 10, 50, 100, 200, 300, 400, 500, or 1000 ppm.

In one embodiment, the concentration of the soluble derivatized chitosan is from about 10 to about 250 ppm.

In one embodiment, the concentration of the soluble derivatized chitosan is about 100 ppm.

In one embodiment, the concentration of the soluble derivatized chitosan is least 5, 10, 50, 100, 200, 300, 400, 500, or 1000 µg/ml.

In one embodiment, the toothbrush further comprises an antiseptic agent, e.g., thymol.

In one embodiment, the toothbrush further comprises an anesthetic agent, e.g., a local anesthetic agent (e.g., menthol).

In one embodiment, the toothbrush further comprises a dissolving agent, e.g., ethanol.

In one embodiment, the toothbrush further comprises a cleaning agent, e.g., methyl salicylate.

In one embodiment, the toothbrush further comprises an anti-cavity agent, e.g., sodium fluoride.

In one embodiment, the toothbrush further comprises a whitening agent, e.g., hydrogen peroxide.

In one embodiment, the toothbrush further comprises an antibacterial or bubbling agent, e.g., hydrogen peroxide, e.g., to improve mechanical removal.

In one embodiment, the toothbrush further comprises a desensitizing agent, e.g., potassium nitrate.

In one embodiment, the toothbrush further comprises a coloring agent.

In one embodiment, the toothbrush further comprises a flavoring agent.

In one embodiment, the composition has a pH at about 5.0, 5.5, 6.0, 6.5, 7.0, 7.5, 8.0, 8.5, or 9.0.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 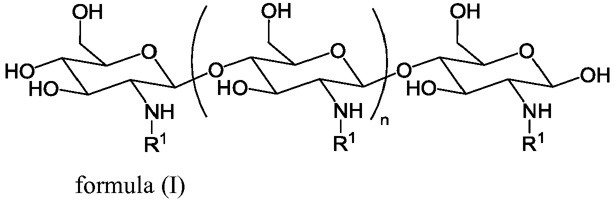 wherein:
n is an integer between 20 and 6000; and
each R¹ is independently selected for each occurrence from hydrogen, acetyl, and a group of formula (II): 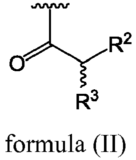 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, R¹ is selected from one of the following:

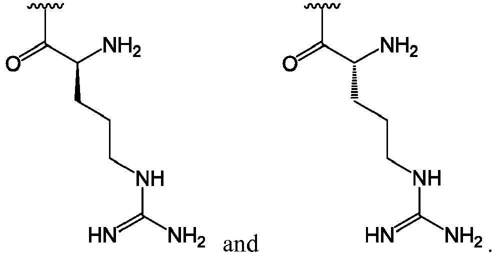

In one embodiment, R² is amino and R³ is a lysine side chain.

In one embodiment, R¹ is selected from one of the following:

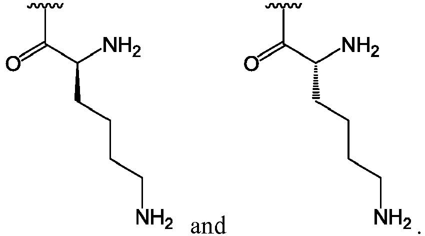

In one embodiment, R² is amino and R³ is a histidine side chain.

In one embodiment, R¹ is selected from one of the following:

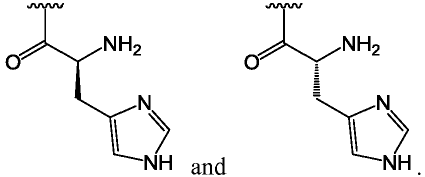

In one embodiment, at least 1% of R¹ substituents are selected from one of the following: 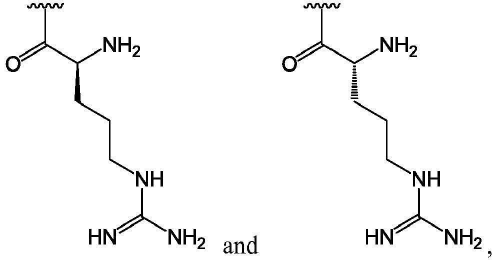 and at least 1% of R¹ substituents are selected from the following:

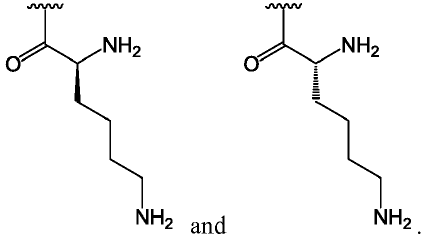

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

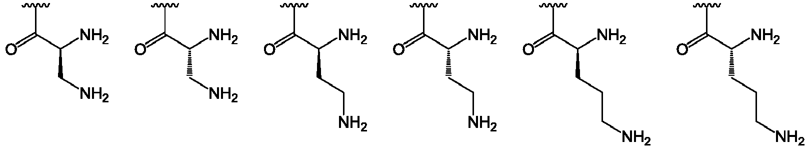

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

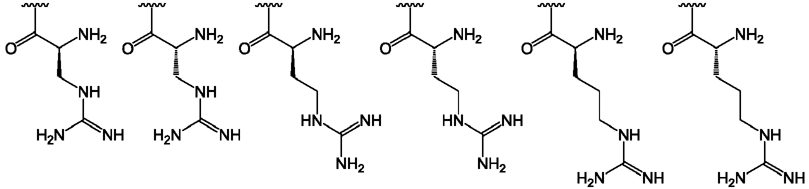

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

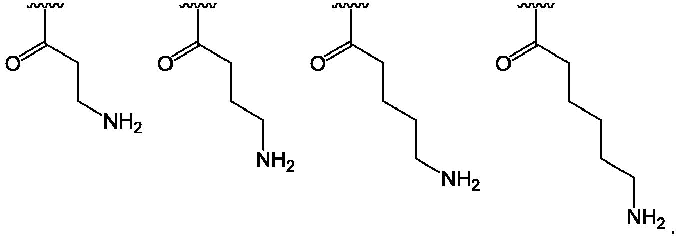

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

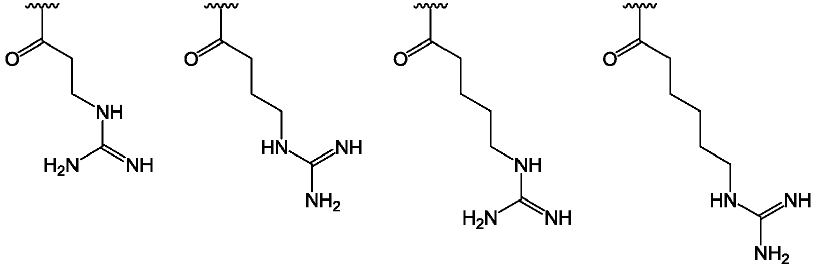

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In a preferred embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the tooth brush further comprises an additional agent, e.g., a pharmaceutical agent such as metronidazole, hydrogen peroxide, cetylpyridinium chloride, xylitol, or chlorhexidine, or a non-pharmaceutical agent (e.g., a non-toxic surfactant). In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the composition has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the composition has less than about 20%, 15%, 10%, 5%, 2%, or 1%, or is substantially free, of a chitosan polymer having a molecular weight of less than 15,000 Da, 10,000 Da, or 5,000 Da.

In another aspect, the disclosure features a dental floss (e.g., in the waxy layer) comprising a soluble derivatized chitosan described herein.

In one embodiment, the concentration of the soluble derivatized chitosan is at least 5, 10, 50, 100, 200, 300, 400, 500, or 1000 ppm.

In one embodiment, the concentration of the soluble derivatized chitosan is from about 10 to about 250 ppm.

In one embodiment, the concentration of the soluble derivatized chitosan is about 100 ppm.

In one embodiment, the concentration of the soluble derivatized chitosan is least 5, 10, 50, 100, 200, 300, 400, 500, or 1000 µg/ml.

In one embodiment, the dental floss further comprises an antiseptic agent, e.g., thymol.

In one embodiment, the dental floss further comprises an anesthetic agent, e.g., a local anesthetic agent (e.g., menthol).

In one embodiment, the dental floss further comprises a dissolving agent, e.g., ethanol.

In one embodiment, the dental floss further comprises a cleaning agent, e.g., methyl salicylate.

In one embodiment, the dental floss further comprises an anti-cavity agent, e.g., sodium fluoride.

In one embodiment, the dental floss further comprises a whitening agent, e.g., hydrogen peroxide.

In one embodiment, the dental floss further comprises an antibacterial or bubbling agent, e.g., hydrogen peroxide, e.g., to improve mechanical removal.

In one embodiment, the dental floss further comprises a desensitizing agent, e.g., potassium nitrate.

In one embodiment, the dental floss further comprises a coloring agent.

In one embodiment, the dental floss further comprises a flavoring agent.

In one embodiment, the composition has a pH at about 5.0, 5.5, 6.0, 6.5, 7.0, 7.5, 8.0, 8.5, or 9.0.

In one embodiment, the derivatized chitosan is soluble in aqueous solution from about pH 6.8 to about pH 7.4.

In one embodiment, the derivatized chitosan is soluble in aqueous solution from about pH 3 to about pH 9.

In one embodiment, the derivatized chitosan comprises a chitosan of the following formula (I): 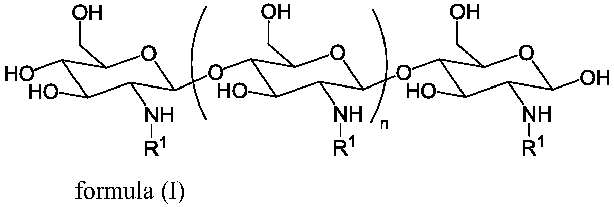 wherein:
n is an integer between 20 and 6000; and
each R¹ is independently selected for each occurrence from hydrogen, acetyl, and a group of formula (II): 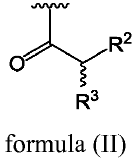 or R¹, when taken together with the nitrogen to which it is attached, forms a guanidine moiety,
wherein R² is hydrogen or amino; and
R³ is amino, guanidino, C₁-C₆ alkyl substituted with an amino or guanidino moiety, or a natural or unnatural amino acid side chain,
wherein at least 25% of R¹ substituents are H, at least 1% of R¹ substituents are acetyl, and at least 2% of R¹ substituents are a group of formula (II).

In one embodiment, between 25-95% of R¹ substituents are hydrogen.

In one embodiment, between 55-90% of R¹ substituents are hydrogen.

In one embodiment, between 1-50% of R¹ substituents are acetyl.

In one embodiment, between 4-20% of R¹ substituents are acetyl.

In one embodiment, between 2-50% of R¹ substituents are a group of formula (II).

In one embodiment, between 4-30% of R¹ substituents are a group of formula (II).

In one embodiment, 55-90% of R¹ substituents are hydrogen, 4-20% of R¹ substituents are acetyl, 4-30% of R¹ substituents are a group of formula (II).

In one embodiment, R² is amino and R³ is an arginine side chain.

In one embodiment, R¹ is selected from one of the following:

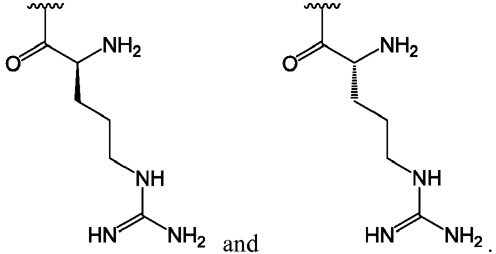

In one embodiment, R² is amino and R³ is a lysine side chain.

In one embodiment, R¹ is selected from one of the following:

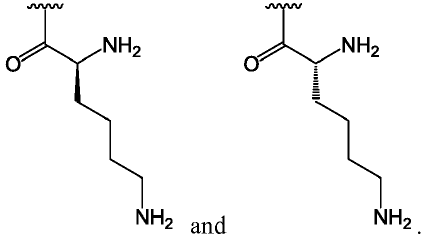

In one embodiment, R² is amino and R³ is a histidine side chain.

In one embodiment, R¹ is selected from one of the following:

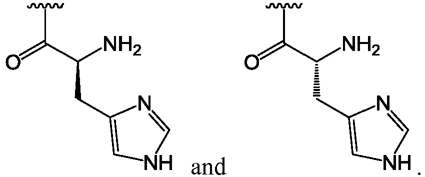

In one embodiment, at least 1% of R¹ substituents are selected from one of the following: 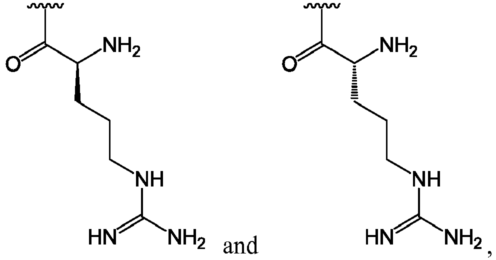 and at least 1% of R¹ substituents are selected from the following:

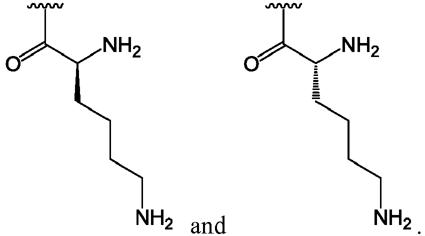

In one embodiment, R² is amino and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

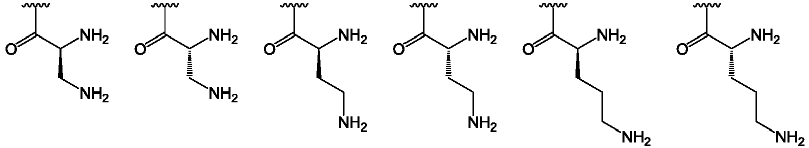

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

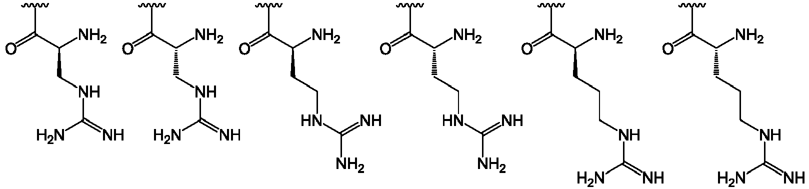

In one embodiment, R² is amino that is substituted with a nitrogen protecting group prior to substitution on chitosan and removed subsequent to substitution on chitosan.

In one embodiment, the nitrogen protecting group is *tert*-butyloxycarbonyl (Boc).

In one embodiment, the derivatized chitosan is made by reacting a chitosan (e.g., a free amino group of one or more of glucosamine monomers of the chitosan) with an amino acid (e.g., a carboxylic acid moiety of the amino acid) wherein the amino group of the amino acid is protected by a protecting group (e.g., Boc). The protecting group can be removed, e.g., by exposure to acid of pH < 3, after the synthesis.

In one embodiment, in the synthetic process a nitrogen protecting group is used, which can provide an intermediate polymer having a nitrogen protecting group such as Boc.

In one embodiment, R² is amino.

In one embodiment, R² is hydrogen and R³ is amino.

In one embodiment, R² is hydrogen and R³ is guanidino.

In one embodiment, R² is hydrogen and R³ is a substituted C₁-C₆ alkyl.

In one embodiment, R³ is C₁-C₆ alkyl substituted with an amino group.

In one embodiment, R³ is C₁ alkyl substituted with an amino group.

In one embodiment, R³ is C₂ alkyl substituted with an amino group.

In one embodiment, R³ is C₃ alkyl substituted with an amino group.

In one embodiment, R³ is C₄ alkyl substituted with an amino group.

In one embodiment, R³ is C₅ alkyl substituted with an amino group.

In one embodiment, R³ is C₆ alkyl substituted with an amino group.

In one embodiment, R¹ is selected from one of the following:

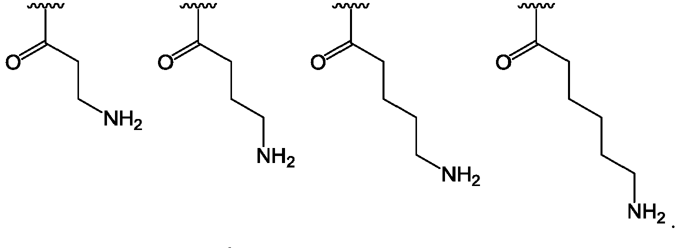

In one embodiment, R³ is C₁-C₆ alkyl substituted with a guanidino group.

In one embodiment, R³ is C₁ alkyl substituted with a guanidino group.

In one embodiment, R³ is C₂ alkyl substituted with a guanidino group.

In one embodiment, R³ is C₃ alkyl substituted with a guanidino group.

In one embodiment, R³ is C₄ alkyl substituted with a guanidino group.

In one embodiment, R³ is C₅ alkyl substituted with a guanidino group.

In one embodiment, R³ is C₆ alkyl substituted with a guanidino group.

In one embodiment, R¹ is selected from one of the following:

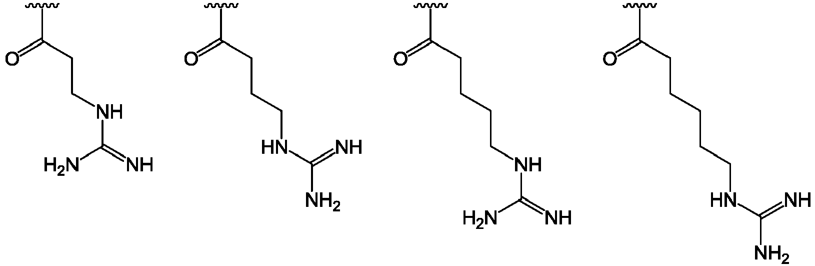

In one embodiment, at least 25% of R¹ substituents are H, at least 1% of R¹ substituents are acetyl, and at least 2% of R¹ substituents independently selected from any of the formulae specifically shown above.

In one embodiment, the chitosan of formula (I) may be further derivatized on the free hydroxyl moieties.

In one embodiment, the molecular weight of the derivatized chitosan is between 5,000 and 1,000,000 Da.

In one embodiment, the molecular weight of the derivatized chitosan is between 5,000 and 350,000 Da.

In one embodiment, the molecular weight of the derivatized chitosan is between 10,000 and 150,000 Da.

In one embodiment, the molecular weight of the derivatized chitosan is between 15,000 and 100,000 Da.

In one embodiment, the molecular weight of the derivatized chitosan is between 15,000 and 50,000 Da.

In one embodiement, the molecular weight of the derivatized chitosan is between 20,000 and 40,000.

In one embodiment, the chitosan is functionalized at between 5% and 50%.

In a preferred embodiment, the chitosan is functionalized at between 20% and 30%.

In one embodiment, the degree of deacetylation (%DDA) of the derivatized chitosan is between 75% and 95%.

In one embodiment, the degree of deacetylation (%DDA) of the derivatized chitosan is between 80% and 90%.

In one embodiment, the polydispersity index (PDI) of the derivatized chitosan is between 1.0 and 2.5.

In one embodiment, the polydispersity index (PDI) of the derivatized chitosan is between 1.5 and 2.0.

In one embodiment, the dental floss further comprises an additional agent, e.g., a pharmaceutical agent such as metronidazole, hydrogen peroxide, cetylpyridinium chloride, xylitol, or chlorhexidine, or a non-pharmaceutical agent (e.g., a non-toxic surfactant). In one embodiment, the second agent comprises another chitosan derivative, e.g., another chitosan derivative described herein.

In one embodiment, the functionalized chitosan is substantially free of other impurities, e.g., salt, e.g., NaCl.

In one embodiment, the dental floss has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In one embodiment, the dental floss has less than about 20%, 15%, 10%, 5%, 2%, or 1%, or is substantially free, of a chitosan polymer having a molecular weight of less than 15,000 Da, 10,000 Da, or 5,000 Da.

### Brief Description of the Drawings

**Figure 1A** depicts *Streptococcus mutans* control (A) in water alone.
**Figures 1B** and **1C** depict *S. mutans* aggregation after 1-minute treatment with 10 µg/mL of chitosan-arginine in water.
Figure 2A depicts *Streptococcus mutans* before treatment with chitosan-arginine.
**Figure 2B** depicts *Streptococcus mutans* following 1-minute treatment with chitosan-arginine at 10 µg/mL.
**Figure 2C** depicts *Streptococcus mutans* following 1-minute treatment with chitosan-arginine at 100 µg/mL.
**Figure 3** depicts *Streptococcus mutans* stained with Rhodamine conjugate as a control.
**Figure 4** depicts viability of *Streptococcus mutans* after exposure to 10 µg/mL of chitosan-arginine.
**Figure 5** depicts removal of *Streptococcus mutans* biofilms after 1-minute treatment with chitosan-arginine. The p value was equal to 0.04 (*) or 0.02 (**) compared to water treatment alone.
**Figure 6(A)** depicts *Streptococcus mutans* plaque on human molars treated with water.
**Figure 6(B)** depicts *Streptococcus mutans* plaque on human molars treated with 10 µg/mL of chitosan-arginine.
**Figure 6(C)** depicts *Streptococcus mutans* plaque on human molars treated with 100 µg/mL of chitosan-arginine.
**Figure 7** depicts quantification of dental plaque using dental dye. Dye was release from the tooth surface with sonication and vortex (*) indicates p value of 0.04 compared to control water treatment.
**Figure 8** depicts plaque stained on hman molars treated with chitosan-arginine.
**Figure 9** depicts quantification of dental plaque using dental dye. Dye was released from the crown surface with ethanol. (*) indicates p value of 0.01 compared to control water treatment.
**Figure 10** shows the ability of chitosan-arginine (24 kDa, 28% functionalized, 83 %DDA, 1.5 PDI) to remove *Streptococcus mutans* (ATCC 35668) 4-day old biofilms compared to delmopinol. *Streptococcus mutans* biofilms were rinsed and strained with Crystal Violet before treatment with 0.01 0r 0.2% chitosan-arginine or Delmopinol (DP). Residual stain was removed with 70% ethanol and OD 595 was recorded to quantify remaining biofilm material.
**Figure 11** depicts the immediate dispersal of *Streptococcus mutans* ATCC 35668 2-day old stationary biofilms treated with 100 µg/mL chitosan-arginine (43kDa, 25% functionalized, 88% DDA, 2.28 PDI), water, or 1.2% Chlorhexidine. The biofilms were rinsed, stained with crystal violet and treated for 1 minute and rinsed.
**Figure 12** shows plaque removal activity from a tooth with 2-day plaque growth *(Streptococcus mutans* ATCC 35668) treated with chitosan-arginine (43kDa, 25% functionalized, 88% DDA, 2.28 PDI, on left) stained to visualize plaque. The middle images show the plaque remaining after gentle agitation in 10 µg/ml chitosan-arginine for 1 minute. The bottom image shows the plaque remaining after further treatment with 100 µg/ml chitosan-arginine for 1 minute.
**Figure 13** shows the aggregation of *Streptococcus mutans* ATCC 35668 visualized with Light microscope (400x) examined before **(A)** and after treatment with either 20 µg/ml **(B)** or 100 µg/ml **(C)** of chitosan derivative (Low= chitosan-arginine 18kDa, 25% functionalized, 88% DDA, 1.47 PDI; C/A High=chitosan-arginine 43kDa, 25% functionalized, 88% DDA, 2.28 PDI; LBA Low=chitosan-lactobionic acid 9-30 kDa; LBA High=>150kDa).
**Figure 14** shows *Streptococcus mutans* ATCC 35668 treated with chitosan derivatives (C/A Low= chitosan-arginine 18kDa, 25% functionalized, 88% DDA, 1.47 PDI; C/A High=chitosan-arginine 43kDa, 25% functionalized, 88% DDA, 2.28 PDI; LBA Low=chitosan-lactobionic acid 9-30 kDa; LBA High=>150kDa) for 5 minutes. The supernatant was isolated and tested to determine the ATP concentration the fold-increase of ATP released into the supernatant compared to untreated bacteria was measured.
**Figure 15** shows the viability of *Streptococcus mutans* ATCC 35668 treated with chitosan derivatives (C/A Low= chitosan-arginine 18kDa, 25% functionalized, 88% DDA, 1.47 PDI; C/A High=chitosan-arginine 43kDa, 25% functionalized, 88% DDA, 2.28 PDI; LBA Low=chitosan-lactobionic acid 9-30 kDa; LBA High=>150kDa) for 5 min to determine the relative antibacterial activity of the chitosan derivatives with respect to ATP leakage (see Figure 14).
**Figure 16** shows the remaining CFU/ml of *Streptococcus mutans* ATCC 35668 associated with the 2-day peg biofilm following 4-hour treatment with C/A High (chitosan-arginine 43kDa, 25% functionalized, 88% DDA, 2.28 PDI).
**Figure 17** shows the remaining CFU/ml of *Streptococcus mutans* ATCC 35668 associated with the 2-day peg biofilm following 4-hour treatment with C/A Low (chitosan-arginine 18kDa, 25% functionalized, 88% DDA, 1.47 PDI).
**Figure 18** depicts mixed oral biofilms (*Streptococcus mutans* ATCC 35668, *Streptococcus sanguinis* ATCC 10556, and *Streptococcus salivarius* ATCC 13419) grown in a flow cell for 30 hours then treated with either water or 100 µg/mL chitosan-arginine (43kDa, 25% functionalized, 88% DDA, 2.28 PDI) for two minutes at 8, 24, and 28 hours post bacterial attachment and finally rinsed and sonicated for 30 seconds.
**Figure 19** shows reduction of flow cell mixed oral biofilm (*Streptococcus mutans* ATCC 35668, *Streptococcus sanguinis* ATCC 10556, and *Streptococcus salivarius* ATCC 13419) wet weight of C/A High (chitosan-arginine 43kDa, 25% functionalized, 88% DDA, 2.28 PDI) treated and untreated before and after sonication.
**Figure 20** shows human third molars before treatment (A) and after exposure to mixed oral bacteria (*Streptococcus mutans* ATCC 35668, *Streptococcus sanguinis* ATCC 10556, and *Streptococcus salivarius* ATCC 13419) in the artificial mouth model without (B) and with (C) 2 minute twice daily 30 second treatment with C/A High (chitosan-arginine 43kDa, 25% functionalized, 88% DDA, 2.28 PDI).

### Detailed Description

Described herein are methods and compositions that contain a soluble chitosan or chitosan derivative for use in oral health. The compositions are generally useful for reducing bacteria (e.g., by clumping and removing) or disrupting a biofilm in the mouth of a subject. The scope of the invention is defined by the appended claims.

In some embodiments, the soluble chitosans or derivatized chitosans exhibit one or more of the following characteristics: for example, long shelf lives, ability to be stored as a dry powder, or ability to dissolve in water, saline, or other neutral solution (e.g., in the human mouth) and to be dispersed as needed (e.g., as a solid, semisolid, or liquid composition). Exemplary compounds include, but are not limited to soluble chitosan compounds, chitosan-arginine compounds, chitosan-guanidine compounds, chitosan-unnatural amino acid compounds, chitosan-acid-amine compounds, chitosan-natural amino acid compounds, and co-derivatives of the just described compounds and the salts thereof. These compounds and their antimicrobial activity are disclosed in US Patent applications 11/657,382 and 11/985,057. Exemplary compounds also include neutral chitosan compounds (e.g., monosaccharide-containing chitosan compounds, chitosan-lactobionic acid compounds, and chitosan-glycolic acid compounds), and co-derivatives of these compounds and the salts thereof.

### Bacterial clumping

Bacterial population, e.g., in a body cavity or epithelial/mucosal surfaces in a subject, can be reduced (e.g., to a level closer to the normal microbial level) by clumping using compounds and compositions described herein. Described herein are also methods of treatment for the colonization of e.g., the mouth, teeth, or throat by pathogenic bacteria.
This clumping can, in some embodiments, act as a "barrier," for example, when a composition described herein is used to contact a bacterial population so as to result in clumping of the bacteria onto the chitosan derivative, and the resulting composition is discarded by the subject (e.g., spit out, for example, as an oral rinse).

The method of clumping (e.g., barrier clumping) includes the step of contacting compositions or compounds described herein (e.g., soluble chitosans or derivatized chitosans) with bacteria, e.g., in the mouth or epithelial/mucosal surfaces. The soluble chitosan or chitosan derivatives described herein can interact with more than one bacterium simultaneously, linking them via a part of the polymer chain. Thus, the contact causes the bacteria to aggregate with one another. These bacteria within clumps are limited in their ability to bind to other surfaces, thereby creating a barrier to colonization by the bacteria. This barrier is a result of the decreased bacterial surface area available for colonization, the bacterial trapping within the aggregate as well as limitation of the exposure of bacterial surface receptors that are often used by bacteria to associate with biological or inert surfaces, thereby preventing the colonizing of pathogenic bacteria. In some preferred embodiments, the derivatized chitosan has a molecular weight of at least about 15 kDa.

Chitosan derivatives, e.g., chitosan-arginine, act though physical means to reduce bacteria in the mouth, and can serve as an adjunct to normal mechanical oral hygiene. The chitosan derivatives in the oral rinse act to prevent the adhesion of bacteria to the dental enamel by clumping the bacteria and allowing for easy removal from the oral cavity during rinsing through a physical interaction between the positively charged chitosan-arginine and the negatively charged cell wall of oral bacteria.

The positively charged characteristic of chitosan derivatives, e.g., chitosan-arginine, allows the composition described herein to be effective in clumping and aggregating oral bacteria. The positively charged polymer interacts with the negatively charged cell wall of the oral bacteria electrostatically. This, in turn, allows the long polymer chains of chitosan derivatives, e.g., chitosan-arginine, to interact with the bacterial cell surface and bridge between bacteria cells. This interaction allows for clumping and aggregation of the oral bacteria cells and prevents them from adhering to oral surfaces. This mechanical action allows for easy removal of the bacteria from the oral cavity during rinsing.

### Biofilm/Bacteria

Methods and compositions described herein can be used to disrupt (e.g., reduce the viscosity of, or dissolve) a preformed biofilm in a subject, e.g., in the mouth. As used herein, the term "dissolve" or "dissolving" means breaking up cohesion in a preformed biofilm such that some or all can be rinsed, flushed or washed away. Methods and compositions described herein can also be used to prevent the formation of a biofilm (e.g., reduce the ability of a biofilm to form) in the mouth of a subject.

A biofilm is a structured community of microorganisms encapsulated within a self-developed polymeric matrix and adherent to a living or inert surface. Biofilms are also often characterized by surface attachment, structural heterogeneity, genetic diversity, complex community interactions, and an extracellular matrix of polymeric substances.

Formation of a biofilm begins with the attachment of free-floating microorganisms to a surface. These first bacterial colonization occurs through adhesion to the surface initially through weak, reversible van der Waals forces. If the bacteria are not immediately separated from the surface, they can anchor themselves more permanently using cell adhesion structures such as pili. The first adherent bacteria facilitate the arrival of other cells by providing more diverse adhesion sites and beginning to build the matrix that holds the biofilm together. Once colonization has begun, the biofilm grows through a combination of cell division and recruitment. The final stage of biofilm formation is known as development, and is the stage in which the biofilm is established and may only change in shape and size. This development of biofilm environment and communication pathway allows for the cells to become more antibiotic resistant.

Biofilms can contain many different types of microorganism, e.g. bacteria, archaea, protozoa, fungi and algae; each group performing specialized metabolic functions. Microorganisms can also form monospecies films.

The biofilm is held together and protected by a matrix of excreted polymeric compounds called Extracellular polymeric substance (EPS). This matrix protects the cells within it and facilitates communication among them through biochemical signals.

Bacteria living in a biofilm can have different properties from free-floating bacteria of the same species, as the dense and protected environment of the film allows them to cooperate and interact in various ways. One benefit of this environment to the bacteria is increased resistance to detergents and antibiotics, as the dense extracellular matrix and the outer layer of cells protect the interior of the community.

Exemplary bacteria associated with biofilm include Gram-positive (e.g., *Staphylococcus aureus* (e.g., strain MW-2), *Streptococcus mutans, Clostridium perfringens, Streptococcus pyogenes* (GAS), *Clostridium difficile* and *Streptococcus sanguis)* and Gram-negative bacteria (*E. coli* (e.g., strain O:157 H:7), *Shigella flexneri, Salmonella typhimurium, Acinitobacter baumannii, Pseudomonas aeruginosa* and Legionella bacteria (e.g., *L. pneumophila*)).

Exemplary bacteria associated with biofilm in the mouth include *Streptococcus mutans, Streptococcus sanguis, Treponema denticola, Porphyromonas gingivalis* , *Aggregatibacter actinomycetemcomitans, Fusospirochetes, Veillonella,* and some forms of pathogenic *Lactobacilli, Actinomyces viscosus,* or *Nocardia spp*

Exemplary bacteria associated with biofilm in the mouth also include bacteria causing oral diseases or conditions, e.g., dental plaque, gingivitis, dental caries, or halitosis. Exemplary bacteria associated with infections in the mouth also include bacteria causing tissue or wound infections in the mouth, ear, nose and throat.

As used herein resistant microorganism or bacterium means, an organism which has become resistant to an antibacterial agent. Also, resistant microorganism or bacterium means its effective MIC has exceeded the effective dosage according to Clinical Laboratory Standards Institute (CLSI) resistance breakpoints, predefined national or internationally accepted limits, at or above which administration of an effective dose of antibiotic produces undesirable side effects. In some embodiments, the minimum inhibitory concentration of a resistant bacterium will be at least, 2, 5, 10, or 100 greater than for that seen with a non-resistant bacterium for a selected anti-bacterial agent.

Exemplary oral diseases and conditions associated with biofilm can also include oral diseases and conditions characterized by the presence of one or more of the bacteria that cause resistant bacterial infections as described herein.

### Treatment

The compositions and compounds described herein (e.g., a soluble chitosan or a derivatized chitosan) can be administered to a subject, e.g., *in vivo,* to treat, prevent, and/or diagnose a variety of disorders, including those described herein below.

As used herein, the term "treat" or "treatment" is defined as the application or administration of a composition or compound (e.g., a compound described herein (e.g., a soluble chitosan or a derivatized chitosan) to a subject, e.g., a patient, or application or administration of the composition or compound to an isolated tissue or cell, e.g., cell line, from a subject, e.g., a patient, who has a disorder (e.g., a disorder as described herein), a symptom of a disorder, or a predisposition toward a disorder, with the purpose to cure, heal, alleviate, relieve, alter, remedy, ameliorate, improve or affect the disorder, one or more symptoms of the disorder or the predisposition toward the disorder (e.g., to prevent at least one symptom of the disorder or to delay onset of at least one symptom of the disorder).

As used herein, the term "prevent" or "prevention" is defined as the application or administration of a composition or compound (e.g., a compound described herein (e.g., a soluble chitosan or a derivatized chitosan)) to a subject, e.g., a subject who is at risk for a disorder (e.g., a disorder described herein), or has a disposition toward a disorder, or application or administration of the compound to an isolated tissue or cell, e.g., cell line, from a subject, e.g., a subject who is at risk for a disorder (e.g., a disorder as described herein), or has a predisposition toward a disorder, with the purpose to avoid or preclude the disorder, or affect the predisposition toward the disorder (e.g., to prevent at least one symptom of the disorder or to delay onset of at least one symptom of the disorder).

As used herein, an amount of a composition or compound effective to treat a disorder, or a "therapeutically effective amount" refers to an amount of the composition or compound which is effective, upon single or multiple dose administration to a subject, in treating a cell, or in curing, alleviating, relieving or improving a subject with a disorder beyond that expected in the absence of such treatment.

As used herein, an amount of a composition or compound effective to prevent a disorder, or "a prophylactically effective amount" of the composition or compound refers to an amount effective, upon single- or multiple-dose administration to the subject, in preventing or delaying the occurrence of the onset or recurrence of a disorder or a symptom of the disorder.

As used herein, the term "subject" is intended to include human and non-human animals. Exemplary human subjects include a human patient having a disorder, e.g., a disorder described herein or a normal subject. The term "non-human animals" of the disclosure includes all vertebrates, e.g., non-mammals (such as chickens, amphibians, reptiles) and mammals, such as non-human primates, domesticated and/or agriculturally useful animals, e.g., sheep, dog, cat, cow, pig, etc.

As used herein, "administered in combination" or a combined administration of two agents means that two or more agents (e.g., compounds described herein) are administered to a subject at the same time or within an interval such that there is overlap of an effect of each agent on the patient. Preferably they are administered within 15, 10, 5, or 1 minute of one another. Preferably the administrations of the agents are spaced sufficiently close together such that a combinatorial (e.g., a synergistic) effect is achieved. Exemplary combinations of a derivatized chitosan described herein and one or more of anti-microbial agent(s) such as an antibiotic are described, e.g., in US Patent Application 61/113904. The combinations can have synergistic effect when used to treat a subject having a bacterial infection. The agents can be administered simultaneously, for example in a combined unit dose (providing simultaneous delivery of both agents). Alternatively, the agents can be administered at a specified time interval, for example, an interval of minutes, hours, days or weeks. Generally, the agents are concurrently bioavailable, e.g., detectable, in the subject.

In a preferred embodiment, the agents are administered essentially simultaneously, for example two unit dosages administered at the same time, or a combined unit dosage of the two agents. In another preferred embodiment, the agents are delivered in separate unit dosages. The agents can be administered in any order, or as one or more preparations that includes two or more agents. In a preferred embodiment, at least one administration of one of the agents, e.g., the first agent, is made within minutes, one, two, three, or four hours, or even within one or two days of the other agent, e.g., the second agent. In some cases, combinations can achieve synergistic results, e.g., greater than additive results, e.g., at least 1.5, 2.0, 5, 10, 20, 50, or 100 times greater than additive.

### Subject

The subject can be a human or an animal. Suitable animal subjects include: but are not limited to, pet, wild, zoo, laboratory, and farm animals. Suitable animal subjects include primates, mammals, rodents, and birds. Examples of said animals include, but not limited to, guinea pigs, hamsters, gerbils, rat, mice, rabbits, dogs, cats, horses, pigs, sheep, cows, goats, deer, rhesus monkeys, monkeys, tamarinds, apes, baboons, gorillas, chimpanzees, orangutans, gibbons, fowl, e.g., pheasant, quail (or other gamebirds), a waterfowl, ostriches, chickens, turkeys, ducks, and geese or free flying bird.

In some embodiments, the subject has an oral disease or a symptom of oral disease. Exemplary oral diseases include gingivitis and dental caries. Exemplary symptoms of oral diseases include swollen gums; mouth sores; bright-red, or purple gums; shiny gums; swollen gums that emit pus; severe oral odor; gums that are painless; except when pressure is applied; gums that bleed easily, even with gentle brushing, and especially when flossing; gums that itch with varying degrees of severity; or toothache.

In some embodiments, the subject has oral diseases or conditions characterized by the presence of one or more of the bacteria described herein, e.g., *Streptococcus mutans, Streptococcus sanguis, Treponema denticola, Porphyromonas gingivalis* , *Aggregatibacter actinomycetemcomitans, Fusospirochetes, Veillonella,* and some forms of pathogenic *Lactobacilli, Actinomyces viscosus,* or *Nocardia spp*

In some embodiments, the subject is at risk of having the oral diseases or conditions described herein.

### Gingivitis

Compositions described herein can be used to treat or prevent gingivitis in a subject.

Gingivitis is a general term for gingival diseases affecting the gingiva (gums). Gingivitis can be defined as inflammation of the gingival tissue without loss of tooth attachment (i.e.periodontal ligament). Gingival inflammation can be induced by bacterial biofilms (also called plaque) adherent to tooth surfaces.

Gingivitis is usually caused by bacterial plaque that accumulates in the small gaps between the gums and the teeth and by calculus (tartar) that forms on the teeth. These accumulations may be tiny, even microscopic, but the bacteria in them produce foreign chemicals and toxins that cause inflammation of the gums around the teeth. This inflammation can cause deep pockets between the teeth and gums and loss of bone around teeth - an effect otherwise known as periodontitis. Pregnancy, uncontrolled diabetes mellitus and the onset of puberty increase the risk of gingivitis, due to hormonal changes that may increase the susceptibility of the gums or alter the composition of the dentogingival microflora. The risk of gingivitis is increased by misaligned teeth, the rough edges of fillings, and ill fitting or unclean dentures, bridges, and crowns, due to their plaque retentive properties. The drug phenytoin, birth control pills, and ingestion of heavy metals such as lead and bismuth may also cause gingivitis.

In some cases, the inflammation of the gingiva can suddenly amplify, such as to cause Acute Necrotizing Ulcerative Gingitivitis (ANUG). The etiology of ANUG is the overgrowth of a particular type of pathogenic bacteria (fusiform-spirochete variety) but risk factors such as stress, poor nutrition and a compromised immune system can exacerbate the infection. This results in the breath being extremely bad-smelling, and the gums feeling considerable pain and degeneration of the periodontium rapidly occurs. This can be treated with a 1-week course of Metronidazole antibiotic, followed by a deep cleaning of the gums by a dental hygienist or dentist and reduction of risk factors such as stress.

The symptoms of gingivitis include, e.g., swollen gums; mouth sores; bright-red, or purple gums; shiny gums; swollen gums that emit pus; severe oral odor; gums that are painless; except when pressure is applied; gums that bleed easily, even with gentle brushing, and especially when flossing; or gums that itch with varying degrees of severity.

Gingivitis can be treated or prevented using soluble chitosans or derivatized chitosans described herein in combination with one or more of agents and/or therapeutics. For example, proper maintenance (varying from "regular cleanings" to periodontal maintenance or scaling and root planing) above and below the gum line, disrupts this plaque biofilm and removes plaque retentive calculus (tartar) to help remove the etiology of inflammation. The methods to prevent gingivitis include, e.g., regular oral hygiene that includes daily brushing and flossing; mouth wash using e.g., a saline solution or chlorhexidine; or rigorous plaque control programs along with periodontal scaling and curettage. In some embodiments, the administrations of a combination of agents and therapeutics are spaced sufficiently close together such that a synergistic effect is achieved.

Gingivitis can promote inflammation of the blood vessels, an important risk factor in inflammatory disorders in a subject, such as atherosclerosis and heart disease. People with gum disease are known, for instance, to have elevated levels of C-reactive protein (CRP), a marker for inflammation that is associated with an increased risk of coronary artery disease. It has also been shown that people with periodontal disease also have elevated levels of lipoprotein-associated phospholipase A2, another significant marker for inflammation that increases cardiac risk. Compositions described herein can be used to treat or prevent gingivitis-associated heart diseases or conditions in a subject.

### Dental caries

Compositions described herein can be used to treat or prevent dental caries in a subject.

Dental caries, also known as tooth decay or cavity, is a disease where bacterial processes damage hard tooth structure (enamel, dentin and cementum). These tissues progressively break down, producing dental cavities (holes in the teeth). Bacteria associated with dental caries include, e.g., *Streptococcus mutans.*

The earliest sign of a new carious lesion is the appearance of a chalky white spot on the surface of the tooth, indicating an area of demineralization of enamel. This is referred to as incipient decay. As the lesion continues to demineralize, it can turn brown but will eventually turn into a cavitation. As the enamel and dentin are destroyed, the cavity becomes more noticeable. The affected areas of the tooth change color and become soft to the touch. Once the decay passes through enamel, the dentinal tubules, which have passages to the nerve of the tooth, can become exposed and cause the tooth to hurt. The pain may worsen with exposure to heat, cold, or sweet foods and drinks. Dental caries can also cause bad breath and foul tastes. In highly progressed cases, infection can spread from the tooth to the surrounding soft tissues. Complications of dental caries include, e.g., cavernous sinus thrombosis and Ludwig's angina.

Dental caries can be caused by infection of bacteria, e.g., *Streptococcus mutans, Streptococcus sanguis, Actinomyces viscosus,* and *Nocardia* spp. Other risk factors include, e.g., disorders or diseases affecting teeth (e.g., Amelogenesis imperfecta), the anatomy of teeth, fermentable carbohydrates, the frequency of which teeth are exposed to cariogenic (acidic) environments, reduce saliva (e.g., caused by medical conditions such as diabetes, or side effect of medications), or the use of tobacco.

Compositions described herein can be used in combination with one or more of agents and/or therapies to treat or prevent dental caries in a subject. For example, dental caries can be treated by e.g., dental restoration or tooth extraction. Dental caries can be prevented by e.g., oral hygiene (e.g., proper brushing and flossing), dental sealants, or fluoride therapy. In some embodiments, the administrations of a combination of agents and therapeutics are spaced sufficiently close together such that a synergistic effect is achieved.

### Dental plaque

Compositions described herein can be used to treat (e.g., disrupt) or prevent dental plaque in a subject.

Dental plaque is biofilm (usually colorless) that builds up on the teeth. If not removed regularly, it can lead to dental cavities (caries) or periodontal problems (such as gingivitis).

The microorganisms that form the dental plaque include bacteria, e.g., *Streptococcus mutans* and anaerobes, with the composition varying by location in the mouth. Examples of such anaerobes include *Fusobacterium* and *Actinobacteria.* Those microorganisms close to the tooth surface can convert to anaerobic respiration and produce acids. Acids released from dental plaque lead to demineralization of the adjacent tooth surface, and consequently to dental caries. Saliva is also unable to penetrate the build-up of plaque and thus cannot act to neutralize the acid produced by the bacteria and remineralize the tooth surface. They also cause irritation of the gums around the teeth that could lead to gingivitis, periodontal disease and tooth loss. Plaque build up can also become mineralized and form calculus (tartar).

Compositions described herein can be used in combination with one or more of agents and therapies to treat or prevent dental plaque. For example, dental plaque can be prevented and removed by e.g., brushing thoroughly at least twice a day, with a fluoride toothpaste; using dental floss daily to remove plaque from between your teeth and under your gum line; checking teeth with plaque disclosing tablets to ensure removing tooth plaque; controlling diet (e.g., limiting sugary or starchy foods); and visiting dentist regularly for professional cleanings and dental examinations. In some embodiments, the administrations of a combination of agents and therapeutics are spaced sufficiently close together such that a synergistic effect is achieved.

### Halitosis

Compositions described herein can be used to treat or prevent halitosis.

Halitosis, also known as oral malodor, breath odor, mouth odor, foul breath, fege bosta, fetor oris, fetor ex ore, or bad breath are terms used to describe noticeably unpleasant odors exhaled in breathing. The origin of halitosis include, e.g., mouth, tongue, gum disease, nose, tonsils, stomach, or systemic diseases and specific sulfur-molecule generating bacteria such as *Solobacterium moorei.*

Compositions described herein can be used in combination with one or more of agents and therapies to treat or prevent halitosis. For example, treatment for halitosis include, e.g., gently cleaning the tongue surface, gargling, or maintaining oral hygiene. In some embodiments, the administrations of a combination of agents and therapeutics are spaced sufficiently close together such that a synergistic effect is achieved.

### Antibacterials

The compositions and compounds described herein (e.g., soluble chitosans or derivatized chitosans) can be used in combination of one or more of antibiotics, to reduce bacteria in the mouth, or to treat or prevent an oral disease or condition, e.g., dental plaque, gingivitis, dental caries, or halitosis. General classes of antibiotics include, e.g., aminoglycosides, bacitracin, beta-lactam antibiotics, cephalosporins, chloramphenicol, glycopeptides, macrolides, lincosamides, penicillins, quinolones, rifampin, glycopeptide, tetracyclines, trimethoprim and sulfonamides. In some embodiments, the administrations of a combination of agents and therapeutics are spaced sufficiently close together such that a synergistic effect is achieved.

Exemplary antibiotics within the classes recited above are provided as follows. Exemplary aminoglycosides include Streptomycin, Neomycin, Framycetin, Parpmycin, Ribostamycin, Kanamycin, Amikacin, Dibekacin, Tobramycin, Hygromycin B, Spectinomycin, Gentamicin, Netilmicin, Sisomicin, Isepamicin, Verdamicin, Amikin, Garamycin, Kantrex, Netromycin, Nebcin, and Humatin. Exemplary carbacephems include Loracarbef (Lorabid). Exemplary carbapenems include Ertapenem, Invanz, Doripenem, Finibax, Imipenem/Cilastatin, Primaxin, Meropenem, and Merrem. Exemplary cephalosporins include Cefadroxil, Durisef, Cefazolin, Ancef, Cefalotin, Cefalothin, Keflin, Cefalexin, Keflex, Cefaclor, Ceclor, Cefamandole, Mandole, Cefoxitin, Mefoxin, Cefprozill, Cefzil, Cefuroxime, Ceftin, Zinnat, Cefixime, Suprax, Cefdinir, Omnicef, Cefditoren, Spectracef, Cefoperazone, Cefobid, Cefotaxime, Claforan, Cefpodoxime, Fortaz, Ceftibuten, Cedax, Ceftizoxime, Ceftriaxone, Rocephin, Cefepime, Maxipime, and Ceftrobriprole. Exemplary glycopeptides include Dalbavancin, Oritavancin, Teicoplanin, Vancomycin, and Vancocin. Exemplary macrolides include Azithromycin, Sithromax, Sumamed, Zitrocin, Clarithromycin, Biaxin, Dirithromycin, Erythromycin, Erythocin, Erythroped, Roxithromycin, Troleandomycin, Telithromycin, Ketek, and Spectinomycin. Exemplary monobactams include Aztreonam. Exemplary penicillins include Amoxicillin, Novamox, Aoxil, Ampicillin, Azlocillin, Carbenicillin, Coxacillin, Diloxacillin, Flucloxacillin Floxapen, Mezlocillin, Methicillin, Nafcillin, Oxacillin, Penicillin, and Ticarcillin. Exemplary polypeptides include Bacitracin, Colistin, and Polymyxin B. Exemplary quinolones include Ciprofloxacin, Cipro, Ciproxin, Ciprobay, Enoxacin, Gatifloxacin, Tequin, Levofloxacin, Levaquin, Lomefloxacin, Moxifloxacin, Avelox, Norfloxacin, Noroxin, Ofloxacin, Ocuflox, Trovafloxacin, and Trovan. Exemplary sulfonamides include Mefenide, Prontosil (archaic), Sulfacetamide, Sulfamethizole, Sulfanilamide (archaic), Sulfasalazine, Sulfisoxazole, Trimethoprim, Trimethoprim-Sulfamethoxazole (cotrimoxazole), and Bactrim. Exemplary tetracyclines include Demeclocyline, Doxycycline, Vibramycin, Minocycline, Minocin, Oxytetracycline, Terracin, Tetracycline, and Sumycin. Other exemplary antibiotics include Salvarsan, Chloamphenicol, Chloromycetin, Clindamycin, Cleocin, Linomycin, Ethambutol, Fosfomycin, Fusidic Acid, Fucidin, Furazolidone, Isoniazid, Linezolid, Zyvox, Metronidazole, Flagyl, Mupirocin, Bactroban, Nitrofurantion, Macrodantin, Macrobid, Platensimycin, Pyrazinamide, Quinupristin/Dalfopristin (Syncerid), Rifampin (rifampicin), and Tinidazole. In some embodiments, the exemplary antibiotics include xylitol, hydrogen peroxide, and cetylpyridinium chloride.

In one embodiment, a soluble chitosan or derivatized chitosan (e.g., a soluble chitosan or derivatized chitosan described herein) is used in combination with xylitol to treat a disease or a symptom of a disease described herein, e.g., an oral disease, e.g., dental caries, dental plaque, gingivitis, halitosis. For example, xylitol can be administered at a daily dosage of less than about 10 gram, 9 gram, 8 gram, 7 gram, 6 gram, 5 gram, 4 gram, 3 gram, 2 gram, or 1 gram.

Other active agents in oral rinses can include, e.g., thymol, eucalyptol, hexetidine, methyl salicylate, menthol, chlorhexidine, chlorhexidine gluconate, benzalkonium chloride, cetylpyridinium chloride, methylparaben, hydrogen peroxide, and domiphen bromide. Other active agents in oral rinses can also include, e.g., fluoride, enzymes and calcium.

In some embodiments, the antibiotic is metronidazole, hydrogen peroxide, cetylpyridinium chloride, xylitol, chlorhexidine, delmopinol, or decapinol.

### Anti-inflammatory

The compositions and compounds described herein (e.g., soluble chitosans and derivatized chitosans) can be used in combination with one or more anti-inflammatory drugs, e.g., steroidal anti-inflammatory drugs and non-steroidal anti-inflammatory drugs (NSAIDs), to reduce bacteria in the mouth, or to treat or prevent an oral disease or condition, e.g., dental plaque, gingivitis, dental caries, or halitosis. In some embodiments, the administrations of a combination of agents and therapeutics are spaced sufficiently close together such that a synergistic effect is achieved.

Exemplary steroidal anti-inflammatory drugs include glucocorticoids (corticosteroids), e.g., Hydrocortisone (Cortisol), Cortisone acetate, Prednisone, Prednisolone, Methylprednisolone, Dexamethasone, Betamethasone, Triamcinolone, Beclometasone, Fludrocortisone acetate, Deoxycorticosterone acetate (DOCA), and Aldosterone. Exemplary non-steroidal anti-inflammatory drugs include Aspirin, Choline and magnesium salicylates, Choline salicylate, Celecoxib, Diclofenac potassium, Diclofenac sodium, Diclofenac sodium with misoprostol, Diflunisal, Etodolac, Fenoprofen calcium, Flurbiprofen, Ibuprofen, Indomethacin, Ketoprofen, Magnesium salicylate, Meclofenamate sodium, Mefenamic acid, Meloxicam, Nabumetone, Naproxen, Naproxen sodium, Oxaprozin, Piroxicam, Rofecoxib, Salsalate, Sodium salicylate, Sulindac, Tolmetin sodium, and Valdecoxib. Examples of non-steroidal anti-inflammatory agents (e.g., peptides) include regulatory cytokines such as interleukins, e.g., IL-1, IL-4, IL-6, IL-10, IL-11, and IL-13.

### Soluble chitosans and chitosan derivatives

Compounds and compositions containing a soluble chitosan or a functionalized chitosan derivative for treating or preventing bacterial infections and damage in the mouth, e.g., gingivitis, and dental caries, are described herein.

Chitosan is an insoluble polymer derived from chitin, which is a polymer of N-acetylglucosamine that is the main component of the exoskeletons of crustaceans (e.g. shrimp, crab, lobster). Chitosan is formed from chitin by deacetylation, and as such is not a single polymeric molecule, but a class of molecules having various molecular weights and varius degrees of deacetylation. The percent deacetylation in commercial chitosans is typically between 50-100%. The chitosan derivatives described herein are generated by functionalizing the resulting free amino groups with positively charged or neutral moieties, as described herein. The degrees of deacetylation and functionalization impart a specific charge density to the functionalized chitosan derivative. The resulting charge density affects solubility, and the strength of interaction with bacterial cell walls and membranes. The molecular weight is also an important factor in the tenacity of bacterial wall interaction and thus bactericidal activity. Thus, in accordance with the present disclosure, the degree of deacetylation, the functionalization and the molecular weight must be optimized for optimal efficacy. The derivatized chitosans described herein have a number of properties which are advantageous including solubility at physiologic pH and antimicrobial activity when in solution or dry at any pH less than about 9.

A soluble chitosan as described herein, refers to a water soluble chitosan that is not derivatized on the hydroxyl or amine moieties. A soluble chitosan is comprised of glucosamine and acetylglucosamine monomers. Generally a water soluble chitosan has a molecular weight of less than or equal to about 10 kDa and a degree of deactylation equal or greater than 80%. Water soluble is defined as being fully dissolvable in water at pH 7.

The chitosan derivatives described herein are generated by functionalizing the resulting free amino groups with positively charged or neutral moieties, as described herein.

Chitosans with any degree of deacetylation (DDA) greater than 50% are used in the present disclosure, with functionalization between 2% and 50% of the available amines. The degree of deacetylation determines the relative content of free amino groups to total monomers in the chitosan polymer. Methods that can be used for determination of the degree of deacetylation of chitosan include, e.g, ninhydrin test, linear potentiometric titration, near-infrared spectroscopy, nuclear magnetic resonance spectroscopy, hydrogen bromide titrimetry, infrared spectroscopy, and first derivative UV-spectrophotometry. Preferably, the degree of deacetylation of a soluble chitosan or a derivatized chitosan described herein is determined by quantitave infrared spectroscopy. Percent functionalization is determined as the % of derivatized amines relative to the total number of available amino moieties prior to reaction on the chitosan polymer. Preferably, the percent functionalization of a derivatized chitosan desccribed herein is determined by H-NMR or quantitative elemental analysis. The degrees of deacetylation and functionalization impart a specific charge density to the functionalized chitosan derivative. The resulting charge density affects solubility, and strength of interaction with bacterial cell walls and membranes. The molecular weight is important in controlling the size of the bacterial clumps. Thus, in accordance with the present disclosure, these properties must be optimized for optimal efficacy. Exemplary chitosan derivatives are described in Baker et al; 11/657,382 filed on January 24, 2007.

The chitosan derivatives described herein have a range of polydispersity index (PDI) between about 1.0 to about 2.5. As used herein, the polydispersity index (PDI), is a measure of the distribution of molecular weights in a given polymer sample. The PDI calculated is the weight averaged molecular weight divided by the number averaged molecular weight. This calculation indicates the distribution of individual molecular weights in a batch of polymers. The PDI has a value always greater than 1, but as the polymer chains approach uniform chain length, the PDI approaches unity (1). The PDI of a polymer derived from a natural source depends on the natural source (e.g. chitin or chitosan from crab vs. shrimp vs. fungi) and can be affected by a variety of reaction, production, processing, handling, storage and purifying conditions. Methods to determine the polydispersity include, e.g., gel permeation chromatography (also known as size exclusion chromatography); light scattering measurements; and direct calculation from MALDI or from electrospray mass spectrometry. Preferably, the PDI of a soluble chitosan or a derivatized chitosan described herein is determined by HPLC and multi angle light scattering methods.

The chitosan derivatives described herein have a range of molecular weights that are soluble at neutral and physiological pH, and include for the purposes of this disclosure molecular weights ranging from 5 - 1,000 kDa. Embodiments described herein are feature moderate molecular weight of derivatized chitosans (25 kDa, e.g., from about 15 to about 300 kDa) which can have clumping, diffusible and biofilm disruption properties.

The functionalized chitosan derivatives described herein include the following:
(A) Chitosan-arginine compounds;
(B) Chitosan-natural amino acid derivative compounds;
(C) Chitosan-unnatural amino acid compounds;
(D) Chitosan-acid amine compounds;
(E) Chitosan-guanidine compounds; and
(F) Neutral chitosan derivative compounds.

### (A) Chitosan-arginine compounds

In some embodiments, the present disclosure is directed to chitosan-arginine compounds, where the arginine is bound through a peptide (amide) bond via its carbonyl to the primary amine on the glucosamines of chitosan: 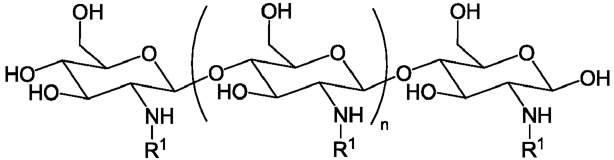 wherein each R¹ is independently selected from hydrogen, acetyl, and a group of the following formula: 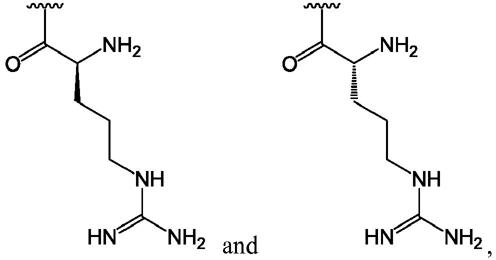 or a racemic mixture thereof,
wherein at least 25% of R¹ substituents are H, at least 1% are acetyl, and at least 2% are a group of the formula shown above.

### (B) Chitosan-natural amino acid derivative compounds

In some embodiments, the present disclosure is directed to chitosan-natural amino acid derivative compounds, wherein the natural amino acid may be histidine or lysine. The amino is bound through a peptide (amide) bond via its carbonyl to the primary amine on the glucosamines of chitosan: 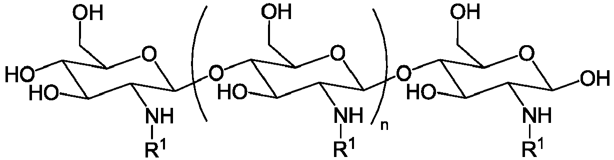 wherein each R¹ is independently selected from hydrogen, acetyl, and a group of the following formula: 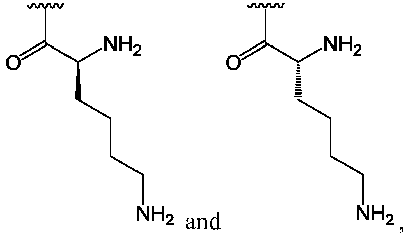 or a racemic mixture thereof, wherein at least 25% of R¹ substituents are H, at least 1% are acetyl, and at least 2% are a group of the formula shown above; OR a group of the following formula: 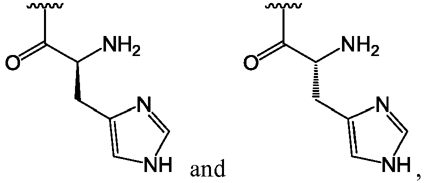 or a racemic mixture thereof, wherein at least 25% of R¹ substituents are H, at least 1% are acetyl, and at least 2% are a group of the formula shown above.

### (C) Chitosan-unnatural amino acid compounds

In some embodiments, the present disclosure is directed to chitosan-unnatural amino acid compounds, where the unnatural amino acid is bound through a peptide (amide) bond via its carbonyl to the primary amine on the glucosamines of chitosan: 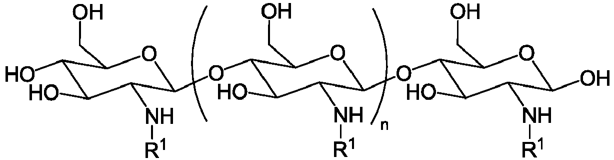 wherein each R¹ is independently selected from hydrogen, acetyl, and a group of the following formula: 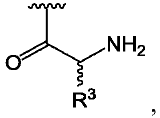 wherein R³ is an unnatural amino acid side chain, and wherein at least 25% of R¹ substituents are H, at least 1% are acetyl, and at least 2% are a group of the formula shown above.

Unnatural amino acids are those with side chains not normally found in biological systems, such as ornithine (2,5-diaminopentanoic acid). Any unnatural amino acid may be used in accordance with the disclosure. In some embodiments, the unnatural amino acids coupled to chitosan have the following formulae:

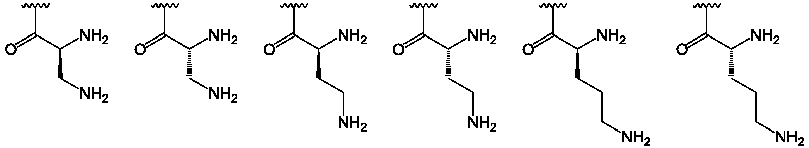

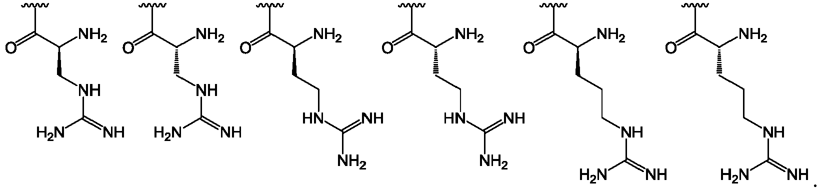

### (D) Chitosan-acid amine and guanidine compounds

In some embodiments, the present disclosure is directed to chitosan-acid amine compounds, or their guanidylated counterparts. The acid amine is bound through a peptide (amide) bond via its carbonyl to the primary amine on the glucosamines of chitosan: 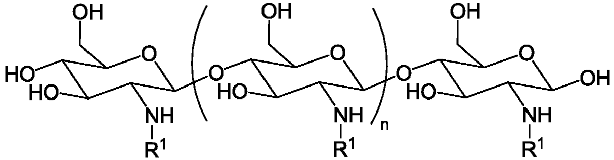 wherein each R¹ is independently selected from hydrogen, acetyl, and a group of the following formula: 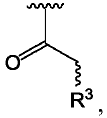 wherein R³ is selected from amino, guanidino, and C₁-C₆ alkyl substituted with an amino or a guanidino group, wherein at least 25% of R¹ substituents are H, at least 1% are acetyl, and at least 2% are a group of the formula shown above

In some embodiments, R¹ is selected from one of the following:

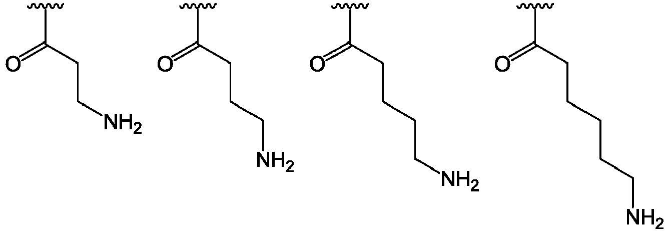

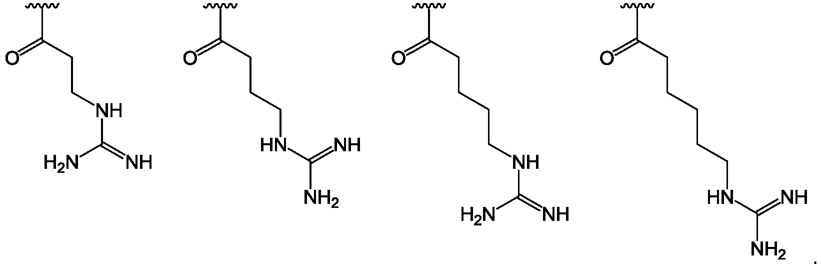

### (E) Chitosan-guanidine compounds

In some embodiments, the present disclosure is directed to chitosan-guanidine compounds. 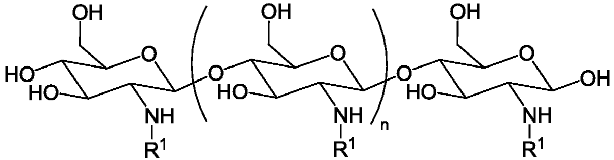 wherein each R¹ is independently selected from hydrogen, acetyl, and or together with the nitrogen to which it is attached, forms a guanidine moiety; wherein at least 25% of R¹ substituents are H, at least 1% are acetyl, and at least 2% are a group of the formula shown above.

### (F) Neutral chitosan derivative compounds

In some embodiments, the present disclosure is directed to neutral chitosan derivative compounds. Exemplary neutral chitosan derivative compounds include those where one or more amine nitrogens of the chitosan has been covalently attached to a neutral moiety such as a sugar: 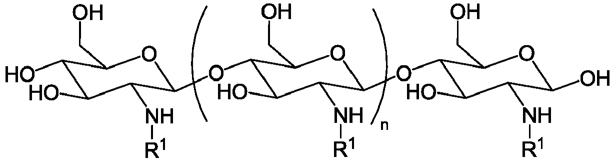 wherein each R¹ is independently selected from hydrogen, acetyl, and a sugar (e.g., a naturally occurring or modified sugar) or an α-hydroxy acid. Sugars can be monosaccharides, disaccharides or polysaccharides such as glucose, mannose, lactose, maltose, cellubiose, sucrose, amylose, glycogen, cellulose, gluconate, or pyruvate. Sugars can be covalently attached via a apacer or via the carboxylic acid, ketone or aldehyde group of the terminal sugar. Examples of α-hydroxy acids include glycolic acid, lactic acid, and citric acid. In some preferred embodiments, the neutral chitosan derivative is chitosan-lactobionic acid compound or chitosan-glycolic acid compound. Exemplary salts and coderivatives include those known in the art, for example, those described in US 20070281904.

### Compositions

Described herein are also compositions comprising a soluble chitosan or a functionalized chitosan derivative, e.g., a sobuble or derivatized chitosan described herein. In some embodiments, the composition is a liquid, solid, or semisolid composition. In some embodiments, the composition is a pharmaceutical composition. In some embodiments, the composition is a reaction mixture. In some embodiments, the composition is an oral rinse. In some embodiments, the composition is a dentifrice. In some embodiments, the composition is a tooth strip, a gel, a semi-solid, liquid, or a component of a device such as a toothbrush.

In some embodiments, the composition further comprises one or more additional compound or agent. In some embodiments, the second compound or agent is another chitosan derivative, e.g., a soluble or derivaized chitosan described herein.

In some embodiments, the composition has less than about 20%, 15%, 10%, 5%, 2%, or 1%, or is substantially free, of a chitosan polymer wherein one or more of the nitrogen-containing groups of the glucosamine monomer is substituted with a polymerized amino acid, e.g., polyarginine (e.g., diargine, triargine, etc).

In some embodiments, the composition has less than about 20%, 15%, 10%, 5%, 2%, or 1%, or is substantially free, of a chitosan polymer having a molecular weight of less than 15,000 Da, 10,000 Da, or 5,000 Da.

The composition described herein can also be used to treat or prevent a disease or a symptom of a disease described herein, e.g., an oral disease or a symptom of an oral disease, e.g, gingivitis, dental caries, dental plaque, halitosis.

### Formulations and routes of administration

The compounds described herein can be formulated in a variety of manners, including for oral treatment and oral delivery (e.g., administered orally). In some embodiments, oral rinse (mouthwash) is used for the oral delivery of a compound descried herein, to reduce bacteria in the mouth, or to treat or prevent an oral disease or condition, e.g., dental plaque, gingivitis, dental caries, or halitosis. In some embodiments, dentifrice (e.g., toothpaste, liquid, tooth powder, tooth gel, or tooth strip), gum, lozenge, or sucker is used for the oral delivery of a compound descried herein, to reduce bacteria in the mouth, or to treat or prevent an oral disease or condition, e.g., dental plaque, gingivitis, dental caries, or halitosis.

The compounds described herein (e.g., a soluble chitosan or a derivatized chitosan) can, for example, be administered for treatment in the oral cavity at concentrations from about 1 µg/mL to about 10 mg/mL, about 10 µg/mL to about 10 mg/mL, about 100 µg/mL to about 10 mg/mL, about 500 µg/mL to about 10 mg/mL, about 1 mg/mL to about 10 mg/mL, about 2 mg/mL to about 10 mg/mL, about 5 mg/mL to about 10 mg/mL, about 1 µg/mL to about 5 mg/mL, about 1 µg/mL to about 2 mg/mL, about 1 µg/mL to about 1 mg/mL, about 1 µg/mL to about 500 µg/mL, about 1 µg/mL to about 100 µg/mL, or about 1 µg/mL to about 10 µg/mL, for example, as required based on the severity of the oral disease and the compliance of the patient, for an about 30 sec to about 2 minute, about 30 sec to about 1 minute, or about 1 minute to about 2 minute rinse. A preferred embodiment is a about 30mL volume administration of from about 10 µg/mL to about 100 µg/mL of the compounds described herein for an about 30 sec to about 2 minute rinse. Another preferred embodiment is from about 10 µg/mL to about 1000 µg/mL, about 25 µg/mL to about 750 µg/mL, about 50 µg/mL to about 500 µg/mL, or about 100 µg/mL to about 250 µg/mL of the compound described herein in a dentifrice. The compound described herein can be administered before or after the onset of the disorder described herein. The methods herein contemplate administration of an effective amount of compound or compound composition to achieve the desired or stated effect. Typically, the compositions of this disclosure will be administered from about 1 to about 6 times, about 1 to about 4 times, or about 2 to about 3 times per day.

Lower or higher doses than those recited above may be required. Specific dosage and treatment regimens for any particular patient will depend upon a variety of factors, including the activity of the specific compound employed, the age, body weight, general health status, sex, diet, time of administration, drug combination, the severity and course of the disease, condition or symptoms, the patient's disposition to the disease, condition or symptoms, and the judgment of the treating physician.

Upon improvement of a patient's condition, a maintenance dose of a compound, composition or combination of this disclosure may be administered, if necessary. Subsequently, the dosage or frequency of administration, or both, may be reduced, as a function of the symptoms, to a level at which the improved condition is retained. Patients may, however, require intermittent treatment on a long-term basis upon any recurrence of disease symptoms.

Pharmaceutical compositions of this disclosure comprise a compound of the formulae described herein or a pharmaceutically acceptable salt thereof; an additional compound including for example, a steroid or an analgesic; and any pharmaceutically acceptable carrier, adjuvant or vehicle. Alternate compositions of this disclosure comprise a compound described herein or a pharmaceutically acceptable salt thereof; and a pharmaceutically acceptable carrier, adjuvant or vehicle. The compositions delineated herein include the compounds described herein, as well as additional therapeutic compounds if present, in amounts effective for achieving a modulation of disease or disease symptoms.

The compositions are generally made by methods including the steps of combining a compound described herein with one or more carriers and, optionally, one or more additional therapeutic compounds delineated herein.

The term "pharmaceutically acceptable carrier or adjuvant" refers to a carrier or adjuvant that may be administered to a patient, together with a compound of this disclosure, and which does not destroy the pharmacological activity thereof and is nontoxic when administered in doses sufficient to deliver a therapeutic amount of the compound.

The pharmaceutical compositions of this disclosure may be orally administered in any orally acceptable dosage form including, but not limited to, oral rinse, gels and solutions. If desired, certain sweetening and/or flavoring and/or coloring agents may be added.

Pharmaceutically acceptable carriers, adjuvants and vehicles that may be used in the pharmaceutical compositions of this disclosure include, but are not limited to, ion exchangers, alumina, aluminum stearate, lecithin, self-emulsifying drug delivery systems (SEDDS) such as d-α-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as α -, β-, and γ-cyclodextrin, may also be advantageously used to enhance delivery of compounds of the formulae described herein.

In some cases, the pH of the formulation may be adjusted with pharmaceutically acceptable acids, bases or buffers to enhance the stability of the formulated compound or its delivery form.

When the compositions of this disclosure comprise a combination of compounds described herein, both the compounds are generally present at dosage levels of between about 1 to 100%, and more preferably between about 5 to 95% of the dosage normally administered in a monotherapy regimen. In synergistic applications, both of the compounds are generally present at dosage levels of from about 1% to about 30%, and more preferably from about 0.1% to about 5% of the dosage normally administered in a monotherapy regimen. Additionally, combinations of a plurality of compounds described herein are also envisioned. The compounds may be administered separately, as part of a multiple dose regimen, from the compounds of this disclosure. Alternatively, those compounds may be part of a single dosage form, mixed together with the compounds of this disclosure in a single composition.

### Oral Rinse Compositions and Components

The compositions and components described herein can be provided in the form of oral rinse.

Ingredients of such oral rinse typically include one or more of an active ingredient (e.g., a soluble chitosan or derivatized chitosan described herein), water (from about 45% to about 99%), ethanol (from about 0% to about 25%), a humectant (from about 0% to about 50%), a surfactant (from about 0.01% to about 7%), a preservative (from about 0.01% to about 0.5%), a thickening agent (from 0% to about 5%), a flavoring agent (from about 0.04% to about 2%), a sweetening agent (from about 0.1% to about 3%), and a coloring agent (from about 0.001% to about 0.5%). Such oral rinses may also include one or more of an anti-caries agent (from about 0.05% to about 0.3% as fluoride ion) and an anti-calculus agent (from about 0.1% to about 3%). Examples of suitable oral rinse ingredients are described below.

***Humectant:*** Generally, humectants are polyols. Examples of humectants include glycerin, sorbitol propyleneglycol, xylitol, lactitol, polypropylene glycol, polyethylene glycol, hydrogenated corn syrup and mixtures thereof.

***Surfactants:*** In some instances, the oral rinse may include one or more surfactants to provide a desirable foaming quality. Surfactants generally include anionic, nonionic, cationic and zwitterionic or amphoteric compositions. Examples of surfactants include soaps, sulfates (e.g., sodium lauryl sulfate and sodium dodecyl benzene sulfonate), sodium lauryl sarcosinate, sorbitan esters of fatty acids, sulfobetaines (e.g., cocamidopropylbatine), and D-glucopyranoside C₁₀₋₁₆ alkyl oligomeric. In some embodiments, the surfactants include sodium lauryl sulphate, cocamidopropyl betaine, and D-glucopyranoside C₁₀-C₁₆ alkyl oligomeric.

***Thickening Agents:*** Examples of thickening agents include thickening silica, polymers, clays, and combinations thereof. Thickening silica, for example, SILODENT 15 hydrated silica, in the amount between about 4% to about 8% by weight (e.g., about 6%) provide desirable in-mouth characteristics.

***Preservatives:*** Examples of preservatives include anti-bacterial agents, antifungal agents (e.g., benzoic acid and sorbic acid), bacteriostatic agents (e.g., thimersol, phenyl mercuric acetate, phenyl mercuric nitrate, and sodium azide), fungistatic agents, and enzyme inhibitors.

***Anti-caries agents:*** Examples of anti-caries agents include water soluble fluoride salts, fluorosilicates, fluorozirconates, fluorostannites, fluoroborates, fluorotitanates, fluorogermanates, mixed halides and casine.

***Anti-calculus agents:*** Examples of anti-calculus agents (e.g. alkali-metal pyrophosphates, hypophosphite-containing polymers, organic phosphocitrates, phosphocitrates, polyphosphates).

The oral rinse composition can also contain one or more of, e.g., an antiseptic agent (e.g., thymol), an anesthetic agent (e.g., a local anesthetic agent (e.g., menthol)), a dissolving agent (e.g., ethanol), a cleaning agent (e.g., methyl salicylate), a whitening agent (e.g., hydrogen peroxide), and a desensitizing agent (e.g., potassium nitrate).

### Dentifrice Compositions and Components

Oral care compositions and components formulated as a dentifrice (e.g., toothpaste, tooth powder, and tooth gel) can include a binder, a carrier, and an active ingredient, e.g., a soluble chitosan or derivatized chitosan described herein. In some instances, the dentifrice may also include one or more of the following: a surfactant and/or detergent, a thickening agent, a polishing agent, a carrier, a humectant, a salt, etc. Examples of suitable dentifrice ingredients are described below.

***Binder:*** The binder system, generally, is a primary factor that determines the rheological characteristics of the oral care composition. The binder also acts to keep any solid phase of an oral care component suspended, thus preventing separation of the solid phase portion of the oral care component from the liquid phase portion. Additionally, the binder can provide body or thickness to the oral care composition. Thus, in some instances, a binder can also provide a thickening function to an oral care composition.

Examples of binders include sodium carboxymethyl-cellulose, cellulose ether, xanthan gum, carrageenan, sodium alginate, carbopol, or silicates such as hydrous sodium lithium magnesium silicate. Other examples of suitable binders include polymers such as hydroxypropyl methylcellulose, hydroxyethyl cellulose, guar gum, tragacanth gum, karaya gum, arabic gum, Irish moss, starch, and alginate. Alternatively, the binder can include a clay, for example, a synthetic clay such as a hectorite, or a natural clay. Each of the binders can be used alone or in combination with other binders.

***Surfactants*/*Detergents:*** In some instances, the dentifrice may include one or more surfactants or detergents to provide a desirable foaming quality. Surfactants generally include anionic, nonionic, cationic and zwitterionic or amphoteric compositions. Examples of surfactants include soaps, sulfates (e.g., sodium lauryl sulfate and sodium dodecyl benzene sulfonate), sodium lauryl sarcosinate, sorbitan esters of fatty acids, sulfobetaines (e.g., cocamidopropylbatine), and D-glucopyranoside C₁₀₋₁₆ alkyl oligomeric. In some embodiments, the surfactants include sodium lauryl sulphate, cocamidopropyl betaine, and D-glucopyranoside C₁₀-C₁₆ alkyl oligomeric. In general, surfactants are present in an amount from about 0.2 to about 8% by weight (e.g., from about 1 to about 5% or from about 1.5 to about 3.5%).

***Thickening Agents:*** Examples of thickening agents include thickening silica, polymers, clays, and combinations thereof. Thickening silica, for example, SILODENT 15 hydrated silica, in the amount between about 4% to about 8% by weight (e.g., about 6%) provide desirable in-mouth characteristics.

***Polishing Agents:*** Examples of polishing agents include abrasives, such as carbonates (e.g., sodium bicarbonate, calcium carbonate) water-colloidal silica, precipitated silicas (e.g., hydrated silica), sodium aluminosilicates, silica grades containing alumina, hydrated alumina, dicalcium phosphates, insoluble sodium metaphosphate, and magnesiums (e.g., trimagnesium phosphate). A suitable amount of polishing agent is an amount that safely provides good polishing and cleaning and which, when combined with other ingredients gives a smooth, flowable, and not excessively gritty composition. In general, when polishing agents are included, they are present in an amount from about 5% to about 50% by weight (e.g., from about 5% to about 35%, or from about 7% to about 25%).

***Carriers:*** Examples of carriers include water, polyethylene glycol, glycerin, polypropylene glycol, starches, sucrose, alcohols (e.g., methanol, ethanol, isopropanol, etc.), or combinations thereof. Examples of combinations include various water and alcohol combinations and various polyethylene glycol and polypropylene glycol combinations. In general, the amount of carrier included is determined based on the concentration of the binder system along with the amount of dissolved salts, surfactants, and dispersed phase.

***Humectants:*** Generally, humectants are polyols. Examples of humectants include glycerin, sorbitol propyleneglycol, xylitol, lactitol, polypropylene glycol, polyethylene glycol, hydrogenated corn syrup and mixtures thereof. In general, when humectants are included they can be present in an amount from about 10% to about 60% by weight.

***Buffers and*/*or Salts:*** Examples of buffers and salts include primary, secondary, or tertiary alkali metal phosphates, citric acid, sodium citrate, sodium saccharin, tetrasodium pyrophosphate, sodium hydroxide, and the like.

***Active Ingredients:*** Dentifrices may include active ingredients including, e.g., a soluble chitosan or derivatized chitosan described herein, for example, to prevent cavities, to whiten teeth, to freshen breath, to deliver oral medication, and to provide other therapeutic and cosmetic benefits such as those described herein. Examples of active ingredients include the following: anti-caries agents (e.g., water soluble fluoride salts, fluorosilicates, fluorozirconates, fluorostannites, fluoroborates, fluorotitanates, fluorogermanates, mixed halides and casine); anti-tarter agents; anti-calculus agents (e.g. alkali-metal pyrophosphates, hypophosphite-containing polymers, organic phosphocitrates, phosphocitrates, and polyphosphates); anti-bacterial agents (e.g., bacteriocins, antibodies, enzymes, chlorhexinol, chlorhexidine, hydrogen peroxide, sodium benzoate, benzalkonium chloride, cetylpyridinium chloride, delmopinol, decapinol, ethanol, and thymol); anti-bacterial enhancing agents; anti-microbial agents (e.g., Triclosan, chlorhexidine, copper-, zinc- and stannous salts such as zinc citrate, zinc sulfate, zinc glycinate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenbis-(4-chloro-6-bromophenol)); desensitizing agents (e.g., potassium citrate, potassium chloride, potassium tartrate, potassium bicarbonate, potassium oxalate, potassium nitrate and strontium salts); whitening agents (e.g., bleaching agents such as peroxy compounds, e.g. potassium peroxydiphosphate); anti-plaque agents; gum protecting agents (e.g., vegetable oils such as sunflower oil, rape seed oil, soybean oil and safflower oil, and other oils such as silicone oils and hydrocarbon oils). The gum protection agent may be an agent capable of improving the permeability barrier of the gums. Other active ingredients include wound healing agents (e.g., urea, allantoin, panthenol, alkali metal thiocyanates, chamomile-based actives and acetylsalicylic acid derivatives, ibuprofen, flurbiprofen, aspirin, indomethacin etc.); tooth buffering agents; demineralization agents; anti-inflammatory agents; anti-malodor agent; breath freshing agents; and agents for the treatment of oral conditions such as gingivitis or periodontitis.

***Analgesic and Anesthetic Agents:*** Dentifrice described herein may include one or more analgesic and/or anesthetic agents. Such agents may include, e.g., strontium chloride, potassium nitrate, sodium fluoride, sodium nitrate, acetanilide, phenacetin, acertophan, thiorphan, spiradoline, aspirin, codeine, thebaine, levorphenol, hydromorphone, oxymorphone, phenazocine, fentanyl, buprenorphine, butaphanol, nalbuphine, pentazocine, natural herbs, such as gall nut, Asarum, Cubebin, Galanga, Scutellaria, Liangmianzhen, Baizhi, acetaminophen, sodium salicylate, trolamine salicylate, lidocaine, and benzocaine.

***Other Ingredients:*** In some instances, dentifrices may include effervescing systems such as sodium bicarbonate citric acid systems, or color change systems. Dentifrices may also include one or more of the following: phenolic compounds (e.g., phenol and its homologues, including 2-methyl-phenol, 3-methyl-phenol. 4-methyl-phenol, 4-ethyl-phenol, 2,4-dimethol-phenol, and 3,4-dimethol-phenol); sweetening agents (e.g., sodium saccharin, sodium cyclamate, sucrose, lactose, maltose, and fructose); flavors (e.g., peppermint oil, spearmint oil, eucalyptus oil, aniseed oil, fennel oil, caraway oil, methyl acetate, cinnamaldehyde, anethol, vanillin, thymol and other natural or nature-identical essential oils or synthetic flavors); preservatives (e.g., p-hydroxybenzoic acid methyl, ethyl, or propyl ester, sodium sorbate, sodium benzoate, bromochlorophene, triclosan, hexetidine, phenyl silicylate, biguanides, and peroxides); opacifying and coloring agents such as titanium dioxide or F D & C dyes; and vitamins such as retinol, tocopherol or ascorbic acid.

### Gel, Liquid or Semisolid (Tooth Strip) Composition and Components

The compositions and components described herein can be provided in the form of gel or semisolid, e.g., tooth strip or film, e.g., a dissolvable strip or film. In some embodiments, the strip or film is a single layer. In some other embodiments, the strip or film can be multi-layered. In some embodiments a tooth strip can include a reservoir for delivery of a liquid composition.

The gel or semisolid composition (e.g., strip or film) has a polymer system and a soluble chitosan or derivatized chitosan described herein. The gel or semisolid composition (e.g., tooth strip or film) can have additional ingredients, some of which may be active, to provide additional oral health benefits that include breath freshening, whitening, tooth mineralization, tooth sensitivity prevention or treatment, and gum health maintenance or treatment.

In some embodiments, the gel or semisolid composition (e.g., strip or film) is dissolvable in saliva that is formed in the oral cavity or mouth. In some embodiments, after application of the gel or semisolid composition (e.g., strip or film) onto teeth, a substantive coating, that is not considered undesired residue, can be left to extend the residence time of the whitening agent or any active ingredient.

The solubility or dissolvability of the gel or semisolid composition (e.g., strip) is controlled by the composition to enable dissolution of the gel or semisolid composition (e.g., strip or film) over a desired pre-determined time frame. The time frame of the dissolution of the gel or semisolid composition (e.g., strip or film) can be adjusted based on the end benefit desired. To adjust the time frame, the nature of the water-soluble or water dispersible polymer system, the degree of crosslinking, if any, and the thickness of the strip or film should be adjusted. Generally, the thickness of the strip or film is from about 5 µm to about 2000 µm. Preferably, the thickness of the strip or film is from about 50 µm to about 500 µm.

The gel or semisolid composition (e.g., tooth strip or film) can be targeted to deliver a soluble chitosan or derivatized chitosan described herein to the desired area at a desired delivery rate, for example, within about 1 minute to about 360 minutes, preferably from about 1 minute to about 30 minutes, and most preferably from about 1 minutes to about 15 minutes, to whiten the teeth.

In another embodiment, the soluble chitosan or derivatized chitosan described herein can be encapsulated in a water-soluble or water dispersible shell, gel or semi-solid and incorporated within or on a surface of the tooth strip or film, or a layer of the tooth strip or film, to further control the delivery rate or in a cup or shell-type delivery system.

The gel or semisolid composition, e.g., slow dissolving gel or semisolid, e.g., can be used in tooth strip has a polymer system, e.g., a water-soluble and/or water swellable and/or water dispersible polymer system. The polymer system has adhesive properties, such that when it is brought to the teeth, the strip or film will adhere to the teeth. The polymer system includes one or more of poly-(vinylpyrrolidone)(PVP) or any of its derivatives, alkyl vinyl ether/maleic anhydride copolymer, alkyl vinyl ether/maleic acid copolymer, alkali metal or an amine salt of alkyl vinyl ether/maleic acid copolymer, partially or fully crosslinked alkyl vinyl ether/maleic anhydride copolymer, vinyl acetate copolymer, polyacrylates, polyurethane interpolymers, chitosan, poly(acrylic acid), poly(vinyl alcohol), poly(vinyl alcohol-g-ethylene glycol) copolymer, cellulose derivatives, hydroxy-propyl-methyl cellulose, hydroxyl-ethyl cellulose, hydroxy-propyl cellulose, poly(ethylene oxide), poly(propylene oxide), Polyquaterium-11, Polyquaterium-39, poloxamer, carbomer, gelatin, starch, alginic acid, salt of alginic acid, natural gums such as gum karaya, xanthan gum, guar gum, arabic gum tragacanth, or any combinations thereof.

Preferably, the water-soluble or water dispersible polymer system is present up to about 99.9 wt % based on the total weight of the gel or semisolid composition (e.g., tooth strip or film). More preferably, the polymer system is about 60 wt % to about 98 wt % of the total weight of the gel or semisolid composition (e.g., tooth strip or film).

The soluble chitosan or derivatized chitosan described herein can, upon contact with saliva, release the active agent onto the teeth in the oral cavity. Alternatively, the active agent can permeate through the gel or semisolid composition (e.g., strip or film) and be released to the surface where it is applied, including surfaces, such as, enamel, gum tissue and tongue.

Other ingredients in the gel or semisolid composition, e.g., slow dissolving gel or semisolid composition, e.g., can be used in tooth strip include e.g., a whitening agent. Exemplary whitening include hydrogen peroxide; carbamide peroxide; peroxycarbamate; persulfate, such as, persulfate salt or percarbonate salt; a perboric acid; perborate salt; PVP-hydrogen peroxide complex; calcium peroxide; metal chlorite (e.g. calcium chlorite, barium chlorite, magnesium chlorite, lithium chlorite, sodium chlorite, and potassium chlorite), hydroperoxide; peroxyacids; organic peroxides (e.g. benzoyl peroxide) chlorine dioxide; hydrogen peroxide adduct of carbodiimide persulfate; peroxide-generating compounds (e.g. azobisisobutyronitrile), phosphates, polyphosphates, alkali metal phosphates, alkali metal polyphosphates, sodium tripolyphosphate, or any combinations thereof.

The whitening agent is present up to about 99 wt % based on the total weight of the gel or semisolid composition (e.g., tooth strip or film). Preferably, the whitening agent is about 0.5 wt % to about 99 wt % of the total weight of the gel or semisolid composition (e.g., tooth strip or film). More preferably, the whitening agent is about 2 wt % to about 75 wt % of the total weight of the gel or semisolid composition (e.g., tooth strip or film).

The gel or semisolid composition, e.g., slow dissolving gel or semisolid composition, e.g., can be used in tooth strip can also have an ingredient that further enhances benefits to the oral cavity and teeth. Such ingredients include: an antimicrobial agent, a mineralization compound, a stain prevention compound, a desensitization compound, an anti-calculus agent, a flavoring agent, an anti-inflammatory agent, an antioxidant, a volatile sulfur scavenger, an odorant neutralizer, and/or a vitamin. The gel or semisolid composition (e.g., tooth strip or film) may also have a penetration enhancer, a plasticizer, a preservative, a surfactant or wetting agent, an anesthetic, an anti-allergenic, a pharmaceutical, or any combinations thereof.

The following antimicrobial agents can preferably be used in the present gel or semisolid composition (e.g., tooth strip or film): polyphenols (e.g. triclosan) zinc salts, stannous fluoride, chlorhexidine, hexetidine, sanguinarine, benzalkonium chloride, salicylanilide, domiphen bromide, cetylpyridinium chloride, tetradecylpyridinium chloride (TPC), N-tetradecyl-4-ethylpyridinium chloride (TDEPC), octenidine, delmopinol, octapinol, and other piperidine derivatives, nicin preparations, zinc/stannous ion agents, antibiotics such as augmentin, amoxicillin, tetracycline, doxycycline, minocycline, and metronidazole, and analogs and salts of the above, essential oils including thymol, menthol, eugenol, geraniol, carvacrol, citral, hinokitiol, eucalyptol, catechol, methyl salicylate, hydrogen peroxide, metal salts of chlorite, or any combinations of all of the above.

The following mineralization compounds are preferred for use in the present gel or semisolid composition (e.g., tooth strip or film): sodium monofluorophosphate, potassium monofluorophosphate, magnesium monofluorophosphate, acidulated fluorophosphate, amine fluoride, water-soluble salts of fluoride, such as, sodium fluoride, potassium fluoride, calcium fluoride, stannous fluoride, sodium fluorosilicate, bis-salicylato-bis-fluorotitanium (IV), ammonium fluorosilicate, calcium salt, phosphate salt, calcium salt/phosphate salt, calcium salt/ionic fluoride sources, zinc salt/phosphate salt), or any combinations thereof.

The following desensitization compounds can preferably be used in the present gel or semisolid composition (e.g., tooth strip or film): water-soluble potassium salt including potassium nitrate, potassium citrate, potassium chloride, potassium bicarbonate, potassium oxalate, and tubular occlusion compounds (e.g., ferric oxalate), or any combinations thereof.

The following anti-calculus agents can preferably be used in the present gel or semisolid composition (e.g., tooth strip or film): phosphates, pyrophosphates, polyphosphates, phosphonates (e.g. ethane-1-hydroxy-1,1-diphosphonate, 1-azacycloheptane-1,1-diphosphonate) polyphosphonates, polyacrylates and other polycarboxylates, ethylenediaminetetraacetic acid and other calcium chelators, carboxylic acids and their salts, zinc salts (e.g. sodium zinc citrate), PVM/MA copolymer or other polymers which interfere with crystal nucleation or growth, or any combinations thereof. It should be understood that when phosphate anti-calculus agent is used in conjunction with phosphate whitening agent, the phosphate anti-calculus agent(s) used is typically different than the phosphate whitening agent(s) used.

The following flavoring agents can preferably be used in the present gel or semisolid composition (e.g., tooth strip or film): flavoring oils, e.g., oils of spearmint, peppermint, wintergreen, sassafras, clove, sage, eucalyptus, marjoram, cinnamon, lemon, menthol, anethole, thymol, parsley oil, oxanone and orange, alpha-irisone, cassia, marjoram, oils thereof, propenyl guaethol, and methyl salicylate. Sweetening agents including, but not limited to, sucrose, lactose, maltose, sorbitol, xylitol, sodium cyclamate, sucralose, acesulfame-K, aspartame, and sodium saccharin. Any combinations of the preceding flavoring agents are also suitable for use in the gel or semisolid composition (e.g., tooth strip or film).

The following anti-inflammatory agents can preferably be used in the present gel or semisolid composition (e.g., tooth strip or film): non-steroidal anti-inflammatory agents, such as, ketorolac, flurbiprofen, ibuprofen, naproxen, indomethacin, aspirin, ketoprofen, piroxicam, meclofenamic acid, or any combinations thereof. Also, steroidal and non-steroidal anti-inflammatory agents and plant extracts that have demonstrated anti-inflammatory activities can be used. Also, polypeptide or protein anti-inflammatory agents can be used such as the anti-inflammatory cytokines.

The following antioxidants can preferably be used in the present gel or semisolid composition (e.g., tooth strip or film): Vitamin E, ascorbic acid, uric acid, kojic acid, coenzyme compounds (e.g. coenzyme Q-10), carotenoids, Vitamin A, flavonoids and polyphenols, herbal antioxidants, melatonin, aminoindoles, lipoic acids, or any combinations thereof.

Other suitable antioxidants include: rosemary extract, tocopherol, a derivative of tocopherol including a tocotriene, carotene, a carotenoid, a phenolic antioxidant including a phenolic acid, a bioflavonoid, a plant extract, curcumin, tetrahydrocurcumin, camphorol, quercetine, epigenine, or any mixtures thereof.

The following vitamins can preferably be used in the present gel or semisolid composition (e.g., tooth strip or film): Vitamin K, retinol (vitamin A), tocopherol, or any combinations thereof.

The tooth strip or film can be prepared by solution deposition, film-casting, dye-casting or extrusion.

### Kits

A compound described herein (e.g., a soluble chitosan or a derivatized chitosan) can be provided in a kit. The kit includes (a) a composition that includes a compound described herein, and, optionally (b) informational material. The informational material can be descriptive, instructional, marketing or other material that relates to the methods described herein and/or the use of the compound described herein for the methods described herein.

The informational material of the kits is not limited in its form. In one embodiment, the informational material can include information about production of the compound, molecular weight of the compound, concentration, date of expiration, batch or production site information, and so forth. In one embodiment, the informational material relates to use of the compound described herein to treat a disorder described herein.

In one embodiment, the informational material can include instructions to administer the compound described herein in a suitable manner to perform the methods described herein, e.g., in a suitable dose, dosage form, or mode of administration (e.g., a dose, dosage form, or mode of administration described herein). Preferred doses, dosage forms, or modes of administration are oral. In another embodiment, the informational material can include instructions to administer the compound described herein to a suitable subject, e.g., a human, e.g., a human having or at risk for a disorder described herein. For example, the material can include instructions to administer the compound described herein to such a subject.

The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as computer readable material, video recording, or audio recording. In another embodiment, the informational material of the kit is contact information, e.g., a physical address, email address, website, or telephone number, where a user of the kit can obtain substantive information about a compound described herein and/or its use in the methods described herein. Of course, the informational material can also be provided in any combination of formats.

The kit can include one or more containers for the composition containing the compound described herein. In some embodiments, the kit contains separate containers, dividers or compartments for the composition and informational material. For example, the composition can be contained in a bottle, or vial, and the informational material can be contained in a plastic sleeve or packet. In other embodiments, the separate elements of the kit are contained within a single, undivided container. For example, the composition is contained in a bottle, or vial that has attached thereto the informational material in the form of a label. In some embodiments, the kit includes a plurality (e.g., a pack) of individual containers, each containing one or more unit dosage forms (e.g., a dosage form described herein) of a compound described herein. The containers of the kits can be air tight, waterproof (e.g., impermeable to changes in moisture or evaporation), and/or light-tight.

The kit optionally includes a device suitable for administration of the composition, e.g., a cup (e.g., a measure cup), or any such delivery device.

### Examples

The following examples provide support for the use of chitosan derivatives, e.g., as an oral rinse to reduce and prevent, e.g., oral plaque accumulation. Chitosan derivatives were able to cause bacteria to aggregate immediately upon contact. As the dose was reduced C/A derivatives were able to retain the ability to clump below 20 µg/ml, where C/LBA was no longer able to aggregate *S. mutans.* For formulation purposes this allows for greater flexibility in the suggested dose to accommodate applications that might require low doses without the loss of bacterial clumping activity. For example, the MICs against *S. mutans, S. salivarius,* and *S. sanguinis* were determined to comparatively examine the antibacterial activity of each derivative against both oral pathogens (*S. mutans* and *S. sanguinis*) and a commensal strain (*S. salivarius*)*.* It was determined that C/A High had the best antibacterial activity, that it varied with respect to strain, and was effective against pathogenic strains at a lower dose (16 or 32 µg/ml) than the commensal strain (128 µg/ml). The demonstration that C/A will induce clumping at these lower doses suggests it may be possible to make an oral rinse product that spares commensal strains like *S. salivarius.* Further, C/LBA lacked significant antibacterial activity and could be used for bacterial clumping applications at higher concentrations without significantly effecting bacterial viability. Membrane permeability studies showed that *S. mutans* ATP leakage occurs within minutes of exposure, is dose dependent and more efficient with C/A rather than C/LBA. Simultaneous viability studies showed rapid cell death in C/A treated *S. mutans* suggesting that charge is important for membrane permeability, antibacterial activity as well as clumping. Biofilms *of S. mutans* were treated with each derivative and demonstrated that C/A was effective at removing mature biofilms. A dose of 128 µg/ml removed 99% of the biofilm in 4 hours. Further testing of C/A High against mixed bacterial population biofilms grown in a flow cell and an artificial mouth model evaluated the ability of C/A to reduce the cohesion of plaque and prevent bacterial attachment, respectively. These studies more closely mimicked actual suggested patient use (brief rinse twice daily) and provided evidence for the mechanism of action. Mixed species bacterial biofilms treated twice daily with C/A during development tended to be less robust and were significantly less cohesive (31%) than biofilms rinsed with only water. Similarly, mixed species biofilms grown on human teeth in an artificial mouth model when treated twice daily with C/A, did not accumulate as much plaque as teeth rinsed only with water. The source of C/A High activity is likely a combination of structure and charge. The ability of the molecule to quickly bind to bacteria and biofilms, permeabilize membranes and facilitate cell death likely contributes to the ability of the derivative to demonstrate such dramatic effects against oral biofilms after a relatively short treatment.

### Example 1. Chitosan-arginine (C/A) clumps Streptococcus mutans

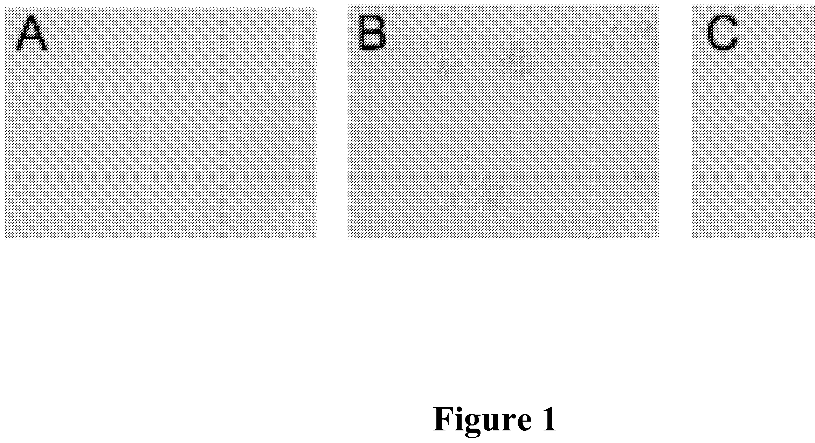

Chitosan-arginine has the ability to bind to bacteria and to aggregate bacterial cells at concentrations in the range of 1 to 1000 µg/mL. A lower concentration of chitosan-arginine in water was examined to determine the lower end of concentration rather where bacterial aggregation was observed. Approximately 10⁷ *S. mutans*/mL were resuspended in 1 mL of water and sonicated. To obtain a 10 µg/mL final concentration, 5 µL of a 2 mg/mL stock solution of chitosan-arginine (25% funtionalized, 43 kDa, PDI 2.28, 88 %DDA used in all studies) in water was added to 995µL *of S. mutans* in water (pH 7). The solution was gently agitated for 1 minute. *S*. *mutans.* Phase contrast microscopy at 400x documented the aggregation *of S. mutans* **(****Figures 1B** and **1C****)** compared to control **(****Figure 1A****).**

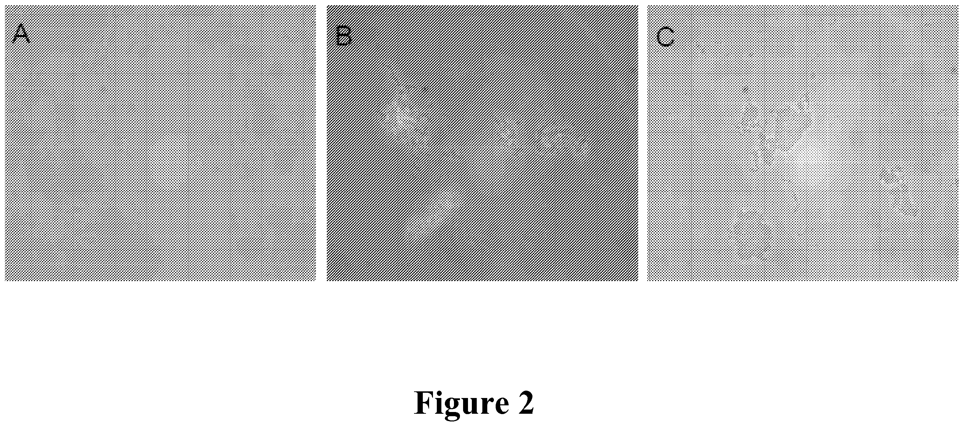

Further studies examined the ability of rhodamine labeled chitosan-arginine (C/A-R) to co-localize with clumped *S. mutans.* Similarly, approximately 10⁷ *S. mutans* were resuspended in 1 mL of water and sonicated. Phase contrast microscopic images show the lack of aggregation before treatment **(****Figure 2A****).** Phase contrast microscopic images document the aggregation of S. mutans after 1- minute of gentle agitation with 10 µg/mL **(****Figure 2B****)** or 100 µg/mL **(****Figure 2C****).** Images are overlaid with epifluorescence images that show the C/A-R associated with the aggregated *S*. *mutans.*

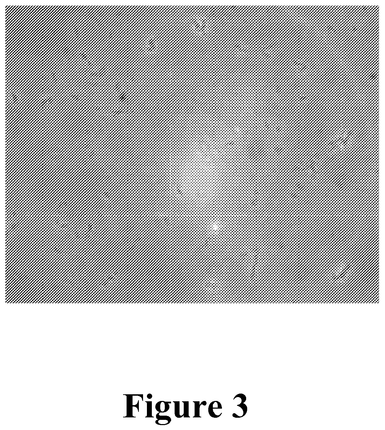

It was also determined that C/A-R when visualized at 100 ppm alone was uniformly distributed (not shown). Further, the Rhodamine BOC conjugate alone was visualized and found not to induce *S. mutans* to clump after 1-minute exposure **(****Figure 3****),** although it does associate with the *S. mutans.*

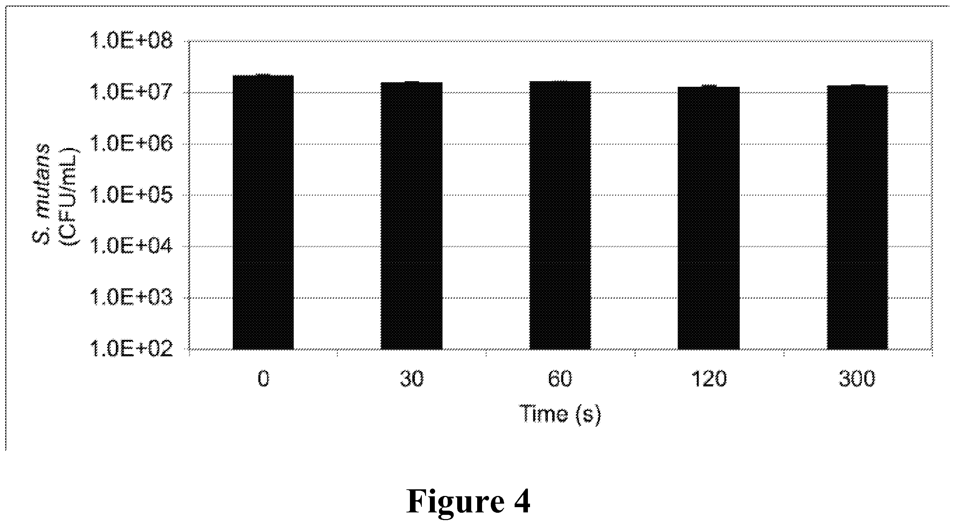

Viability of *S. mutans* exposed to chitosan-arginine was determined over the course of time. Overnight cultures of *S. mutans* were resuspended in water to approximately 10⁷ *S. mutans*/mL. *S. mutans* was treated for 30 seconds, 1, 2, or 5 minutes with a final concentration of 10 µg/mL of chitosan-arginine. At the specified time the treatment was neutralized and plated for cfu to determine viability. No significant difference in viability of the bacteria relative to control was observed following treatment with 10 µg/mL of chitosan-arginine up to 5 minutes **(****Figure 4****).**

### Example 2. Chitosan arginine (C/A) disrupts Streptococcus mutans biofilm

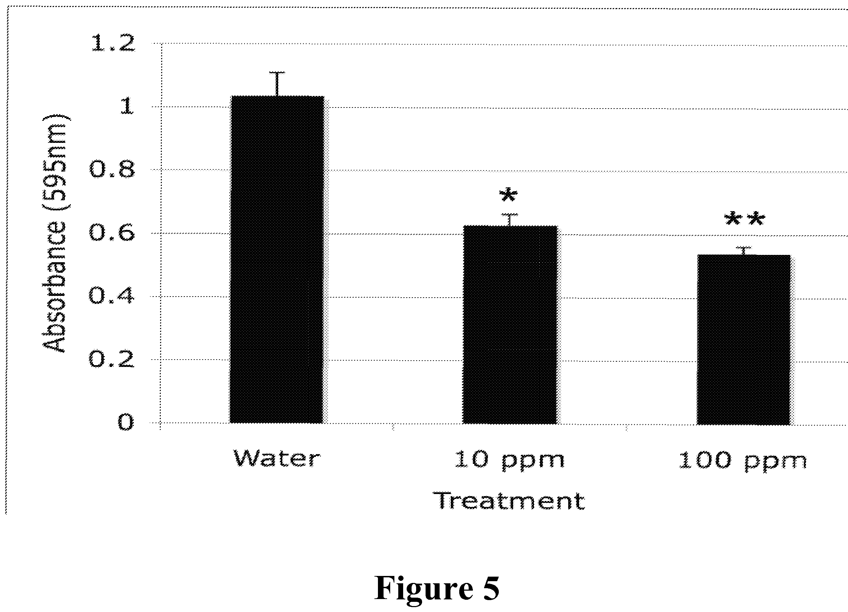

Several *S. mutans* biofilms were grown in 12-well untreated tissue culture plates. Biofilms were grown in 3mL of Todd Hewitt broth for 2 days stationary at 37 °C. Each well was washed with water then treated (in triplicate) with 1 mL of water (control), 10 ppm, or 100 ppm of chitosan-arginine for 1 minute with gentle agitation. Following treatment the remaining biofilm was stained with crystal violet for two minutes and washed with water to remove residual dye. Dye associated with the biofilm was released with 200µL of isopropanol and the absorbance (595nm) of each sample was determined to quantify the biofilm remaining associated with the surface. A 40% reduction (p=0.04) in the *S. mutans* biofilm was observed after treatment with 10 ppm of chitosan-arginine, and almost a 50% reduction (p=0.02) was observed following the 100 ppm of chitosan-arginine treatment compared to the water treatment **(****Figure 5****).**

### Example 3. Dental plaque removal with chitosan-arginine.

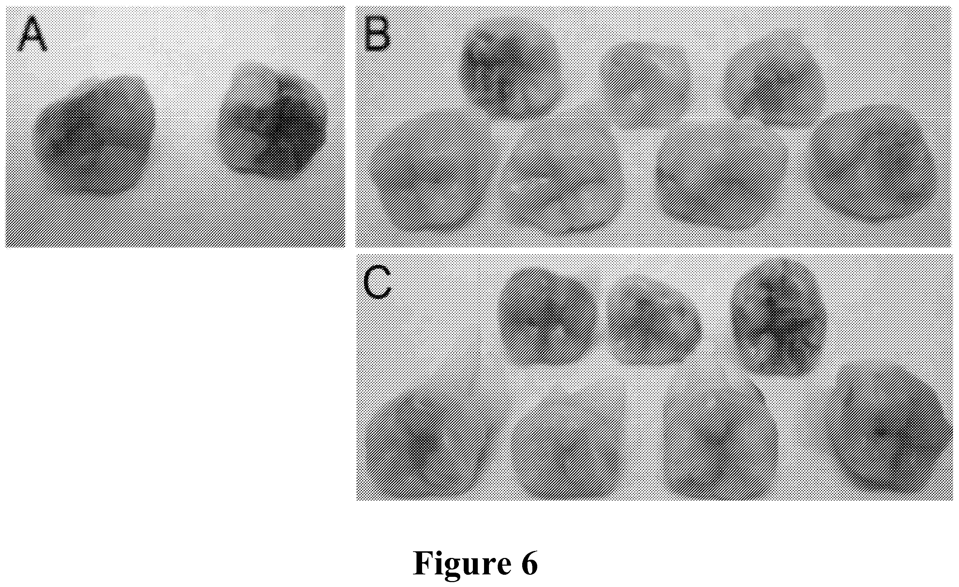

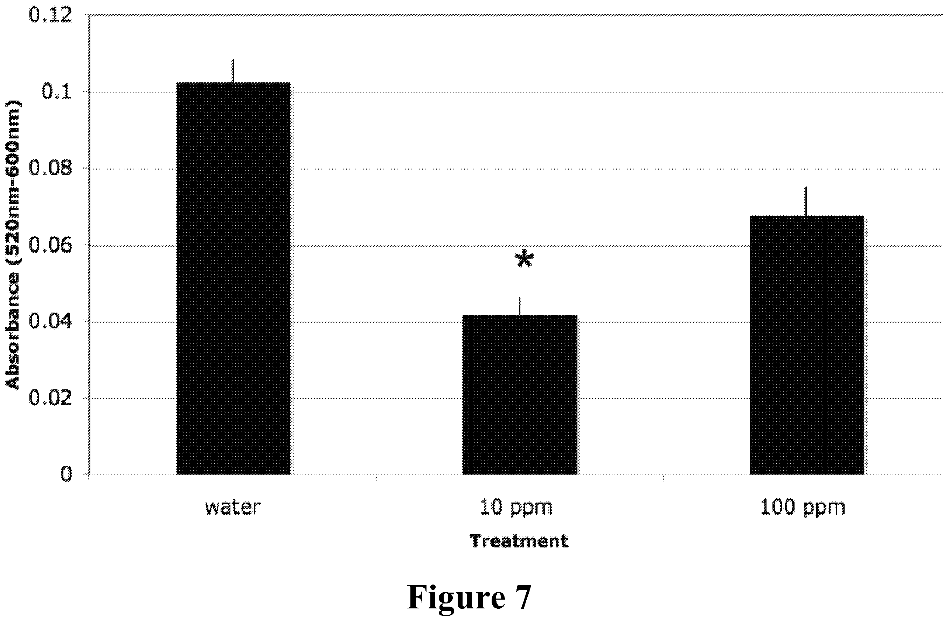

Several teeth (molars) were placed in a 50 mL tube containing 5 mL of Brain Heart Infusion broth supplemented with 50% saliva and 1% sucrose and inoculated with 50 µL of overnight *S. mutans* culture. The teeth were incubated with gentle agitation at 37 °C for 3 days, replacing the spent media with fresh Brain Heart Infusion broth supplemented with 50% saliva and 1% sucrose every 24 hours. After the 3-day incubation was completed, each tooth was briefly rinsed in PBS. Each tooth (7 per C/A treatment, 2 for water control) was placed in 50mL tube with either 5 mL of water (negative control), or C/A (10 µg/mL treatment) in water. Gentle agitation was applied for 1-minute then the teeth were placed in a 50% saliva solution for 6 hours. The treatment was repeated after the 6-hour incubation. The crowns of the teeth were stained with 2TONE dental dye for qualitative and quantitative analysis of the plaque on the teeth. Staining occurred for 2 minutes followed by a rinse in PBS to remove excess dye. Qualitative observations were documented and showed that less plaque was observed on chitosan-arginine treated teeth than teeth treated only with water **(****Figures 6A-6C****).** Each tooth was placed in 2 mL of PBS, vortexed and sonicated to remove any plaque and bacteria that will have incorporated the dye. Absorbance at 520 and 600nm were measured to quantify the retained dye and subtract the background of the material and bacteria associated with the dental plaque. The data shows that a significant difference (p=0.04) is achieved in reducing dental plaque following treatment with 10 µg/mL of chitosan-arginine **(****Figure 7****).** Some tooth variation was observed with the 100 ppm treatment.

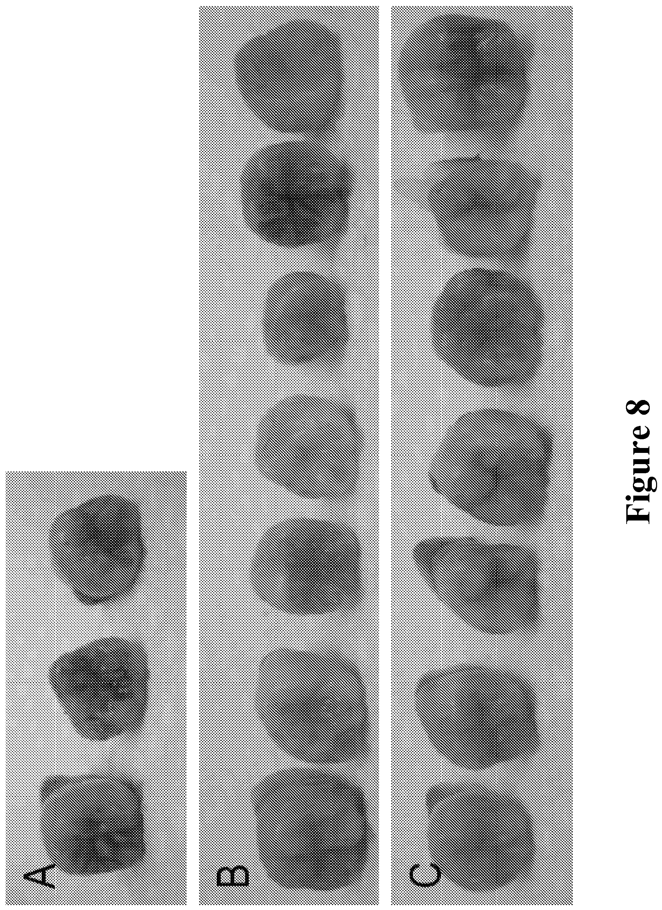

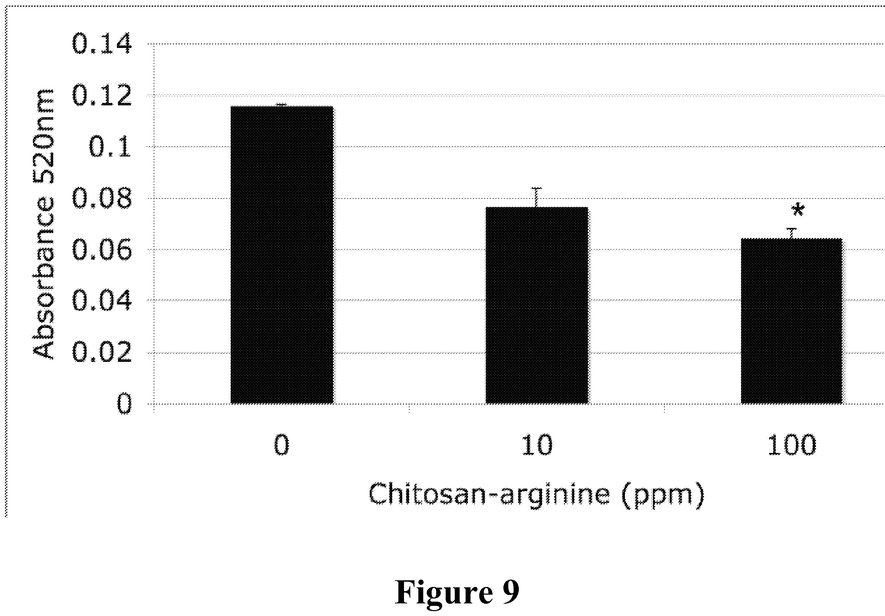

The assay as described above was repeated. Qualitative observations were documented and again show a reduction in the amount of plaque following treatement with chitosan-arginine compared to water **(****Figure 8****).** The data show that a significant difference (p=0.04) is achived in reducing dental plaque following treatment with 100 ppm of chitosan-arginine with some tooth variation observed with the 10 ppm treatment **(****Figure 9****).**

### Example 4. The ability of chitosan-arginine to remove Streptococcus mutans biofilms compared to Delmopinol (Decapinol).

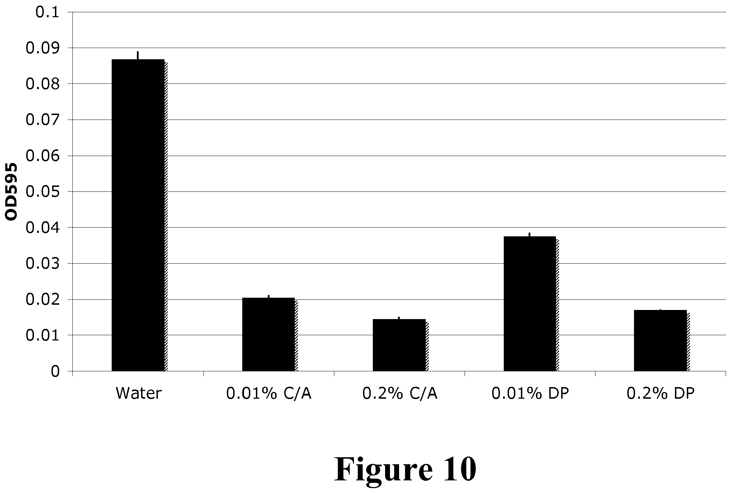

The ability of chitosan-arginine (C/A) to remove *S. mutans* biofilms compared to delmopinol, a similar dental rinse device, was investigated. **Figure 10** shows chitosan-arginine was more effective at removing *S. mutans* biofilms than Delmopinol. *S. mutans* culture was inoculated 1:1000 in Todd Hewitt broth and biofilms were grown statically in 12-well tissue culture plates for 4 days at 37 °C. The biofilms were washed three times with water to remove non-adherent cells. Crystal Violet stain was added to the biofilms for 2 minutes before treatment with either 0.01% (100 µg/ml) C/A or 0.2% (2 mg/ml) Delmopinol (Decapinol). The solutions were gently agitated in the well during treatment for 1 minute then the solution was removed and the wells were briefly rinsed with water. Ethanol was placed in the well to remove any residual dye and the OD595 was measured to quantify the amount of bacteria still in biofilm associated with the surface. The chitosan-arginine treatment of 0.01% (100 µg/ml) resulted in almost 5-fold reduction of biofilm compared to water rinsing alone. Further, this concentration of C/A was twenty-fold lower than the concentration of Delmopinol that is currently used (0.2%) These data suggest that C/A may be more effective at concentrations less than Delmopinol that is currently used for a similar purpose.

### Example 5. The effect of chitosan-arginine on Streptococcus mutans biofilms

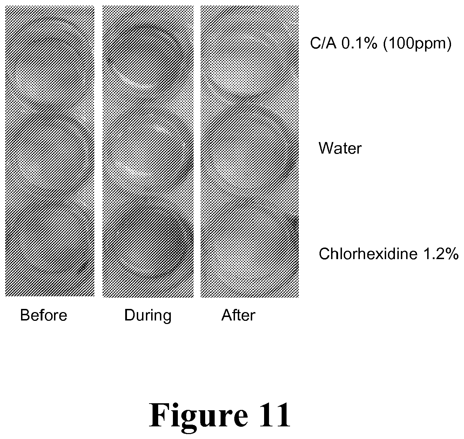

The *S. mutans* biofilms were grown in 12-well untreated tissue culture plates containing BHI media supplemented with 1% sucrose for approximately 3 days. The biofilms were rinsed with water three times and stained with crystal violet for 2 minutes then rinsed three times **(****Figure 11****, 'Before').** Water, 100 µg/ml chitosan-arginine or 1.2% chlorhexidine were used to treat each biofilm for 1 minute at room temperature **(****Figure 11****, 'During').** Following treatment the biofilms were rinsed three times and both the Chlorhexidine and chitosan-arginine treated biofilms were removed from the surface while the water only treated biofilm was unaffected **(****Figure 11****, 'After').**

### Example 6. The plaque removal activity of chitosan-arginine

Human molars were used as the substrate for *S. mutans* biofilm growth. This study was designed to simulate patient use of a product, and utilized plenary dye (2Tone) to quantify plaque adherent to human teeth immediately after treatment with 10 and 100 µg/ml of C/A High. An artificial mouth was constructed from 15 ml centrifuge tubes with holes drilled into the cap and bottom. The third molars were sterilized and placed inside the tube in a rack over a collection beaker in an incubator set at 37°C. A sterile Y connector was used to connect tubing from the bacteria and media (BHI supplemented with 1% sucrose) was pumped into the tube onto the tooth from separate reservoirs mixed periodically and kept at room temperature. Approximately 1x10⁷ cfu/ml *of S. mutans* was grown and resuspended in diluted media (1:20) BHI:water. The bacteria and 50% human pooled saliva was dripped over the tooth surface continuously at a rate of 1ml/hour for 48-hours. The plaque disolving effect was qualitatively assessed following staining with 2Tone dye to determine the coverage of the tooth surface by plaque.

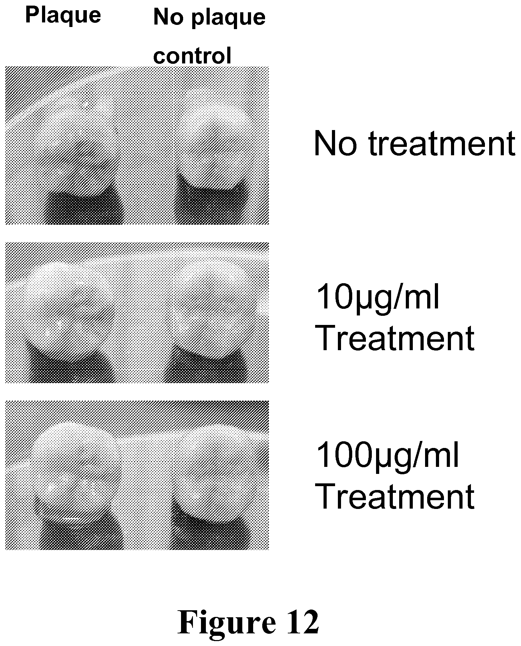

The tooth was removed from the tube and stained for 2 minutes with the 2Tone dye then rinsed for 30 seconds in PBS. Sterilized teeth were also stained and served as a negative control **(****Figure 12). Figure 12** shows the plaque remaining after gentle agitation in 10 µg/ml chitosan-arginine for 1 minute and after further treatment with 100 µg/ml chitosan-arginine for 1 minute. The image suggests that plaque is dissolved from the tooth surface immediately after treatment with chitosan-arginine and that repeated treatment as well as increased concentration can increase the effect.

### Example 7. Aggregation effect

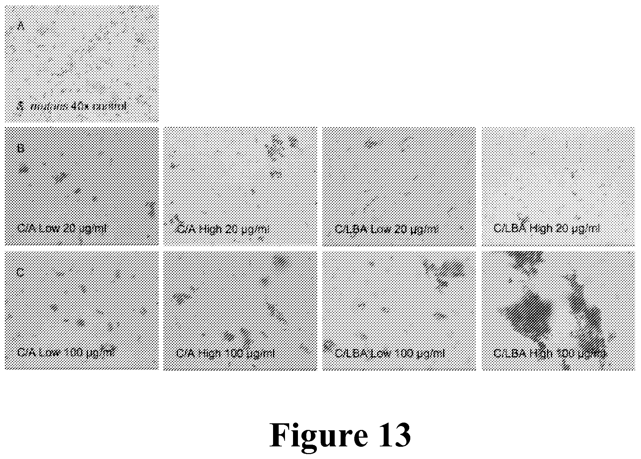

The soluble chitosan derivatives described herein have been shown to cause bacteria to aggregate in addition to the inherent antibacterial properties. It is hypothesized that the MW and charge of a chitosan derivative establishes the aggregative and antimicrobial efficiency, respectively. Therefore studies to investigate the dose range and kinetics of these activities were completed to compare charged and uncharged chitosan derivatives. First, a study was completed to determine the minimum dose *in vitro* that could effectively aggregate *S. mutans. S. mutans* was grown in Todd Hewitt broth overnight at 37° C and resuspended in water at a concentration of 10⁷ CFU/ml. *S. mutans* tested with each derivative and clumping was observed with all derivatives treated at a concentration of 100 µg/ml **(****Figure 13C****).** The C/LBA (especially C/LBA High) showed more dramatic clumping than C/A at 100 µg/ml. A significant decrease in clumping was observed for all derivatives when the concentration was reduced to 20 µg/ml **(****Figure 13B****).** However C/A seemed more able to retain clumping activity at lower doses than C/LBA. This suggests that charge is an important factor for chitosan derivative clumping over a broad range of concentrations.

To further examine the lower limits of the broad range of aggregation observed by the C/A derivatives the interaction of C/A High and Low MW with *S*. *mutans* was examined at lower concentrations. Both C/A High and Low were tested for clumping at 10, 5, and 2 µg/ml. It was observed that C/A High was able to maintain the ability to clump and aggregate *S. mutans* at a concentration of 10 or 5 µg/ml, but was not able to when treated with 2 µg/ml when compared to the control. Further, C/A Low was also not able to clump *S. mutans* at 2 µg/ml and was less able to clump at 10 and 5 µg/ml compared to C/A High. This test suggests that while charge is important to facilitate clumping over a broader range of concentrations, higher MW may also influence clumping ability.

### Example 8. The minimum inhibitory concentrations (MICs) of C/A Low and C/A High against Streptococcus mutans, Streptococcus sanguinis, and Streptococcus salivarius

The broth microdilution assay was used to determine the MIC of each chitosan derivative (C/A Low (chitosan-arginine 18kDa, 25% functionalized, 88% DDA, 1.47 PDI) and C/A High (chitosan-arginine 43kDa, 25% functionalized, 88% DDA, 2.28 PDI)) toward three oral bacteria (*Streptococcus mutans* ATCC 35668, *Streptococcus sanguinis* ATCC 10556, and *Streptococcus salivarius* ATCC 13419). Serial dilutions of each chitosan derivative were prepared as 2x solutions in sterile MH media for testing of concentrations between 1-512 µg/ml. A volume of 100 µl was added to a 96-well microtiter plate in 6 replicates. Each well was inoculated with 100µl of *S*. *mutans, S. salivarius,* or *S. sanguinis* (1 x 10⁶ CFU/ml) in Mueller-Hinton media to a final volume of 200µl per well. The MIC was defined as the lowest concentration of chitosan derivatives were visible bacterial growth was not apparent after 20 hours incubated at 37°C. Controls included media only and media plus bacteria only. An entire 96-well plate examined the media and chitosan derivative alone over the range of concentrations used to determine if any background was detected that could have interfered with the assay. Each assay was repeated in three independent experiments. Both C/LBA Low and C/LBA High were unable to inhibit the growth of any of the oral bacteria tested with as much as 256 µg/ml **(Table 1).** The C/A derivatives were able to inhibit oral bacteria. Specifically C/A High MIC against oral pathogens *S*. *mutans* and *S. sanguinis* were 16 and 32 µg/ml, respectively **(Table 1).** The MIC for *S. salivarius* was much higher (128 µg/ml) indicating that the positive chitosan derivative is more effective against oral pathogens and that the treatment concentration may be adjusted to below this level to preserve the natural oral flora. In general, the C/A low MW derivative was less effective at inhibiting the growth of the bacteria tested than C/A high MW. The neutral derivative, while retaining the ability to clump bacteria, is not effective for bacteriostatic treatments.

**Table 1. The minimum inhibitory concentrations**

| | **MIC C/A Low (18K)** | **MIC C/A High (43K)** |
|---|---|---|
| ***S*. *mutans* ATCC 35668** | >256 | 16 |
| ***S*. *salivarius* ATCC 13419** | >256 | 128 |
| ***S*. *sanguinis* ATCC 10556** | 64 | 32 |

### Example 9. The effect of chitosan-arginine on membrane permeability

Experiments were done to examine the release of ATP into the supernatant of *S. mutans* to determine the level of membrane permeability that occurred following exposure to chitosan derivatives as a function of time and concentration. The BacTiter Glo™ Assay is a luminescent assay to determine bacterial viability based on quantitation of ATP. In this test it was used according to manufacturers instructions (Promega, Madison, WI) to determine the amount of ATP in the bacterial supernatant. This assay was select because it is a sensitive (10⁻¹⁰ M ATP), stable, homogeneous method, and is compatible with tube and 96-well plate formats.

For these studies, *S. mutans* was grown over night then resuspended to 10⁸ cfu/ml in water. The bacteria were placed in 1 ml Aliquots into microcentrifuge tubes and treated with 0,5 or 100 µg/ml of each derivative for 5 or 60 minutes. Following treatment, a 200µl sample was removed for cfu enumeration, and then the tube was centrifuged to collect the supernatant to quantify the ATP released. Two 100µl supernatant samples were collected from each tube and the data was averaged. The ATP efflux and viability was tested in triplicate (three separate tubes) in at least two independent experiments. Data in Figures 3 and 4 is representative.

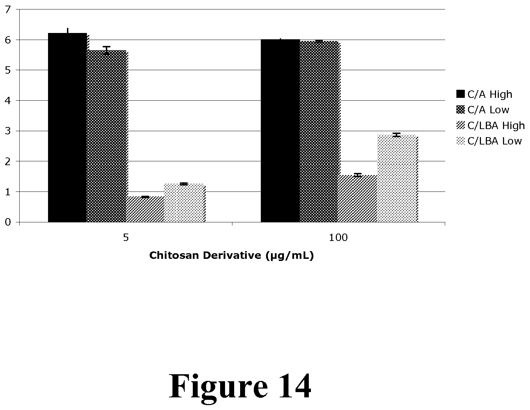

When C/A was applied at 5 µg/ml ATP leakage was observed within 5 minutes **(****Figure 14****).** A significant influence of increased dose (100 µg/ml) or time (1 hr) at either high or low molecular weight was not observed (data not shown). This observation suggests that extremely low concentrations of C/A induce membrane permeability that saturates the dose response at very low doses.

When C/LBA was applied at these concentrations, the low MW derivative induced greater ATP leakage but both showed increased ATP leakage **(****Figure 14****).** However, the neutral derivative did not have nearly the same magnitude of effect. Clearly, positive charge has a significant effect on the membrane damage.

Exposure time did not significantly increase ATP leakage. This suggests that ATP leakage upon contact with chitosan derivatives is rapid, dose dependent and more efficient with C/A rather than C/LBA.

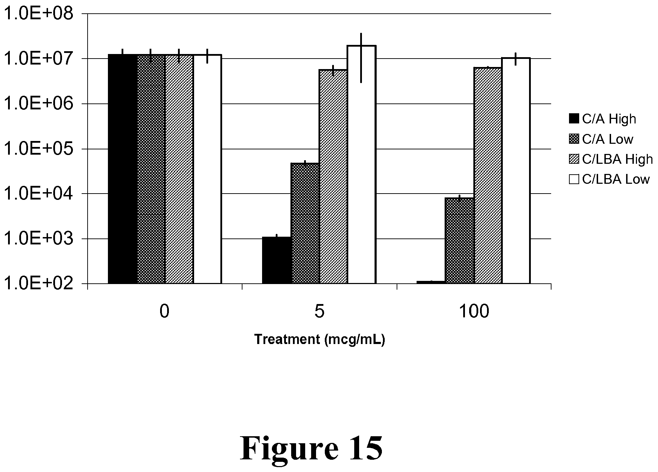

As shown in **Figure 15****,** the viability *of S. mutans* treated with C/A decreased significantly with 5 minutes exposure but more so with high molecular weight C/A (4 logs) than low molecular weight (about 2.5 logs). The viability *of S. mutans* treated with C/LBA showed no significant reduction in viability after 5 minutes and only modest reductions observed after 1-hour exposure to 100 µg/ml (1 log; **Figure 15**)**.** This observation correlates the membrane leakage to cell death, and again corroborates the role of positive charge in the permeabilization and killing of bacteria by chitosan derivatives. It is interesting to note that for applications where bacterial cell death is not warranted, the neutral derivatives will provide a platform to clump the bacteria with similar efficacy to the positive derivatives. Bacterial clumping is more dependent on MW than on charge.

### Example 10. The effect of different chitosan derivatives on biofilm reduction

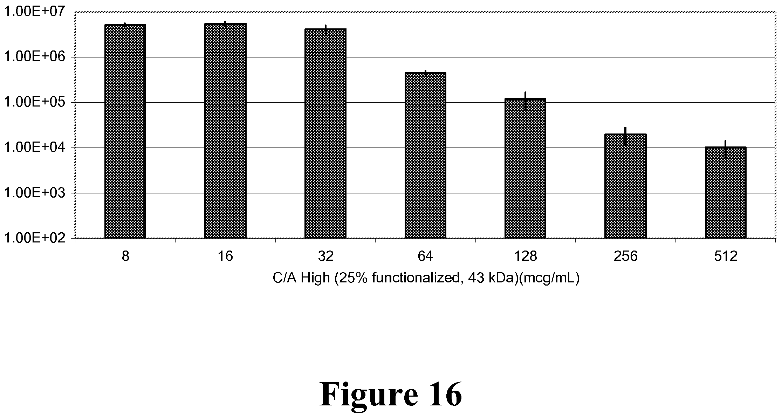

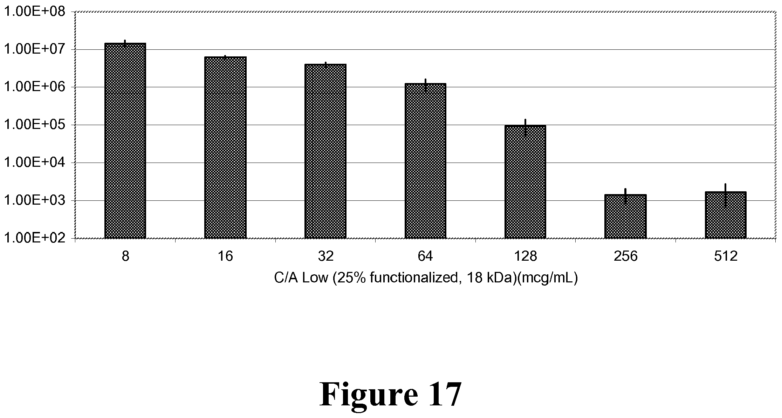

The different chitosan derivatives were analyzed with respect to biofilm reduction with previously established methods against mature *S. mutans* biofilms (Harrison, J.J. et al. (2005) High-throughput metal susceptibility testing of microbial biofilms Environ Microbiol 7,981-994). The biofilms were grown according to MBEC™ for High-throughput Screening (Innovotech, Edmonton, AB Canada) methods on a peg lid placed in trough containing BHI media supplemented with 1% sucrose for 36 hours. The pegs were rinsed and placed into a 96-well plate with serial dilutions of the chitosan derivative or controls and exposed for 4 hours at room temperature. The biofilms were rinsed, and the pegs removed and placed into microfuge tubes in 200µl of water. The tubes were sonicated to remove the peg biofilm. Aliquots of recovered biofilms were diluted and plated onto BHI agar to quantify growth. Testing was done in duplicate and representative assays are depicted. The *S. mutans* biofilms showed that the bacterial CFU were significantly reduced by both C/A derivatives. Both C/A High MW and Low MW demonstrated 99% and 99.9% reduction in the biofilm cfu recovered with a 128 µg/ml or 256 µg/ml treatment over 4 hours, respectively **(****Figures 16** and **17****).** These studies indicate that a concentration of 128 µg/ml of C/A effectively reduced 99% of preformed *S. mutans* biofilm after 4-hour exposure. Also, C/LBA was not effective at reducing mature *S*. *mutans* biofilms under the same conditions (data not shown). This suggests that the charged chitosan derivative, regardless of molecular weight, can more efficiently remove mature biofilms of a single species (*S. mutans*) than uncharged derivatives. The ability to remove mature preformed biofilms without mechanical disruption over time may be facilitated by the ability of the charged derivatives to rapidly diffuse into the biofilm. Most natural carbohydrate structures, such as bacterial capsule material and biofilm exopolysaccharide, can be found in negatively charged groups. Once in the biofilm, the positively charged derivatives may reduce biofilm cohesion by interfering with molecular interactions influenced by charge by either displacing or weakening bond.

### Example 11. The effect of chitosan-arginine on mixed oral biofilms

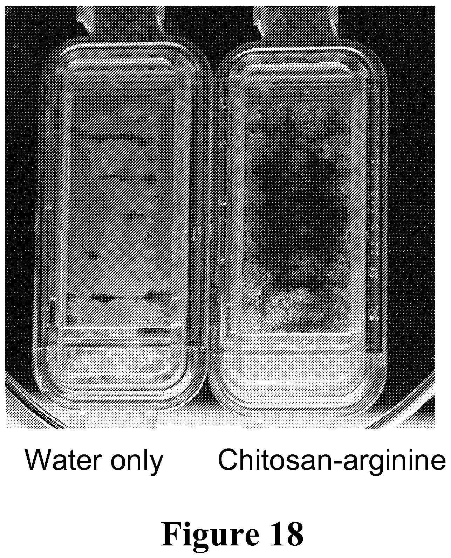

Using the C/A High derivative, the flow cell experiment was repeated with 6 replicates to compare water and C/A High (43kDa, 25% functionalized, 88% DDA, 2.28 PDI) treated mixed biofilms (*Streptococcus mutans* ATCC 35668, *Streptococcus sanguinis* ATCC 10556, and *Streptococcus salivarius* ATCC 13419) and obtain statistically significant data. This study indicated a 31% reduction in the wet weight (p=0.0007) after treatment with C/A High compared to water alone **(****Figure 18****).** This study also indicates a trend for less plaque accumulation without sonication.

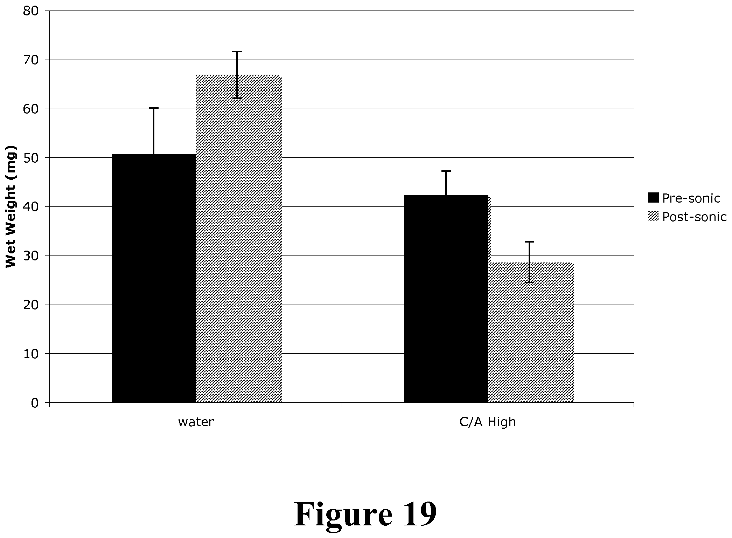

A comparison of the 2-minute treatments of mixed oral biofilm (*Streptococcus mutans* ATCC 35668, *Streptococcus sanguinis* ATCC 10556, and *Streptococcus salivarius* ATCC 13419) with water versus C/A High (43kDa, 25% functionalized, 88% DDA, 2.28 PDI) is shown in **Figure 19****.** This visual representation of the reduction in biofilm is striking and clearly demonstrated a lack of cohesion of the biofilm that was treated with C/A High **(****Figure 19****).** Plaque cohesion was reduced upon treatment with C/A, and facilitated mechanical removal.

### Example 12. The effect of chitosan-arginine on oral plaque reduction in a human teeth model

In order to examine the effectiveness of the selected C/A derivative in reducing oral plaque in a realistic model, human molars were used as the substrate for biofilm growth. This study was designed to simulate patient use of a product, and utilized plenary dye (2Tone) to quantify plaque adherent to human teeth treated twice daily with water or C/A High (100 µg/ml) over two days. An artificial mouth was constructed from 15 ml centrifuge tubes with holes drilled into the cap and bottom. The third molars were sterilized and placed inside the tube in a rack over a collection beaker in an incubator set at 37°C. A sterile Y connector was used to connect tubing from the bacteria and media (BMM supplemented with 1% sucrose) that was pumped into the tube onto the tooth from separate reservoirs mixed periodically and kept at room temperature. Approximately 1x10⁷ cfu/ml of *S. mutans, S. salivarius,* and *S*. *sanguinis* was grown and resuspended in diluted media (1:20) BHI:water. The bacteria and artificial saliva media was dripped over the tooth surface continuously at a rate of 1ml/hour for 48-hours. Twice daily at 8-hour intervals, including prior to starting the drip, the teeth were submerged in either water or C/A High at 100 µg/ml and vortexed gently at approximately 1000 rpm for only 30 seconds. The plaque inhibiting effect as qualitatively assessed following staining with 2Tone dye to determine the coverage of the tooth surface by old plaque.

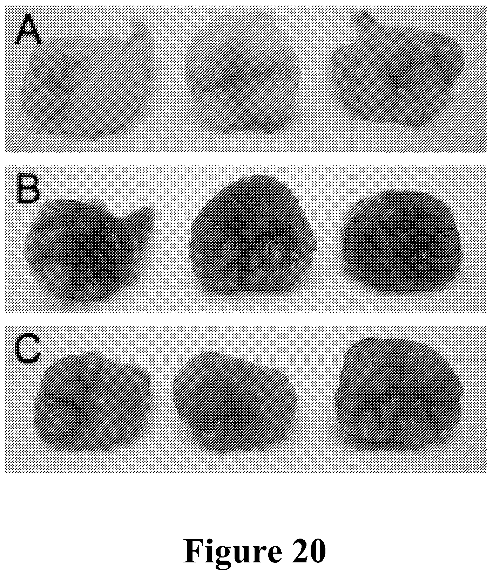

The teeth were removed from the tube and stained for 2 minutes with the 2Tone dye then rinsed for 30 seconds in PBS. Sterilized teeth were also stained and served as a negative control **(****Figure 20A****).** The three molars that were rinsed with water stained dark purple over the entire tooth with rough surface due to plaque build up **(****Figure 20B****).** The three teeth treated with C/A High stained pink indicating that only new plaque growth was present. Also the tooth surface was smooth **(****Figure 20C****).** This indicates that a twice-daily treatment with C/A High over time will prevent the accumulation of plaque on human teeth in this model.

### Example 13. The synergy of chitosan-arginine with other drugs or oral rinse formulations

Synergy of chitosan-arginine in combination with another drugs or oral rinse formulations was examined. This assay utilized combinations of chitosan-arginine ((21kDa, 29% functionalization, 88%DDA, 1.49 PDI) 8, or 16 µg/ml and 0.0125 or 0.00625% chlorhexidine to determine if the molecules together demonstrated greater antibacterial activity than each alone. Synergy was defined by achieving at least a 2-log reduction beyond the most antibacterial agent alone. Specifically, *Streptococcus mutans* (ATCC 35668) was treated with combinations of chitosan-arginine and chlorhexidine in a 96-well plate format for 1-hour in water. Solutions of both agents were prepared at 4X their intended final concentration. Two concentrations for each agent are used for each assay and the drugs were combined in 4 ways: CA(16µg/ml) CH(0.0125%), CA(16µg/ml) CH(0.00625%), CA8µg/ml CH(0.0125%), or CA8µg/ml CH(0.00625%). Controls of each drug alone as well as positive (*S. mutans* only) and negative (media only) controls were prepared. Approximately 2 x 10⁶ CFU/mL of *S*. *mutans* is added to all wells in a volume of 100 µl and incubated for the 1-hour at room temperature. After incubation, the plates were centrifuged for 10 minutes at 4000 RPM on a microtiter plate rotor, before being resuspended, sonicated, diluted and plated on BHI for remaining CFU. The data was examined and the log reduction beyond the most active antibacterial agent is reported in **Table 2.** At chitosan-arginine concentrations of 16 and 8 µg/ml and Chlorhexidine concentrations of 0.0125 and 0.00625% synergy was observed.

**Table 2. Log CFU reduction beyond most active agent**

| | Chlorhexidine 0.0125% | Chlorhexidine 0.00625% | Chlorhexidine 0% |
|---|---|---|---|
| C/A 16 µg/ml | 2.5 | 3.0 | 1.7 |
| C/A 8 µg/ml | 4.8 | 3.3 | 1.7 |
| C/A 0 µg/ml | 1.8 | 1.9 | 0 |

## Claims

1. A soluble derivatized chitosan for use in an oral rinse composition to reduce bacteria in the mouth, wherein the derivatized chitosan comprises a chitosan of the following formula (I): 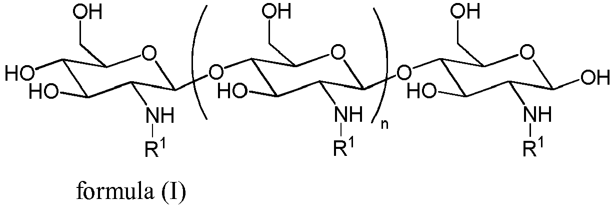 wherein:
n is an integer between 20 and 6000; and
each R¹ is independently selected for each occurrence from hydrogen, acetyl, and a group of formula (II): 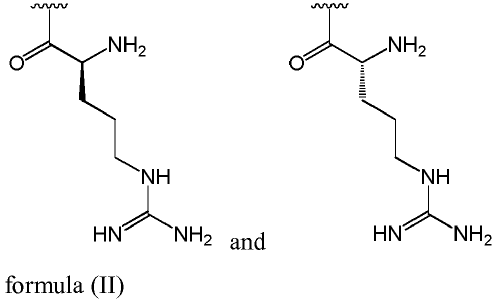 wherein at least 25% of R¹ substituents are H, at least 1% of R¹ substituents are acetyl, and at least 2% of R¹ substituents are a group of formula (II);
wherein the concentration of the soluble derivatized chitosan is from 10 to 250 ppm.

2. The soluble derivatized chitosan for use in an oral rinse composition of claim 1, wherein the composition further comprises xylitol.

3. The soluble derivatized chitosan for use in an oral rinse composition of claim 1, wherein the composition further comprises an anesthetic agent, e.g., a local anesthetic agent (e.g., menthol).

4. The soluble derivatized chitosan for use in an oral rinse composition of claim 1, wherein the composition further comprises a dissolving agent, e.g., ethanol.

5. The soluble derivatized chitosan for use in an oral rinse composition of claim 1, wherein the composition further comprises a cleaning agent, e.g., methyl salicylate.

6. The soluble derivatized chitosan for use in an oral rinse composition of claim 1, wherein the composition further comprises an anti-cavity agent, e.g., sodium fluoride.

7. The soluble derivatized chitosan for use in an oral rinse composition of claim 1, wherein the composition further comprises a whitening agent, e.g., hydrogen peroxide.

8. The soluble derivatized chitosan for use in an oral rinse composition of claim 1, wherein the composition further comprises an antibacterial or bubbling agent, e.g., hydrogen peroxide, e.g., to improve mechanical removal.

9. The soluble derivatized chitosan for use in an oral rinse composition of claim 1, wherein the composition further comprises a desensitizing agent, e.g., potassium nitrate.

10. The soluble derivatized chitosan for use in an oral rinse composition of claim 1, wherein the composition further comprises a coloring agent.

11. The soluble derivatized chitosan for use in an oral rinse composition of claim 1, wherein the composition further comprises a flavoring agent.

12. The soluble derivatized chitosan for use in an oral rinse composition of claim 1, wherein the composition further comprises one or more of water from 45% to 99%, ethanol from 0% to 25%, a humectant from 0% to 50%, a surfactant from 0.01% to 7%, a preservative from 0.01% to 0.5%, a thickening agent from 0% to 5%, a flavoring agent from 0.04% to 2%, a sweetening agent from 0.1% to 3%, and a coloring agent from 0.001% to 0.5%.

13. The soluble derivatized chitosan for use in an oral rinse composition of claim 12, wherein the composition further comprises one or more of fluoride ion as anti-caries agent from 0.05% to 0.3%, and an anti-calculus agent from 0.1% to 3%.

## Patentansprüche

1. Lösliches derivatisiertes Chitosan zur Verwendung in einer Zusammensetzung zum Mundspülen, um Bakterien in dem Mund zu verringern, wobei das derivatisierte Chitosan ein Chitosan der folgenden Formel (I) umfasst: 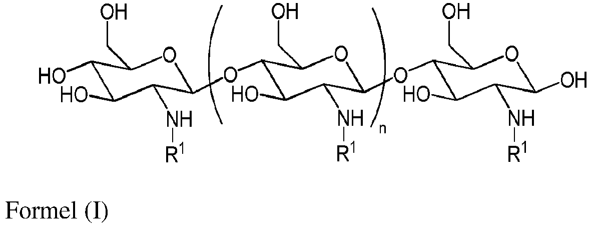 worin:
n eine ganze Zahl zwischen 20 und 6.000 ist; und
jedes R¹ unabhängig ausgewählt ist für jedes Auftreten von Wasserstoff, Acetyl und einer Gruppe der Formel (II): 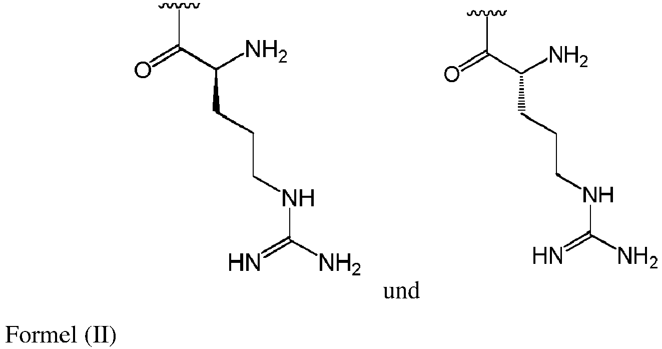 wobei mindestens 25% von Substituenten R¹ H sind, mindestens 1% von Substituenten R¹ Acetyl sind und mindestens 2% von Substituenten R¹ eine Gruppe der Formel (II) sind;
wobei die Konzentration des löslichen derivatisierten Chitosans von 10 bis 250 ppm beträgt.

2. Lösliches derivatisiertes Chitosan zur Verwendung in einer Zusammensetzung zum Mundspülen nach Anspruch 1, wobei die Zusammensetzung ferner Xylit umfasst.

3. Lösliches derivatisiertes Chitosan zur Verwendung in einer Zusammensetzung zum Mundspülen nach Anspruch 1, wobei die Zusammensetzung ferner ein Anästhetikum umfasst, z.B. ein Lokalanästhetikum (z.B. Menthol).

4. Lösliches derivatisiertes Chitosan zur Verwendung in einer Zusammensetzung zum Mundspülen nach Anspruch 1, wobei die Zusammensetzung ferner ein auflösendes Mittel umfasst, z.B. Ethanol.

5. Lösliches derivatisiertes Chitosan zur Verwendung in einer Zusammensetzung zum Mundspülen nach Anspruch 1, wobei die Zusammensetzung ferner ein Mittel zum Reinigen umfasst, z.B. Methylsalicylat.

6. Lösliches derivatisiertes Chitosan zur Verwendung in einer Zusammensetzung zum Mundspülen nach Anspruch 1, wobei die Zusammensetzung ferner ein Antikavitätsmittel umfasst, z.B. Natriumfluorid.

7. Lösliches derivatisiertes Chitosan zur Verwendung in einer Zusammensetzung zum Mundspülen nach Anspruch 1, wobei die Zusammensetzung ferner ein Mittel zum Aufhellen umfasst, z. B. Wasserstoffperoxid.

8. Lösliches derivatisiertes Chitosan zur Verwendung in einer Zusammensetzung zum Mundspülen nach Anspruch 1, wobei die Zusammensetzung ferner ein antibakterielles Mittel oder ein Mittel zum Blasenbilden umfasst, z.B. Wasserstoffperoxid, z.B. zum Verbessern des mechanischen Entfernens.

9. Lösliches derivatisiertes Chitosan zur Verwendung in einer Zusammensetzung zum Mundspülen nach Anspruch 1, wobei die Zusammensetzung ferner ein Mittel zur Desensibilisierung umfasst, z.B. Kaliumnitrat.

10. Lösliches derivatisiertes Chitosan zur Verwendung in einer Zusammensetzung zum Mundspülen nach Anspruch 1, wobei die Zusammensetzung ferner ein Mittel zum Färben umfasst.

11. Lösliches derivatisiertes Chitosan zur Verwendung in einer Zusammensetzung zum Mundspülen nach Anspruch 1, wobei die Zusammensetzung ferner ein geschmackverbesserndes Mittel umfasst.

12. Lösliches derivatisiertes Chitosan zur Verwendung in einer Zusammensetzung zum Mundspülen nach Anspruch 1, wobei die Zusammensetzung ferner ein oder mehrere der folgenden umfasst: von 45% bis 99% Wasser, von 0% bis 25% Ethanol, von 0% bis 50% eines Feuchthaltemittels, von 0,01% bis 7% eines oberflächenaktiven Mittels, von 0,01% bis 0,5% eines Fäulnis verhütenden Mittels, von 0% bis 5% eines Quellmittels, von 0,04% bis 2% eines geschmackverbessernden Mittels, von 0,1% bis 3% eines Süßungsmittels, von 0,001% bis 0,5% eines färbenden Mittels.

13. Lösliches derivatisiertes Chitosan zur Verwendung in einer Zusammensetzung zum Mundspülen nach Anspruch 12, wobei die Zusammensetzung ferner ein oder mehrere Fluorid-Ionen als Antikariesmittel von 0,05% bis 0,3% und ein Mittel gegen Zaahnstein von 0,1% bis 3% umfasst.

## Revendications

1. Chitosane modifié soluble pour l'utilisation dans une composition de rince-bouche pour réduire les bactéries dans la bouche, lequel chitosane modifié comprend un chitosane de la formule (I) suivante: 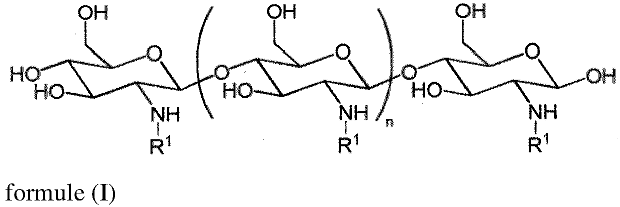 dans laquelle:
n est un entier entre 20 et 6000; et
chaque R¹ est indépendamment choisi pour chaque occurrence parmi hydrogène, acétyle et un groupe de formule (II): 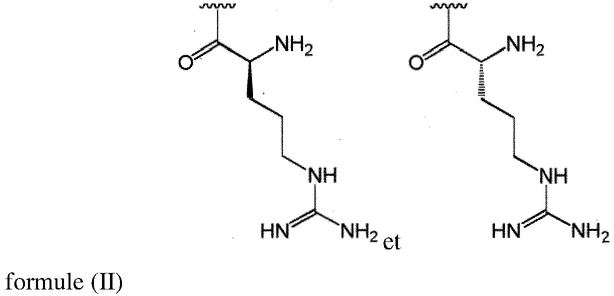 où au moins 25% des substituants R¹ sont H, au moins 1% des substituants R¹ sont acétyle et au moins 2% des substituants R¹ sont un groupe de formule (II);
dans lequel la concentration du chitosane modifié soluble est de 10 à 250ppm.

2. Chitosane modifié soluble pour l'utilisation dans une composition de rince-bouche selon la revendication 1, dans lequel la composition comprend en outre du xylitol.

3. Chitosane modifié soluble pour l'utilisation dans une composition de rince-bouche selon la revendication 1, dans lequel la composition comprend en outre un agent anesthésique, par exemple un agent anesthésique local (par exemple, menthol).

4. Chitosane modifié soluble pour l'utilisation dans une composition de rince-bouche selon la revendication 1, dans lequel la composition comprend en outre un agent dissolvant, par exemple l'éthanol.

5. Chitosane modifié soluble pour l'utilisation dans une composition de rince-bouche selon la revendication 1, dans lequel la composition comprend en outre un agent nettoyant, par exemple le salicylate de méthyle.

6. Chitosane modifié soluble pour l'utilisation dans une composition de rince-bouche selon la revendication 1, dans lequel la composition comprend en outre un agent anticarie, par exemple le fluorure de sodium.

7. Chitosane modifié soluble pour l'utilisation dans une composition de rince-bouche selon la revendication 1, dans lequel la composition comprend en outre un agent blanchissant, par exemple le peroxyde d'hydrogène.

8. Chitosane modifié soluble pour l'utilisation dans une composition de rince-bouche selon la revendication 1, dans lequel la composition comprend en outre un agent antibactérien ou formant des bulles, par exemple le peroxyde d'hydrogène, par exemple pour améliorer l'enlèvement mécanique.

9. Chitosane modifié soluble pour l'utilisation dans une composition de rince-bouche selon la revendication 1, dans lequel la composition comprend en outre un agent désensibilisant, par exemple le nitrate de potassium.

10. Chitosane modifié soluble pour l'utilisation dans une composition de rince-bouche selon la revendication 1, dans lequel la composition comprend en outre un agent colorant.

11. Chitosane modifié soluble pour l'utilisation dans une composition de rince-bouche selon la revendication 1, dans lequel la composition comprend en outre un agent aromatisant.

12. Chitosane modifié soluble pour l'utilisation dans une composition de rince-bouche selon la revendication 1, dans lequel la composition comprend en outre un ou plusieurs parmi l'eau de 45% à 99%, l'éthanol de 0% à 25%, un humectant de 0% à 50%, un tensioactif de 0,01 à 7%, un conservateur de 0,01% à 0,5%, un agent épaississant de 0% à 5%, un agent aromatisant de 0,04% à 2%, un agent édulcorant de 0,1% à 3% et un agent colorant de 0,001% à 0,5%.

13. Chitosane modifié soluble pour l'utilisation dans une composition de rince-bouche selon la revendication 12, dans lequel la composition comprend en outre un ou plusieurs parmi l'ion fluorure en tant qu'agent anticarie de 0,05% à 0,3% et un agent antitartre de 0,1% à 3%.
